# EUROPEAN PATENT APPLICATION

(11) **EP 2 606 884 A1**
(43) Date of publication of application: **26.06.2013**
(21) Application number: 11010130.0
(22) Date of filing: 21.12.2011
(51) Int. Cl.: A61K 31/351, A61K 31/407, A61K 31/4412, A61P 35/00

(54) **Inhibitors of notch signaling pathway and use thereof in treatment of cancers**

(71) Applicant: Ecole Polytechnique Fédérale de Lausanne (EPFL), 1015 Lausanne (CH)
(72) Inventor: Lehal, Rajwinder, 1015 Lausanne (CH); Radtke, Freddy, 1015 Lausanne (CH)
(74) Representative: KATZAROV S.A.

(57) **Abstract**

The present invention relates to use of inhibitors of Notch signaling pathway selected from the group consisting of 6-4[-(tertbutyl)-phenoxy] pyridine-3-amine (13), Cyclopiazinic acid and Lasatocid in treating and/or preventing cancers.

## Description

### FIELD OF THE INVENTION

The preset invention relates to use of inhibitors of Notch signaling pathway selected from the group consisting of 6-4[-(tertbutyl)-phenoxy] pyridine-3-amine (13), Cyclopiazonic acid and Lasalocid in treating and/or preventing cancers.

### BACKGROUND OF THE INVENTION

The Notch signaling pathway represents a critical component in the molecular circuits that control cell fate during development, cell survival and cell proliferation. (Cancer Res 2007;67(5):1879-82). Aberrant activation of this pathway contributes to tumorigenesis. The Notch family members are being revealed as oncogenes in an ever-increasing number of cancers. The role of Notch in human cancer has been highlighted recently by the presence of activating mutations and amplification of Notch genes in human cancer and by the demonstration that genes in the Notch signaling pathway could be potential therapeutic targets. It has become clear that one of the major therapeutic targets in the Notch pathway arc the Notch receptors, in which γ-secretase inhibitors prevent the generation of the oncogenic (intracellular) domain of Notch molecules and suppress the Notch activity.

Though significant progress has been made in dissenting the complex workings of this signaling pathway, there arc very limited options available for Notch inhibitors. However the pioneering class of Notch inhibitors is already in clinical trials for few cancer types, such as MK0752 of Merck Sharp & Dohme Corp. MK0752, a synthetic small molecule, inhibits the Notch signaling pathway, which may result in induction of growth arrest and apoptosis in tumor cells in which the Notch signaling pathway is overactivated.

However there is a need to identify and develop further specific inhibitors of Notch signaling pathway useful for treating and/or preventing cancers.

### SUMMARY OF THE INVENTION

The present invention concerns an Inhibitor of Notch signaling pathway for use in treating and/or preventing a cancer, wherein said inhibitor of Notch signaling pathway is selected from the group consisting of 6-4[-(tertbutyl)-phenoxy] pyridine-3-amine (I3), Cyclopiazonic acid and Lasalocid

A further object of the present invention is to provide a pharmaceutical composition comprising an inhibitor of Notch signaling pathway selected from the group consisting of 6-4[-(tertbutyl)-phenoxy] pyridine-3-amine (I3), Cyclopiazonic acid and Lasalocid, and a pharmaceutically acceptable carrier.

The invention also contemplates a kit comprising one or more doses of an inhibitor of Notch signaling pathway selected from the group consisting of 6-4[-(tertbutyl)-phenoxy] pyridine-3-amino (13), Cyclopiazonic acid and Lasalocid.

### BRlEF DESCRIPTION OF THE FIGURES

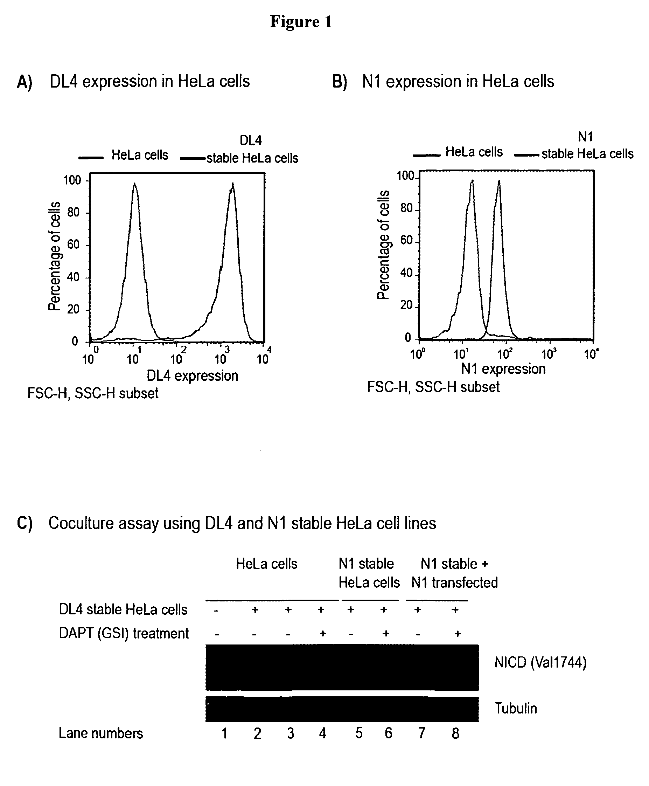

Figure 1 shows DL4 and NI surface expression on stable DL4 and N1-HeLa cells with concomitant Notch signaling activation in coculture assay: A-B) Flow cytometric analysis was performed on parental HeLa, stable DL4 lentivirally transduced HeLa (A) and stable N1 lentivirally transduced HeLa cells (B). Histograms for each stable cell line are shown with the parental cell line as negative control and stained with either anti-DL4 mAb (A) or anti-N1 mAb (B). Blue lines indicate the expression profiles of the stably transfected cell lines and the red line histograms depict the staining profile of the parental HeLa cells. Both histograms are gated on live cells (FSC vs SSC). (C) Western Blot analysis is shown on whole cell extracts to assess Notch signaling activation using the NICD (Val1744) Ab. Lane 1 shows the parental HeLa cells alone, lane 2 and 3 depict parental HeLa cells cocultured with DL4 HeLa cells and lane 4 shows the parental HeLa cells cocultured with DL4-HeLa in the presence of DAPT (a gamma secretase inhibitor - GSI). Lane 5 depicts NICD from cocultures of N1 HeLa and DL4-HeLa cells and lane 6 is the same coculture but in the presence of DAPT. Lane 7 and 8 uses a coculture system of N1-HeLa cells transfected additionally with full length Notch1 (Notch1 transfected) in the absence (lane7) or presence of DAPT (lane 8). Tubulin was used as an equal loading control.

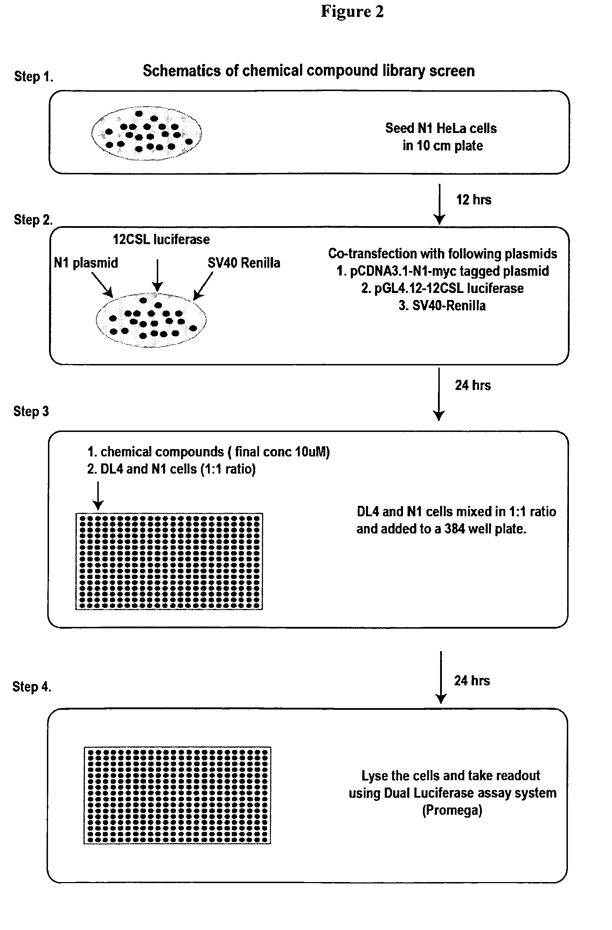

Figure 2. Schematics of assay development for chemical compound library screening. Step 1) N1-HeLa cells were seeded in a 10 cm plate. Step 2) N1-HeLa cells were co-transfected with full length Notch1, 12x CSL luciferase and SV40 Renilla plasmids. Step 3) Twenty-four hours later, DL4- and N1-HeLa cells were mixed in 1:1 ratio (5000: 5000 cells/well) and dispensed into 384 well plate containing chemical compounds. Step 4) The Notch pathway activation was measured using dual luciferase assay system.

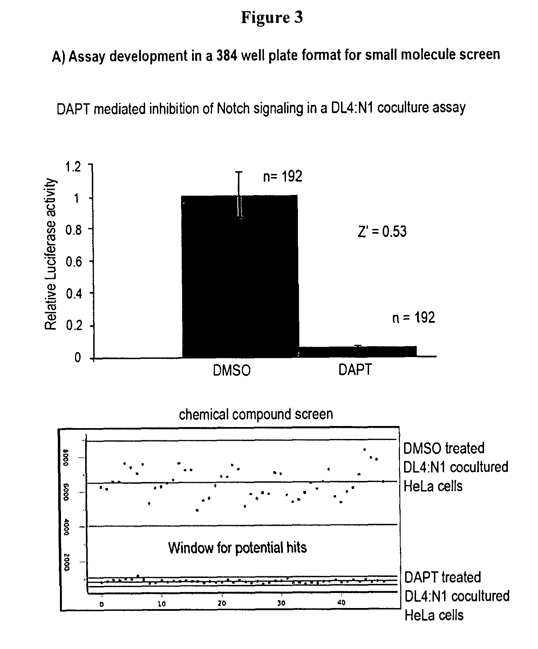

Figure 3 shows Assay development. A) Experimental set up for chemical library screen. Upper panel) N1 HeLa cells were transfected with full length Notch1, 12xCSL luciferase and SV40 renilla plasmids were coculture with DL4- HcLa in 1:1 ratio (5000:5000 cells/well) and seeded on to 384 well plate. As controls, half of the plate (192 wells) was treated with DMSO and the second half was treated with 10 µM DAPT. DAPT treatment of the system leads to 10-fold downregulation of the Notch pathway. Lower panel) A representation of DAPT mediated downregulation of Notch signaling. The blue window represents Notch signaling activation in DMSO treated cells and green window represents pathway downregulation in DAPT treated cells. The white window represents the window of opportunity where potential negative regulators of Notch signaling will be present following chemical library screen.

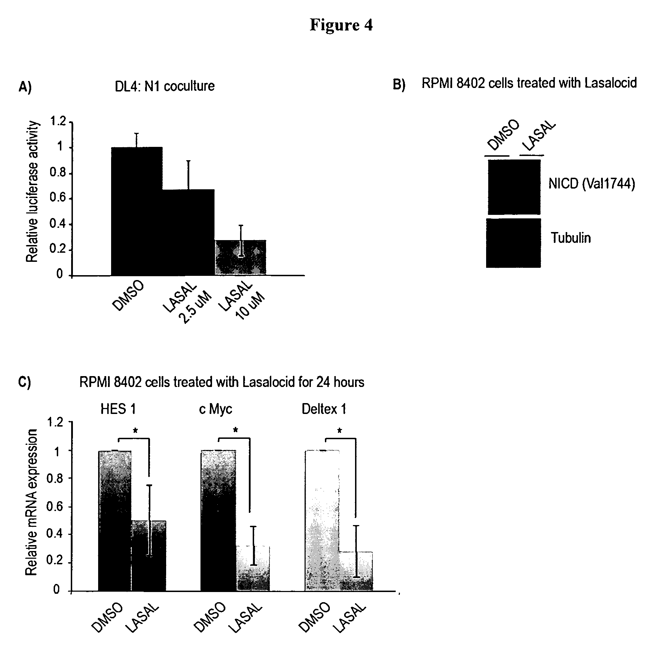

Figure 4 shows Lasalocid mediated inhibition of Notch signaling. A) DL4- and N1-HeLa cells were cocultured in 1:1 ratio and treated with DMSO or 2.5µM and 10µM of Lasalocid for 24 hours. The effect of Lasalocid treatment on Notch signaling was determined by quantification of 12xCSL driven luciferase activity. SV40 renilla readout was used as an internal control. Treatment of DL4:N1 coculture assay with 10µM of Lasalocid caused a 70% reduction in the luciferase activity. B) RPMI 8402 cells were treated with 10µM Lasalocid or with DMSO for 24 hours. Whole cell lysate was analyzed by western blot for the expression of NICD using Val1744 antibody. Treatment of RPMI 8402 with Lasalocid caused a downregulation of NICD levels compared to DMSO treated cells. C) Quantification (by qRT-PCR) of mRNA levels of HesI, cMyc and Deltex1 in Lasalocid and DMSO treated RPMI 8402 cells. Cells treated with Lasalocid exhibit more than 50 % downregulation of Hes1, cMyc and Deltex I transcripts compared to DMSO treated cells. Statistical analyses were done using 2-tailed t.test. * - p value < 0.05. Data normalized to HPRT as a house-keeping gene.

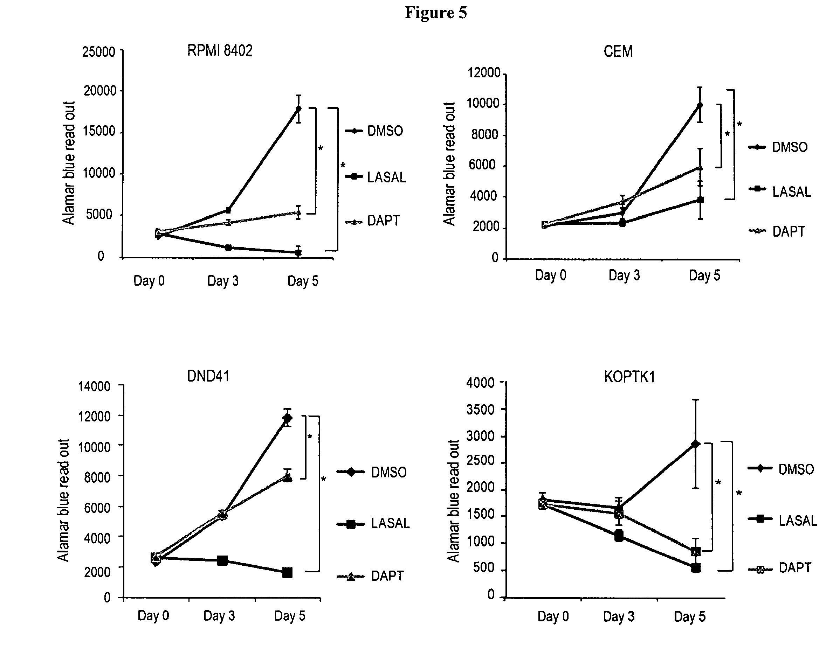

Figure 5 shows Lasalocid causes a growth arrest in Notch-dependent T-ALL cell lines. Human T-All cell lines RPMI 8402, CEM, DND41 and KOPTK1 (5000 cells/well for each line) were seeded in a 96 well plate (8 replicates for each time point and treatment). The cells were treated with 10 µM of Lasalocid, DAPT and DMSO. To quantify the effect of Lasalocid on proliferation, Alamar blue readout was taken at day 0, day 3 and day 5. Statistical analyses were done using 2-tailed t.test. * = p value < 0.05.

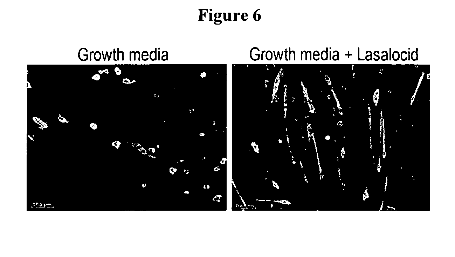

Figure 6 shows Lasalocid mediated differentiation of myoblasts. C2C12 cells were seeded on cover slips and grown in the presence of 10% serum (growth media) supplemented with DMSO or 10 µM of Laslacid for three days. Cells were allowed to grow to 100% confluency, On day 3, cells were fixed, blocked with 1% BSA and stained with an antibody against Myosin heavy chain (MIIC). An Alexa 488 conjugated (green colour) secondary antibody was used to detect MHC positive cells. The cell nuclei were stained with DAPI. Immunofluorescence microscopy showed an absence of MHC positive multinucleated myotubes in the C2C12 cells grown in the presence of growth media. The myoblasts grown in the presence of Lasalocid become multinucleated myotubes and express MHC.

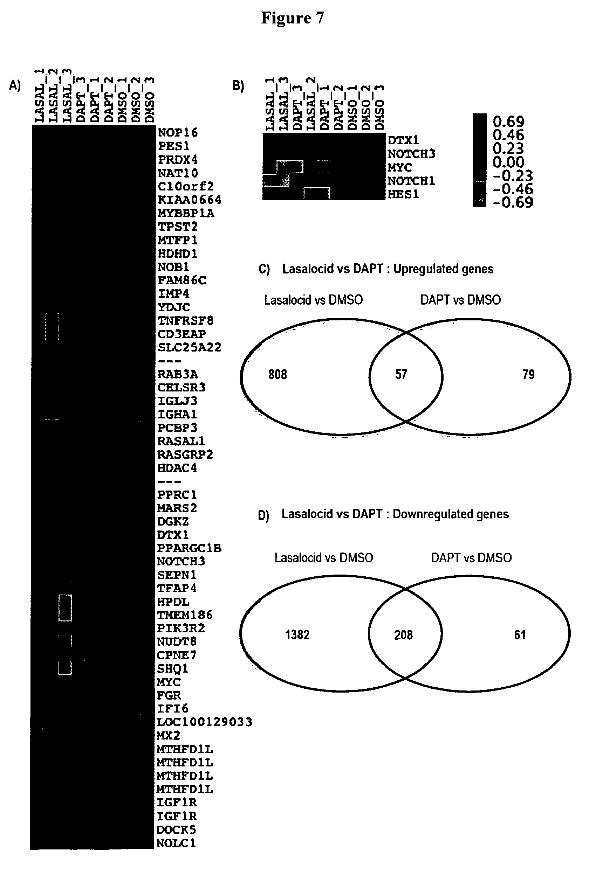

Figure 7 shows Lasalocid mediated changes in the global gene expression in KOPTK1 cell line. A) Top 54 genes (pvalue <0.05) commonly downregulated by Lasalocid and DAPT treatment when compared to DMSO. Analyses done with Cluster 3.0 software using normalized data sets. Red color indicates an upregulation and green colour represents downregulation of the gene expression. B) The effect of Lasalocid and DAPT on the Notch pathway compared to DMSO treated cells. Red color indicates an upregulation and green colour represents downregulation of the gene expression. C) Venn diagram of genes differentially or commonly upregulated between Lasalocid and DAPT. In total 808 genes were differentially upregulated in Lasalocid treated cells while 57 genes were upregulated by both Lasalocid and DAPT treatment. D) Venn diagram showing 208 genes downregulated by both Lasalocid and DAPT treatment while Lasalocid treatment downregulated additional 1382 genes while DAPT differentially downregulated another set of 61 genes.

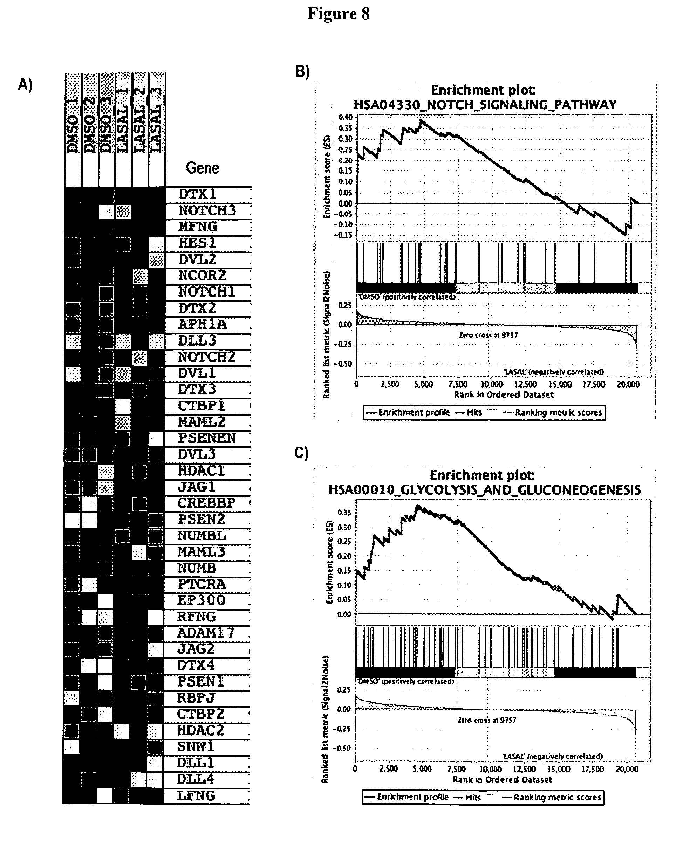

Figure 8 shows Gene set enrichment analyses (GSEA) of gene expression profile after Lasalocid treatment. A) Heat map representation of the Notch pathway following GSEA. GSEA were performed using differentially regulated genes with a p value < 0.05. Red colour represents an upregulation and blue colour represents a downregulation of the genes. B) GSEA analyses (with FDR value < 5%) show an enrichment of Notch components in the DMSO treated cells suggesting a downregulation of Notch pathway in Lasalocid treated cells. C) Treatment of cells with Lasalocid led to a downregulation of several genes involved in general metabolism processes. An example shows an enrichment of genes involved in glycolysis and gluconeogenesis pathway in DMSO treated cells due to downrcgulation metabolic genes in the Lasalocid treated cells.

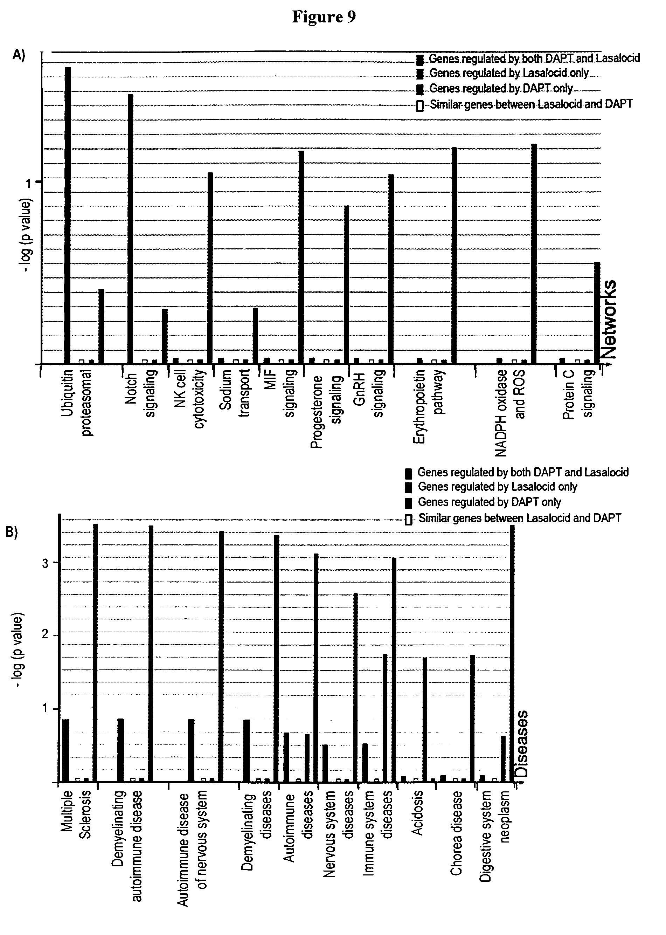

Figure 9 shows GeneGo analyses of genes regulated by Lasalocid and DAPT: GeneGo analyses were carried out using genes significantly represented in Lasalocid and DAPT treated samples (fold change = 2, p value < 0.05). Black bars represent genes significantly regulated by both Lasalocid and DAPT. Orange bars represent genes regulated by Lasalocid only and blue bar represents genes regulated uniquely by DAPT in a given pathway or network. White bars represents genes that may be similar in both Lasalocid and DAPT treated cells. The graphs were drawn using-log (p value) on Y-axis and X-axis shows networks and diseases. A) A set of genes belonging to Ubiquitin proteasomal and Notch pathways were regulated equally by both Lasalocid and DAPT (black bars). In addition there are several genes belonging to these pathways that are only regulated by Lasalocid not DAPT (orange bars). Lasalocid treated cells were also significantly enriched for genes involved in NK cell cytotoxicity, sodium transport, MIF signaling, progesterone signaling, GnRH signaling, Erythropoietin signaling and protein C signaling. B) Lasalocid treatment causes an alteration in the genes involved in various diseases of nervous system such as Multiple sclerosis, Demyelinating diseases, autoimmune diseases and digestive system neoplasm (all orange bars). DAPT regulated genes (blue bars) were particularly enriched in the immune system diseases and Acidosis.

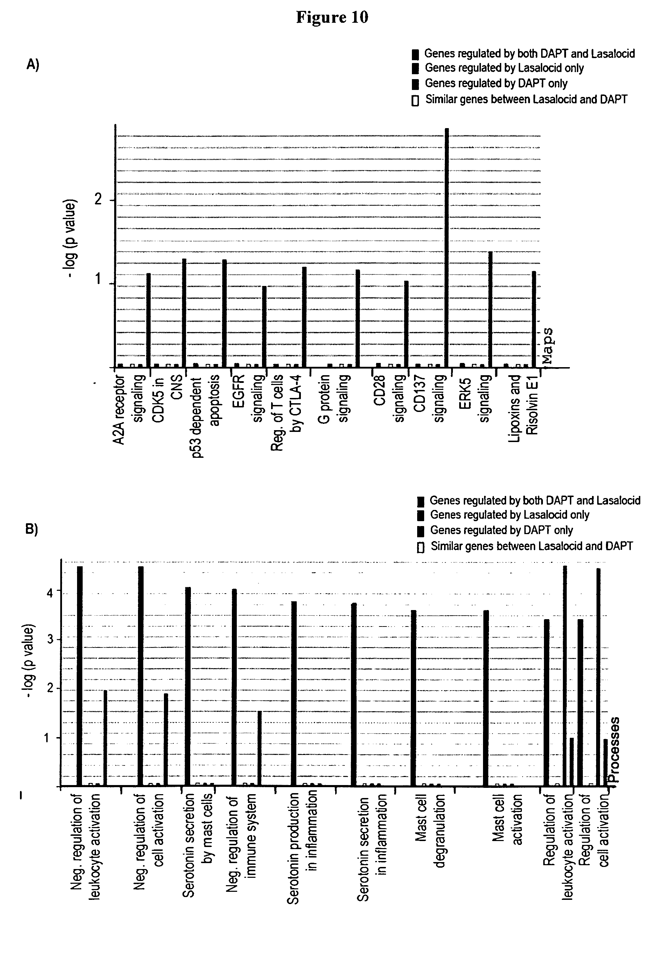

Figure 10 shows GeneGo analyses of Lasalocid and DAPT regulated genes in KOPTK1 cells: GeneGo analyses were curried out using genes significantly represented in Lasalocid and DAPT ucated samples (fold change -2, pvalue < 0.05). Black bars represent genes significantly regulated by both Lasalocid and DAPT. Orange bars represent genes regulated by Lasalocid only and blue bar represents genes regulated uniquely by DAPT in a given pathway or cellular processes. White bars represents genes that may be similar in both Lasalocid and DAPT treated cells. The graphs were drawn using-log ( pvalue ) on Y-axis and X-axis shows networks and pathways. A) Lasalocid causes statistically significant alterations of genes involved in several pathways such as A2A receptor signaling, p53 dependent apoptosis, EGFR signaling, CD137 signaling and ERK5 signaling. B) Lasalocid and DAPT regulates several common genes involved in cellular processes such as leukocyte activation, Serotonin production and secretion, regulation of immune system and Mast cell activation (all black bars). DAPT regulates additional genes involved in leukocyte activation and cell activation processes.

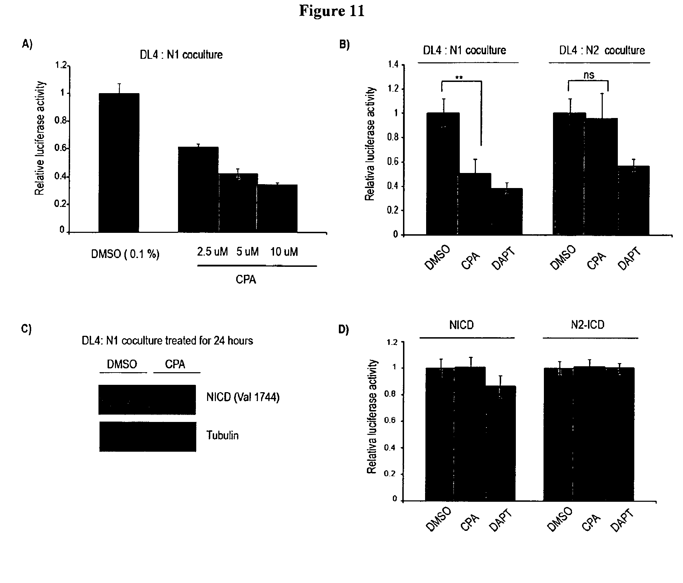

Figure 11 shows CPA inhibits Notch1 signaling in a concentration dependent manner. A) N1-HeLa cells were co-transfected Notch1 expression plasmid, 12xCSL luciferase and SV40 renilla plasmids. DL4- and N1-HeLa cells were cocultured in a 96 well plate in 1:1 ratio (20,000: 20,000 cells/well) and treated with DMSO or 2.5, 5 and 10 µM of CPA for 24 hours. The Notch pathway activation was measured by quantifying Notch signaling driven luciferase activity. B) DL4:N1 and DL4:N2 coculture assay was treated with CPA and DAPT (each 10 µM) for 24 hours. The effect of CPA and DAPT on DL4-N1 and DL4-N2 driven pathway activation was measured by Notch driven luciferase activity quantification. C) DL4:N1 coculture assay was treated with 10µM of CPA for 24 hours. The NICD protein levels were determined by western blotting using Val1744 antibody. D) HeLa cells were transfected with plasmids expressing Notch1-intracellular domain (NICD) or Notch2-intracellular domain (N2-ICD). The Notch signaling activation was measured by introduction of 12xCSL luciferase plasmid and SV40 renilla served as an internal control. CPA and DAPT treatment of NICD or N2-ICD expressing cells docs not block pathway activation. P values were determined using student's two tailed t test. ** = p value <0.05.

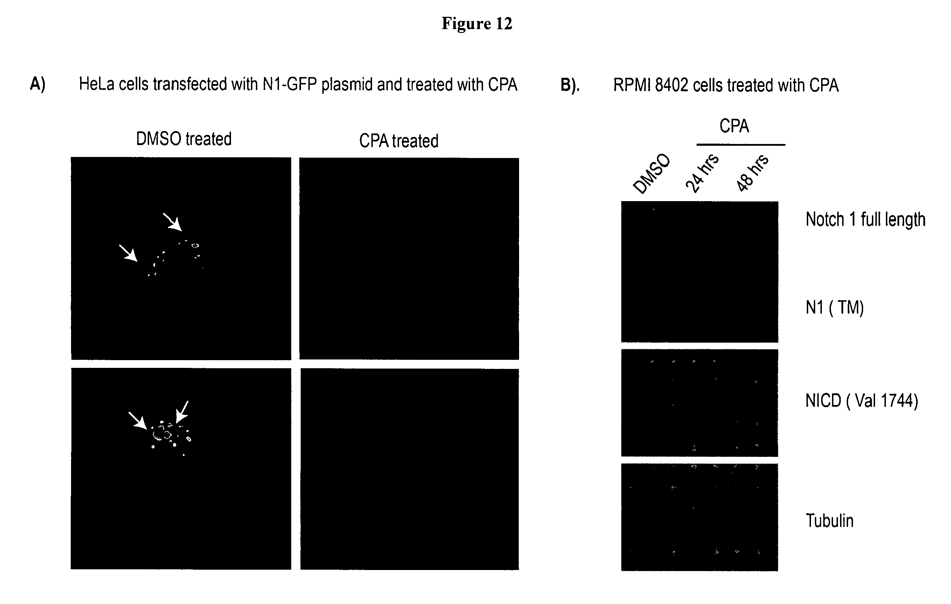

Figure 12 shows CPA treatment blocks S1 cleavage and membrane trafficking of the Notch1 receptor. A) HeLa cells were transfected with Notch1-GFP plasmid and treated with DMSO or CPA for 24 hours. Notch1-GFP fusion Protein localization was determined by fluorescence microscopy by tracing GFP signal CPA treatment leads to a loss of punctate signal, which is present in the DMSO treated cells. B) Human leukemic cell line RPM1 8402 was treated with DMSO or CPA for 24 and 48 hours. Whole cell lysate was analyzed by western blotting. Full length and membrane bound intracellular domain of Notch1 was detected using antibodies directed against the C-terminus of Notch1 protein. Active form of Notch1, NICD was detected using Val1744 antibody that detects specifically cleaved form. An antibody against tubulin was used to detect the levels of tubulin as a loading control. Treatment of RPMI 8402 cells with CPA leads to a loss of membrane bound intracellular domain of Notch1 and NICD.

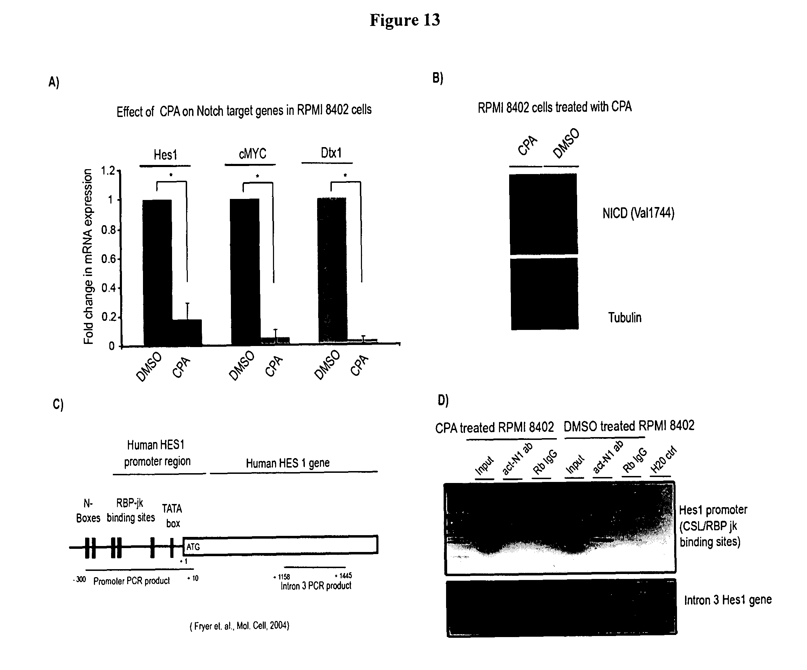

Figure 13 shows CPA treatment induces downregulation of Notch target genes. A) The human T-ALL cell line RPMI 8402 was treated with DMSO and 10 µM CPA for 24 hours. Following total RNA extraction and cDNA synthesis, qRT-PCR was carried out to quantitate the transcript levels of Notch target genes Hes1, cMyc and Dtx1. Data normalized to HPRT as a house-keeping gene. B) RPMI 8402 cells treated with DMSO or 10 µM CPA were analyzed by western blot for the expression levels of NICD using Val1744 antibody. CPA treatment led to a downregulation of NICD in RPMI 8402 cells. C) Schematic diagram of CSL/RBP-jk binding sites in Hes1 promoter. Human Hes1 promoter contains two CSL/RBP-jk binding sites in a head to head conformation (red bars), Primer sequences were designed to amplify the Hes1 promoter region containing CSL/RBP-jk binding sites. D) RPMI 8402 cells were treated with CPA and DMSO for 24 hours. Cells were fixed and sonicated to shear the genomic DNA. Val1744 antibody was used to pull down NICD bound chromatin DNA. Rabbit IgG was used as a negative control. PCR amplification of intron 3 of Hes1 genes also served as an internal control for the specificity of Val1744 antibody. Chromatin Immunoprecipitation analyses of Hes1 promoter in RPMI 8402 cells showed absence of NICD recruitment to the CSL/RBP-jk binding sites upon treatment with CPA. Act-N1 ab= val1744 antibody against NICD, Rb IgG- rabbit IgG. * = p value < 0.005.

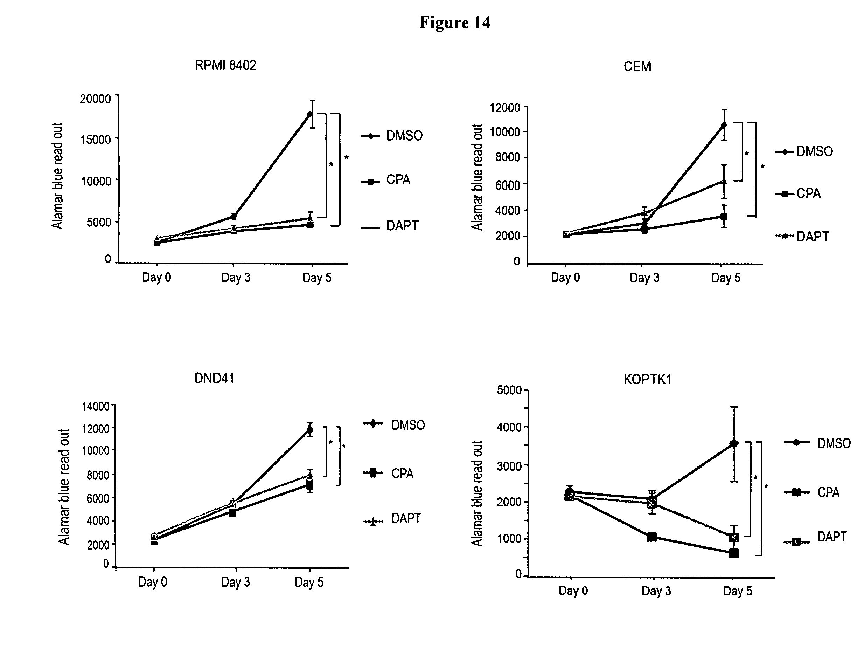

Figure 14 shows CPA treatment blocks proliferation of T-ALL cell lines. T-ALL cell lines, namely RPMI 8402, CEM, DND41 and KOPTK1 were treated in a 96 well plate (8 replicates for each time point/ treatment group) with 10µM concentration of CPA and DAPT. The growth kinetics of control and compound treated samples were determined hy Alamar blue readout at day 0, day 3 and day 5. The growth curves were compared with DMSO treated cells. The CPA treatment causes a growth arrest phenotype when compared to DMSO treated cells. In case of CEM and KOPTK1, CPA has more pronounced effect on proliferation when compared to DAPT. * = p value < 0.005.

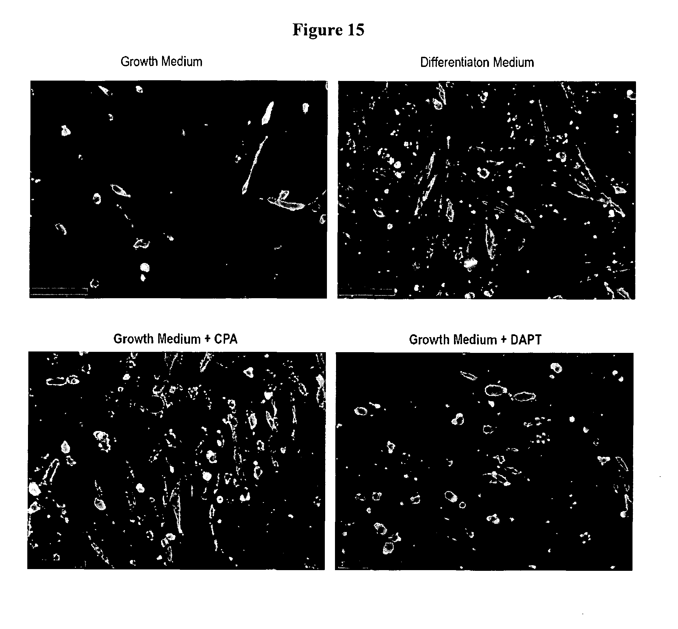

Figure 15 shows CPA induces myoblast differentiation. C2C12 cells were seeded on cover slips and gown in the presence of 10% scrum (growth media) supplemented with DMSO or 10 µM of CPA. As a positive control C2C12 cells were also grown in differentiation medium containing 2% horse serum and in the presence of DAPT to induce differentiation and myotubes formation. Cells were allowed to grow to 100% confluency. On day 3, cells were fixed, blocked with 1% BSA and stained with an antibody against Myosin heavy chain (MIIC). An Alexa 488 conjugated (green colour) secondary antibody was used to detect MHC positive cells. The cell nuclei were stained with DAP1. Immunofluorescence microscopy showed an accumulation of MHC positive multinucleated myotubes in CPA treated cells compared to myoblast grown in the growth medium. Treatment with DAPT and growth in differentiation medium also induced myoblast differentiation into myotubes.

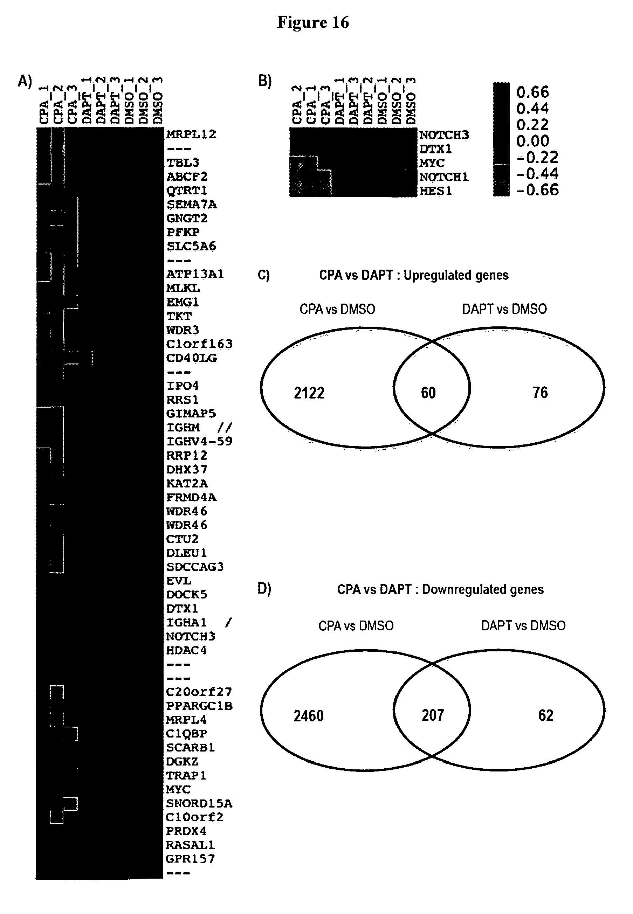

Figure 16 shows Clustering analyses of gene expression profile of CPA treated KOPTK1 cells. Cluster 3.0 software was used to perform clustering analyses. These analyses contains all the genes with pvalue < 0.05 irrespective of their fold change in the expression level. A) A list of 56 genes commonly downregulated by CPA and DAPT treatment of KOPTK1 cells. B) List of genes involved in Notch signaling following CPA, DAPT and DMSO treatment of KOPTK1 cells. C) Venn diagram of upregulated genes following CPA and DAPT treatment. CPA treatment led to an upregulation of 2122 genes and DAPT treatment led to an upregulation of 76 genes, while both compounds also caused an upregulation of 60 genes common to both treatments. D) Venn diagram of genes that were downregulated after treatment with CPA and DAPT when compared to DMSO treatment. Treatment with CPA and DAPT led to a downregulation of 207 genes common to both treatments. CPA treatment also caused a downregulation of 2460 genes unique for this treatment, white DAPT led to a downregulation of 62 unique genes.

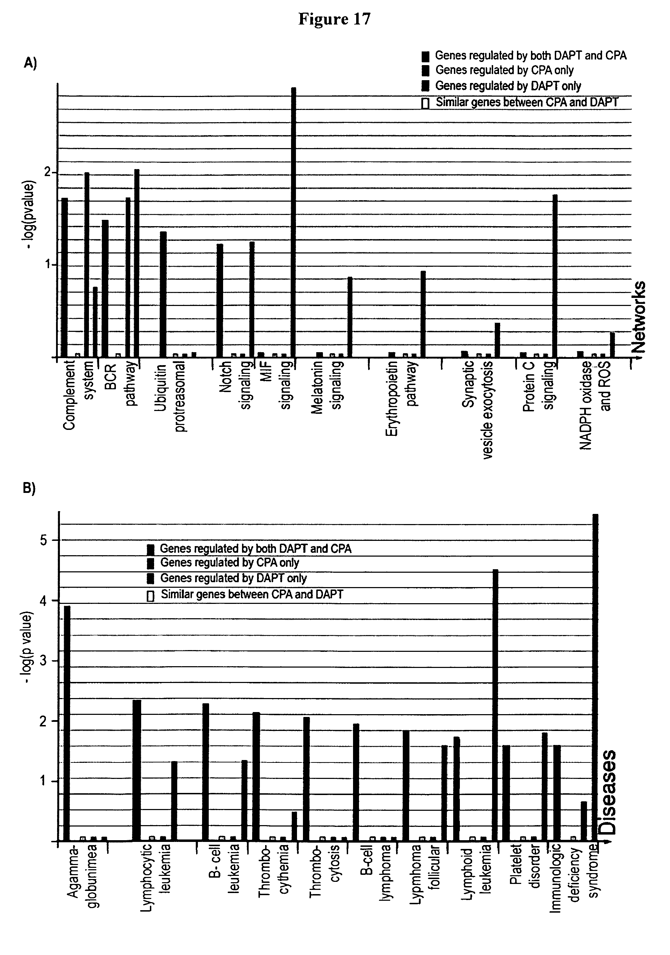

Figure 17 shows GeneGo comparison of genes regulated by CPA and DAPT. GeneGo analyses were carried out using genes significantly represented in CPA and DAPT treated samples (fold change = 2, pvalue < 0.05). Black bars represent, genes significantly regulated by both CPA and DAPT. Orange bars represent genes regulated by CPA only and blue bar represents genes regulates uniquely by DAPT in a given pathway or network. White bars represents genes that may be similar in both CPA and DAPT treated cells. The graphs were drawn using -log (pvalue) on Y-axis and X-axis shows networks and pathways. A) CPA and DAPT treatment led to an enrichment of genes common to both treatment and implicated in complement, BCR, Ubiquitin and Notch signaling (black bars). The Complement and BCR pathways also exhibit an enrichment of genes unique to DAPT treatment (blue bars), while Notch, MIF and protein C signaling led to an accumulation of CPA specific genes.

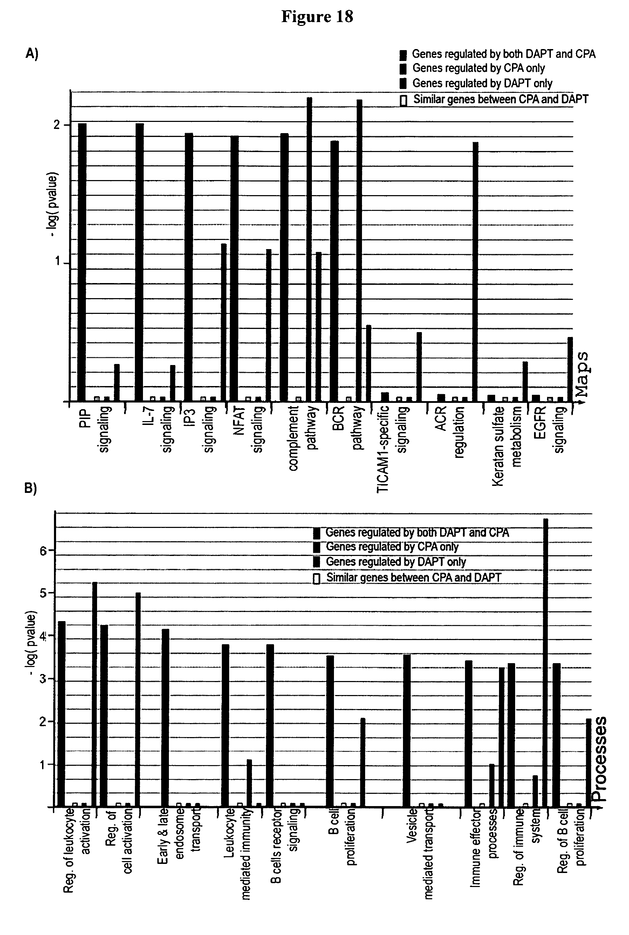

Figure 18 shows GeneGo analyses of CPA and DAPT regulated genes GeneGo analyses were carried out using genes significantly represented in CPA and DAPT treated samples (fold change = 2, pvalue < 0.05). Black bars represent genes significantly regulated by both CPA and DAPT. Orange bars represent genes regulated by CPA only and blue bar represents genes regulated uniquely by DAPT in a given pathway or cellular processes. White bars represents genes that may be similar in both CPA and DAPT treated cells. The graphs were drawn using-log (pvaluc) on Y-axis and X-axis shows networks and pathways. A) Black bars represent statistically significant genes regulated by CPA and DAPT in PIP, IL-7, IP3, NFAT, complement and BCR pathways. B) Lcukocyte activation, endosome transport, vesicle transport, B cell proliferation and B cell receptor processes enriched for genes regulated by CPA and DAPT.

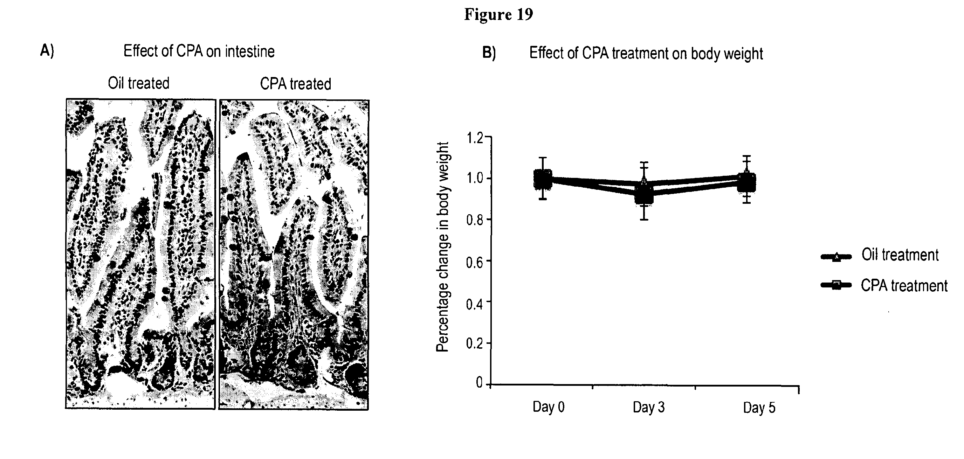

Figure 19 shows CPA treatment does not perturb intestinal homeostasis. A) Mice (n=3) were treated with CPA (10 mg/kg) or oil as control for 7 days. Intestinal tissues were collected and analyzed for goblet cell metaplasia. CPA treatment docs not lead to an altered number of goblet cells per villi. Eosin staining shows a normal architecture of the intestine. B) Mice (n=4) were treated with oil or CPA (10 mg/kg) for 5 consecutive days and the changes in their body weights were recorded. Treatment of mice with CPA docs not lead to a significant weight loss.

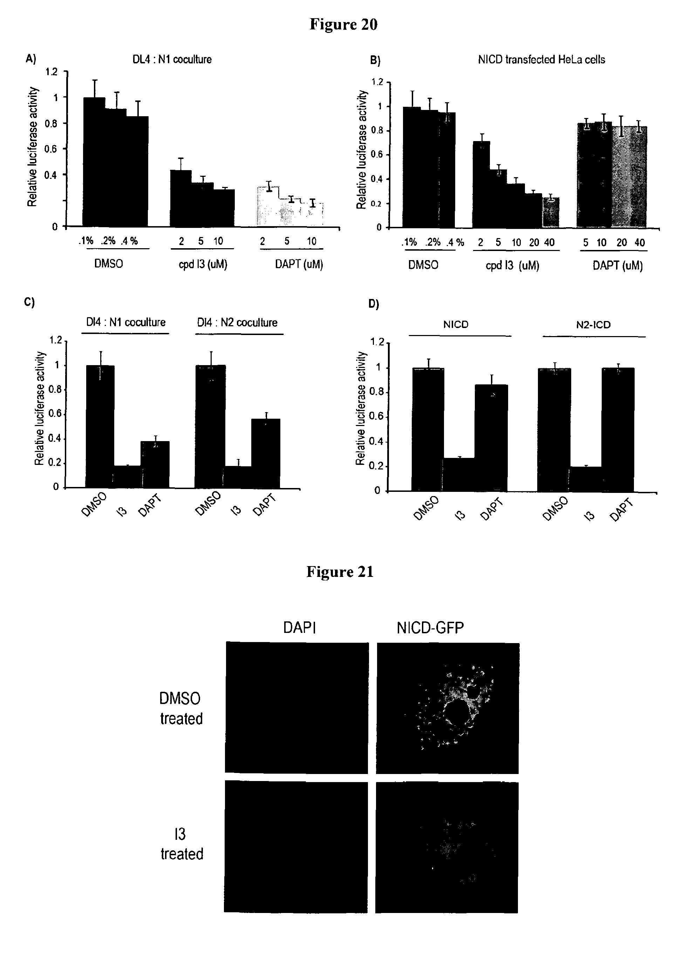

Figure 20 shows I3 blocks NICD mediated Notch signaling activation. A) N1-HcLa cells were co-transfected with Notch1 expression plasmid, 12xCSL luciferase and SV40 rcnilla plasmids, DL4- and N1-HeLa cells were cocultured in a 96 well plate in 1:1 ratio (20,000: 20,000 cells/well) and treated with DMSO or with 2, 5 and 10 µM of 13 and DAPT for 24 hours. The Notch pathway activation was measured by quantifying Notch signaling driven luciferase reporter assay. Treatment of DL4:N1 coculture assay with I3 and DAPT causes a concentration dependent decrease in Notch signaling activation. B) HeLa cells were transfected with NICD and treated with DMSO or with 2, 5, 10, 20 and 40 µM or I3. As a control cocultured cells were also treated with 5, 10, 20 and 40 µM of DAPT. The pathway activation was measured using Notch driven luciferase reporter assay. 13 treatment of NICD expressing cells led to an attenuation of the signaling, while DAPT treatment had no effect on Notch signaling activation mediated by NICD. C) DL4:N I and DL4:N2 coculture assay was treated with 13 and DAPT (each 10 µM) for 24 hours. The effect of 13 and DAPT on DL4-N1 and DL4-N2 driven pathway activation was measured by Notch driven luciferase activity. Both 13 and DAPT treatment block Notch1 and Notch2 induced pathway activation. D) 13 inhibits pathway activation via intracellular domains of Notch1 (NICD) and Notch2 (N2-ICD).
Figure 21 shows I3 does not block nuclear localization of NICD. HeLa cells were transfected with NICD-GFP expression plasmid and were treated with DMSO or 13 (10µM) for 24 hours. Immunofluorescence microscopy was used to detect the localization of NICD-GFP fusion protein. DAPI was used to stain the nuclei. NICD-GFP fusion protein localizes in the nucleus in a diffused manner and I3 treatment did not alter its nuclear localization. Pictures were taken with Axioplan microscope using 100x objective.

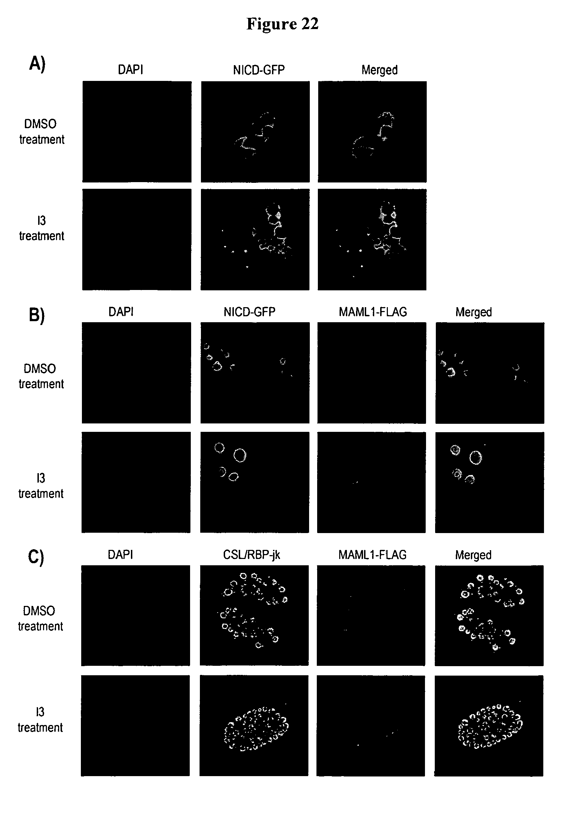

Figure 22 shows I3 treatment does not perturb colocalization of NICD-CSL/RBP-jk-MAML1, A) HeLa cells were transfected with NTCD-GFP expression plasmid and treated with DMSO and I3 (10µM) for 24 hours. Immunofluorescence microscopy showed a diffused nuclear localization of NICD-GFP, which was not altered upon 13 treatment. B) NICD-GFP (800 ng) and MAML1-FLAG (1µg) was co-expressed in HeLa cells. NTCD-GFP was detected by tracing GFP protein and MAML1-FLAG was detected by using anti-FLAG antibody. Immunofluorescence microscopy showed a redistribution of NICD-GFP and MAML1 protein into the sub-nuclear compartment that was not altered by I3 treatment C) Endogenous CSL/RBP-jk was detected using anti-CSL/RBP-jk antibody and MAML1-FLAG was detected with anti-FLAG antibody. Both proteins co-localized into a sub nuclear compartment. This colocalization of MAML1 and CSL/RBP-jk was not perturbed by I3. Pictures were taken using Axioplan microscope with 100x objective.

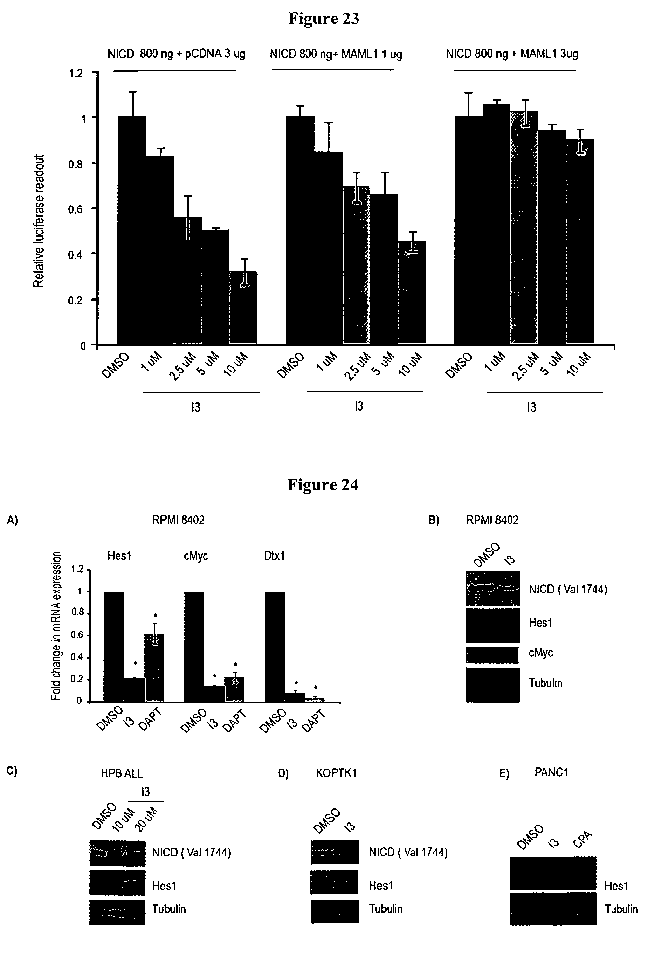

Figure 23 shows T3 mediated inhibition of Notch signaling can be rescued with increasing concentration of MAML1. HeLa cells were co-transfected 800ng of NICD + 3µg of pCDNA3.1 or 800ng of NICD + 1µg of MAML1-FLAG or 800ng of NICD + 3µg of MAML1-FLAG expression vectors. To measure Notch pathway activation, 12xCSL luciferase plasmid was also introduced into the cells. SV40 renilla was used as an internal control. Cells transfected with different combinations and amounts of plasmid were treated with DMSO or increasing concentration of 13 (1, 2.5, 5 and 10 µM) for 24 hours. 12xCSL driven luciferase activity was measured using dual luciferase assay system. In the absence of MAML1, 13 could block Notch signaling activation, but Notch inhibitory effect of 13 was diminished with increasing amount of MAML1.
Figure 24 shows 13 inhibits Notch signaling and downregulates its target genes in human cancer cell lines. A) RPMM 8402 cells were treated with DMSO, I3 and DAPT (10 µM) for 24 hours and analyzed for the expression of Notch target genes, Hes1, cMyc and Dtx1 by qRT-PCR. Data normalized to HPRT us a house-keeping gene. B) Whole cell lysate from I3 treated cells was analyzed by Western blot. Using antibodies against NICD (Val1744), Hes1 and cMyc, the protein levels of NICD and Notch target genes were determined. C and D) The human T-ALL cell lines HPB ALL and KOPTK1 were treated with DMSO or I3 for 24 hours. Western blot analyses were performed using NTCD (Val1744) and Hes1 specific antibodies. Tubulin served as a loading control. E) Whole cell lysate from DMSO, I3 and CPA treated cells were analyzed by Western blot. Hes1 protein levels were determined using Hes1 specific antibodies. Statistical analyses were done using student's two-tailed t.test. * = p value < 0.05.

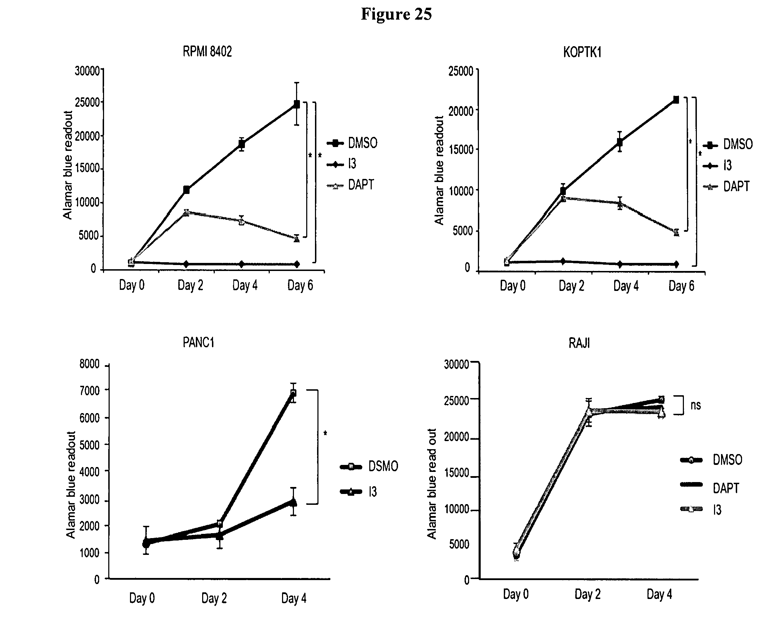

Figure 25 shows T3 induces a proliferative block in human cancer cells. Human T-ALL cell lines RPMI 8402 and KOPTK1, and pancreatic cancer cell line PANC1 were seeded in a 96 well plate and treated with 10 µM concentration of 13 and DAPT for several days. Their growth inhibitory effects were compared with cells treated with equal amount of DMSO. Using Alamar blue assay, the growth kinetics of RPMI 8402 and KOPTK1 were followed for upto 6 days, while PANC1 and RAJI cells were monitored for 4 days. 13 treatment of RPM1 8402, KOPTK1 and PANC1 cells caused a significant reduction in their growth potential. Treatment of RAJ1 cells (Notch-independent) with I3 and DAPT did not cause a block in their proliferation. Statistical analyses were done using student's t.test. ^{‡} = p value < 0.05. ns= not significant.

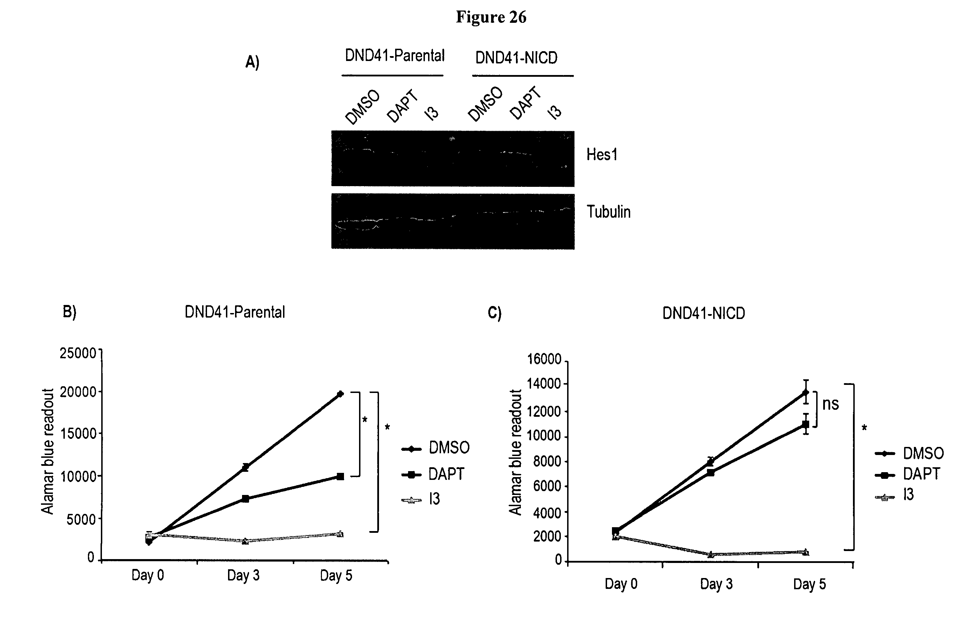

Figure 26 shows 13 blocks NICD dependent growth of human cancer cells. A) DND41 - Parental and DND41-NICD cells were treated with I3 and DAPT for 24 hours. Western blot analyses were carried out for Hes1 protein using Hes1 specific antibodies. Both DAPT and I3 caused a downregulation of Hes1 in DND41-Parental cells. DND41-NICD cells showed a downregulalion of Hes1 only when treated with I3.B) Five thousand DND41-Parental cells were seeded and treated with DMSO, 13 and DAPT in a 96 well plate. Growth kinetics of the parental cell line was followed over 5 days using Alamar blue readout. Treatment of DND41-Parental cell line with both I3 and DAPT caused a proliferation arrest. C) Similarly, DND41-NICD cells treated with DMSO, I3 and DAPT and their growth kinetics were monitored using Alamar blue readout over 5 days. The treatment of DND41-NICD cells with DAPT did not have a significant impact on their proliferation, while 13 treatment induced a proliferation arrest. P values were calculated using Student's t.test. * = p value < 0.05. ns= not significant.

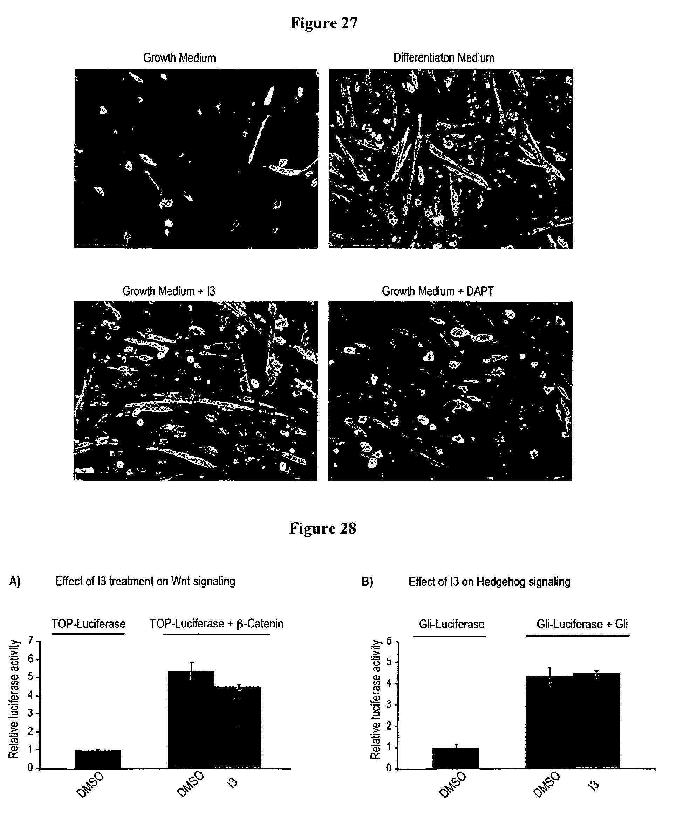

Figure 27 shows I3 mediated induction of myoblast differentiation. C2C12 myoblasts were plated on coverslip and grown to 100% confluency in the presence of growth medium or DAPT and 13 for 3 days. Cells were also grown in the differentiation medium (2% horse serum) as a positive control. Myoblast differentiation was determined by staining with an antibody against Myosin heavy chain (MHC). An Alexa 488 conjugated (green colour) secondary antibody was used to detect MHC positive cells. The cell nuclei were stained with DAPI. Immunofluorescence microscopy showed an abundance of MHC positive multinucleated myotubes in 13 and DAPT treated C2C12 cell cultures compared to cells cultured in the growth media alone. C2C12 cells cultured in differentiation medium or treated with DAPT served as a positive control for differentiation phenotype.
Figure 28 shows I3 does not impede upon Wnt and Hedgehog signaling. A) Activation of Wnt signaling in HeLa cells. HeLa cells were transfected with TCF/LEF luciferase plasmid or cotransfected with TCF/LEF luciferase and β-catenin expression plasmid to measure Wnt signaling. Cultures were treated with DMSO or 10 µM of I3 for 24 hours. SV40 rcnilla readout served as a transfection control. Measurement of luciferase activity by dual luciferase assay system showed an upregulation of Wnt signaling upon transient introduction of β-catenin in HeLa cells, which was resistant to 13 treatment. B) Hedgehog pathway activation in HeLa cells. HeLa cells were transfected with a plasmid encoding For luciferase downstream of a promoter containing Gli-1 binding site to measure the Hedgehog pathway activation. Hedgehog signaling was induced by transient introduction of Gli-1 transcription factor. Treatment of cell cultures with I3 did not attenuate Hedgehog signaling activation in HcLa cells. TOP- Optimal TCF binding site.

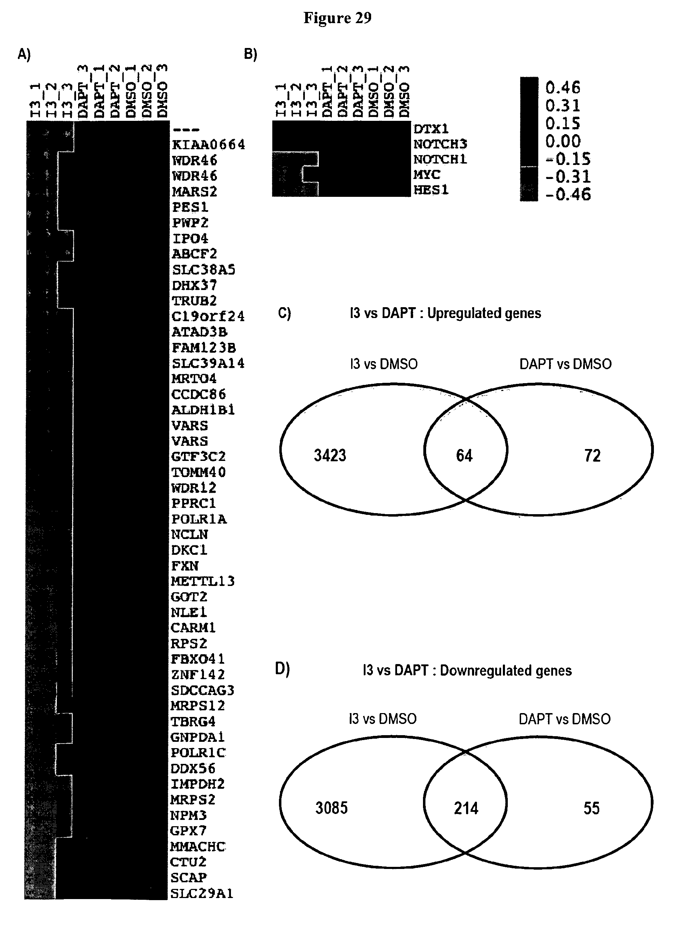

Figure 29 shows Clustering and Venn diagram of 13 and DAPT regulated genes in KOPTK1 cells. A and B) Cluster 3.0 software was used to perform clustering analyses. Analyses were carried out using a list of differentially regulated genes with a p value <0.05. Red colour indicates an upregulation and green colour represents a downregulation of the gene expression in the heat map analyses. A) Heat map analysis of top of 50 genes downregulated by both 13 and DAPT. B) Heat map analysis of genes involve in Notch signaling following 13, DAPT and DMSO treatment. C and D) Venn Diagrams were prepared using all the genes with a p value < 0.05, irrespective of their fold change in the expression level between different treatments. C) Venn diagram of upregulated genes following I3 and DAPT treatment compared to DMSO. 13 treatment led to an upregulation of 3423 genes and DAPT treatment led to an upregulation of 72 genes, while both compounds also caused an upregulation of 64 genes. D) Venn diagram of genes that were downregulated after treatment with I3 and DAPT when compared to DMSO treatment. Treatment with I3 and DAPT led to a downregulation of 214 genes common to both treatments, I3 treatment also caused a downrcgulation of 3085 genes unique for this treatment, while DAPT led to a downregulation of 55 unique genes.

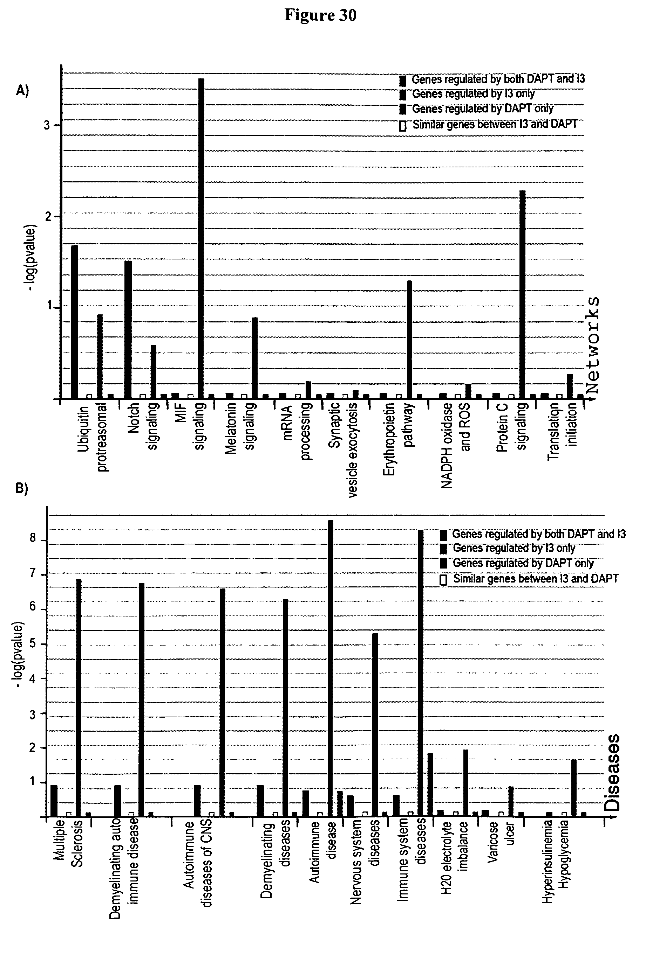

Figure 30 shows GeneGo analyses of I3 and DAPT regulated gene expression. GeneGo analyses were carried out using genes significantly represented in 13 and DAPT treated samples (fold change = 2, p value < 0.05). Black bars represent genes significantly regulated by both 13 and DAPT. Orange bars represent genes regulated by I3 only and blue bar represents genes regulated uniquely by DAPT in a given pathway or network. White bars represents genes that may be similar in both 13 and DAPT treated cells. The graphs were drawn using -log (p value) on Y-axis and X-axis shows networks and pathways. A) I3 and DAPT treatment led to an enrichment of genes implicated in Ubiquitin and Notch signaling (black bars), while 13 also regulates MIF and protein C signaling (orange bars). B) KOPTK1 cells treated with I3 enriched for genes involved in multiple sclerosis, Demyelinating disease, and autoimmune disease of central nervous system and immune diseases (orange bars).

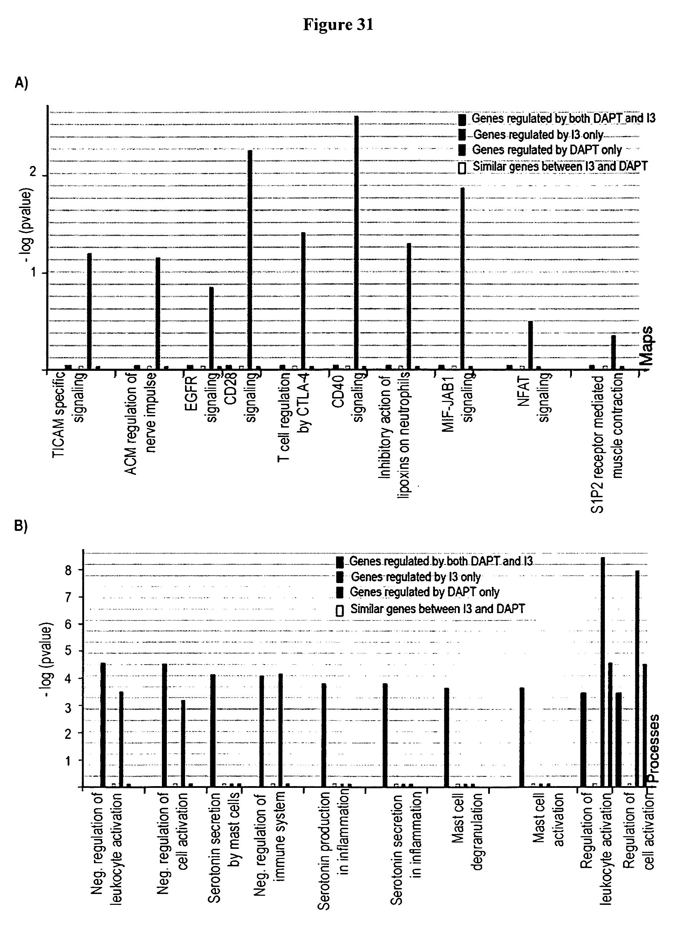

Figure 31 shows GeneGo analyses of I3 and DAPT regulated gene expression in KOPTK1 cells. GeneGo analyses were carried out using genes significantly represented in 13 and DAPT treated samples (fold change = 2, p value < 0.05). Black bars represent genes significantly regulated by both 13 and DAPT. Orange bars represent genes regulated by 13 only and blue bar represents genes regulated uniquely by DAPT in a given pathway or network. White bars represents genes that may be similar in both I3 and DAPT treated cells. The graphs were drawn using log (p value) on Y-axis and X-axis shows networks and pathways. A) 13 treatment of KOPTK1 cells significantly altered the expression profile of genes involved in several pathways (orange bars). B) KOPTK1 cells enriched with genes regulated by both I3 and CPA and involved in several cellular processes (black bars).

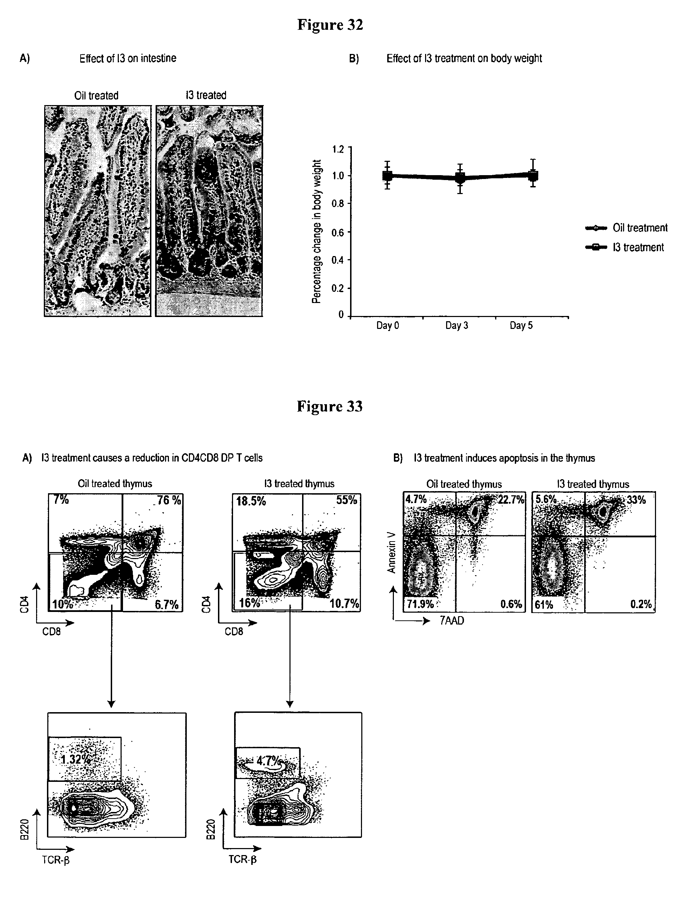

Figure 32 shows 13 effect on the intestinal tissue. A) C57BL6 mice (n=3) were treated (I.P injections) with oil as a control or with 25 mg/kg of I3 for 7 days. Tissues were analyzed on day 8 by Alcian blue staining of the intestine sections. Alcian blue stains for mucous producing goblet cells. B) Mice (n=4 for each treatment) were treated with oil or 25 mg/kg of 13 (i.p injections) for 5 consecutive days. Body weights were measured on day 0, day 3 and day 5. Average body weights were calculated for each group. The average body weights were set to 100% at day 0. The fluctuations in the body weights were determined as a percentage of gain or loss of body weight compared to day 0.
Figure 33 shows I3 treatment causes apoptosis in the thymus. A) C57B16 mice were treated with oil or 25 mg/kg of I3 for 7 consecutive days. On day 8, thymus were harvested and analyzed for T cell development using fluorochrome-conjugated antibodies against CD4 and CD8. Ectopic B cell development in the thymus was determined by gating on DN compartment. Cells were stained for B220, a B cell marker using pacific blue conjugated anti-B220 antibody. B) Thymocytes were stained for AnnexinV and with 7AAD to quantitatc apoptotic cells by flow cytometric analyses.

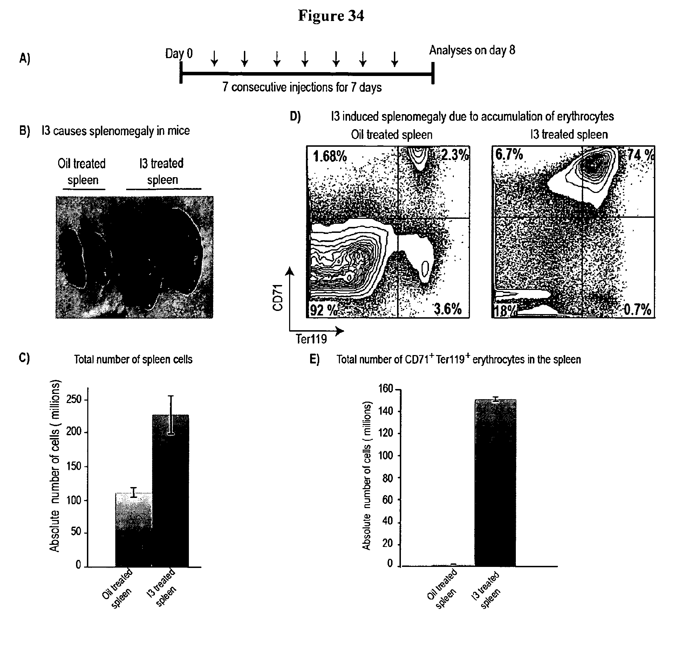

Figure 34 shows Mice treated with 13 develop splenomegaly. A) Schematics of frequency of I3 injections (I.P) and analyses time points. The mice (n= 2 for each treatment) were injected with oil or 25 mg/kg of I3 for 7 consecutive days (one injection per day) and were analyzed on day 8. B) Gross morphology and size of the spleen following oil or 13 treatment. C) Total numbers of cells in the spleen following oil or 13 treatment. D) Flow cytometry analyses using antibodies against erythrocyte markers, CD71 and Ter119 (gated on live cells). E) Absolute numbers of erythrocytes following oil or 13 treatment. Absolute numbers of erythrocytes were deduced using data from flow cytometry analyses and the total numbers of spleen cells.

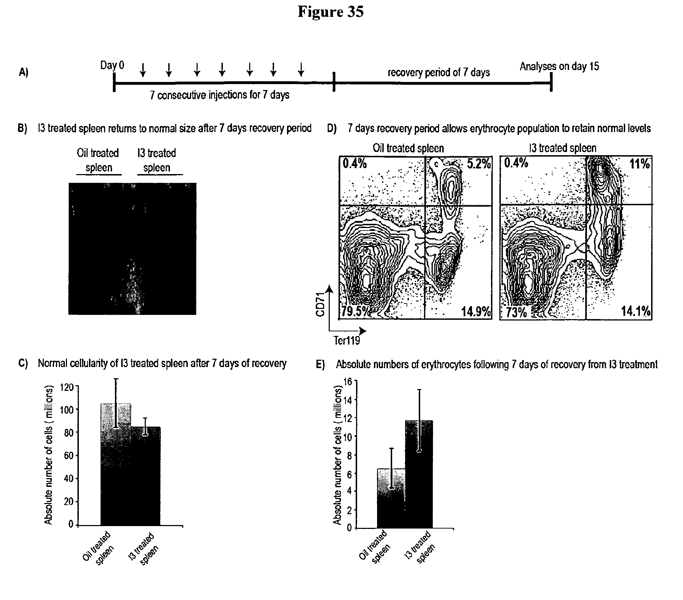

Figure 35 shows I3 driven splenomegaly is reversible. A) Schematics of the experiment. Animals (n= 2 for each treatment) were i.p injected with oil or 25 mg/kg of I3 for seven consecutive days (one injection per day) and let to recover for another week. Animals were then analyzed on day 15 for splenomegaly and accumulation of erythrocytes in the spleen. B) Gross morphology and size of the spleen. C) Total numbers of cells in the spleen. D) Flow cytometry analyses using antibodies against erythrocyte markers, CD71 and Ter119 (gated on live cells). E) Absolute numbers of erythrocytes following oil or 13 treatment. Absolute numbers of erythrocytes were deduced using data from flow cytometry analyses and the total numbers of spleen cells.

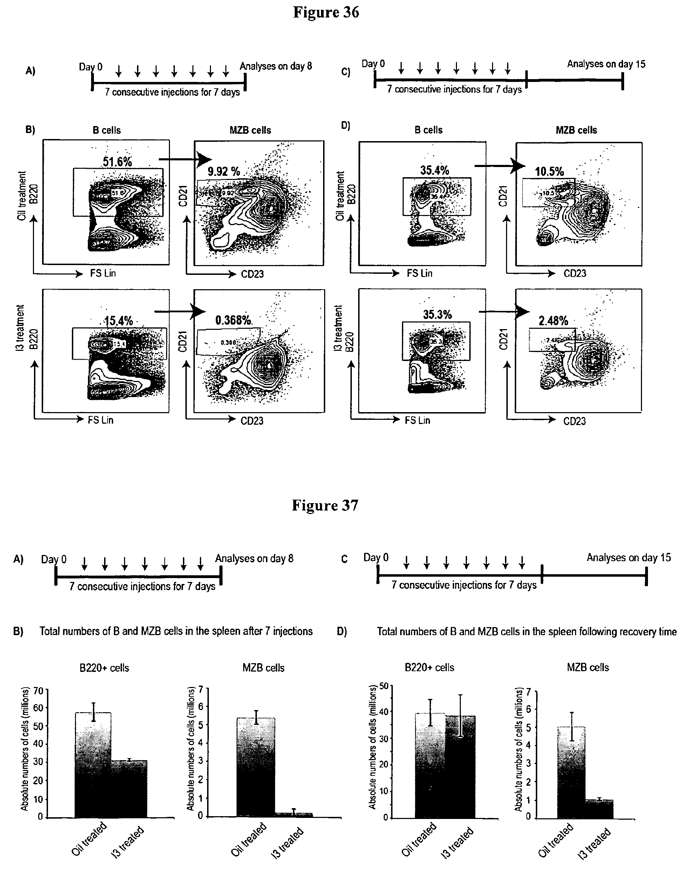

Figure 36 shows 13 treatment leads to a reduction in MZB cells in the spleen. A) Schematics of the experimental plan. B) Mice (n=2) were treated with oil or 25 mg/kg of I3 for 7 consecutive days. Spleens were analyzed on day 8. Using B220 specific antibodies, B cells in the spleen were identified. MZB cells within the B cell compartment were detected using antibodies against CD23 and CD21 cell surface markers. C) Schematics of 13 treatment followed by a recovery phase. D) 13 and oil treated mice (n=2) were allowed to recover for one week. B cell and MZB cell development was analyzed as described above.
Figure 37 shows Loss of MZB cells induced by 13 is not due to the loss of B cell compartment. A) Schematics of experimental plan without a recovery phase. B) Absolute numbers of B220⁺ and MZB cells following 13 treatment. C) Experimental plans with incorporation of recovery phase. D) Absolute numbers of B cells and MZB cells were calculated from oil and 13 treated mice following a recovery period of one week.

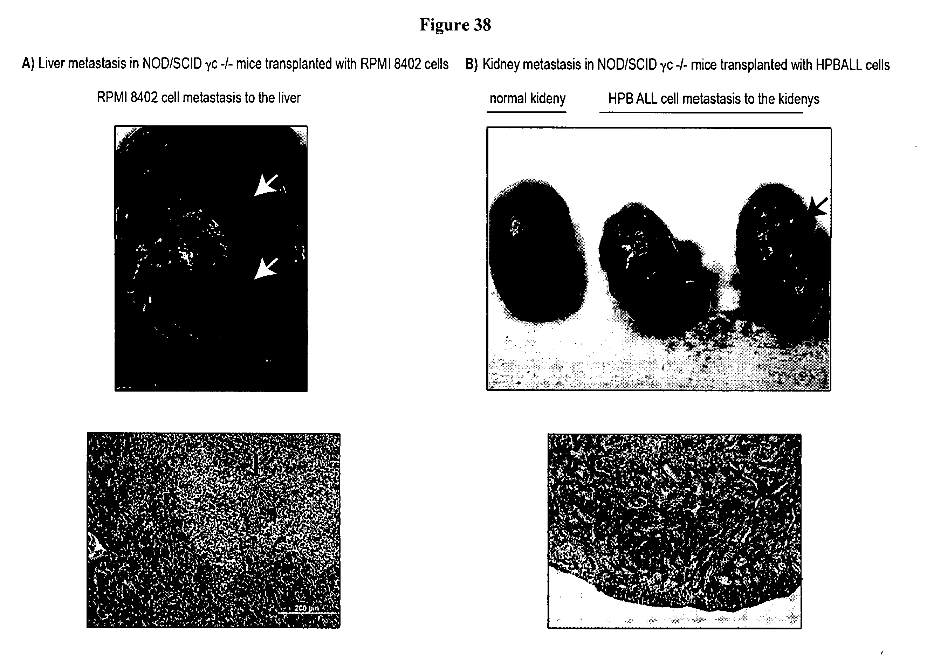

Figure 38 shows Liver and kidney metastasis in RPMI 8402 and HPB ALL transplanted mice. A) Gross morphology of mouse liver infiltrated by RPMI 8402 leukemic cells. Leukemic cells invading liver tissue appear as white spots on the liver (marked with yellow arrows). Lower left panel shows hcmotoxylin and eosin staining of liver sections. I = Leukemia cell infiltration in the liver. B) Gross morphology of mouse kidney infiltrated by HPB ALL human leukemic cells. Leukemic cells invading kidney tissue marked with black arrows. Lower left panel shows hemotoxylin and cosin staining of kidney section. I = HPB ALL infiltration into kidney.

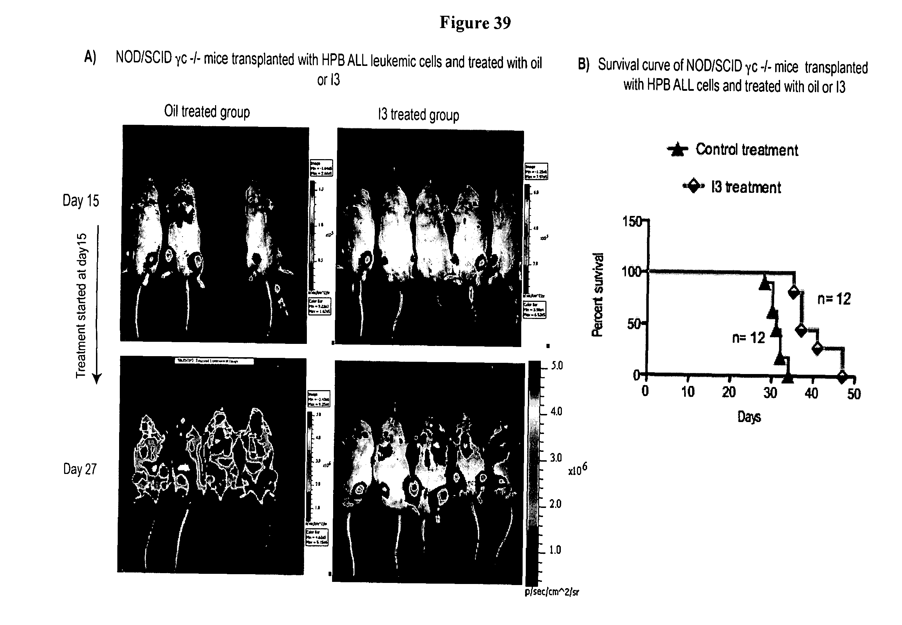

Figure 39 shows I3 treatment increases latency of leukemia development in mice. A) NOD/SCID γc^{-/-} mice were injected with 1 x 10⁶ HPB ALL (luciferase expressing) cells. On day 15, leukemic cells were established in the bone marrow. Mice were treated with oil or 13 for every day. Mice were imaged on day 27 using Caliper IVIS (Xenogen) live imaging system. Red and blue colour indicates the intensity of luciferase signal and correlates with the number of leukemic cells. B) Survival curve of NOD/SCID γc-/- mice transplanted with HPB ALL leukemic cells and treated with oil or 13 chemical compound. Each group consists of 12 animals. Mice were i.v injected with 1 x 10⁶ HPB ALL cells (luciferase expressing cells). Tumor development was monitoring by Caliper IVIV (Xenogen) live imaging system. On day 15, a daily treatment regimen was started with oil or 13.

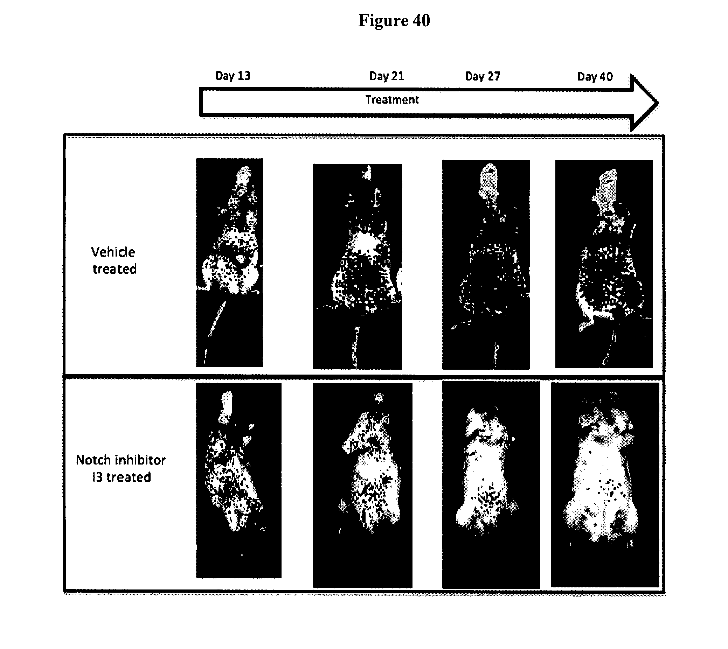

Figure 40 shows I3 treatment blocks RPMI 8402 leukemic cell growth in xenotransplantation assay. NOD/SCID γc^{-/-} mice were transplanted with 5 x 10⁵ RPMI 8402 (luciferase expressing) cells. Leukemia development was followed using Caliper IVIS (Xenogen) live imaging system. On day 13, a daily treatment was started using oil (n=3) or 13 (n-4). Animals were treated for 27 days (end point of the experiment).

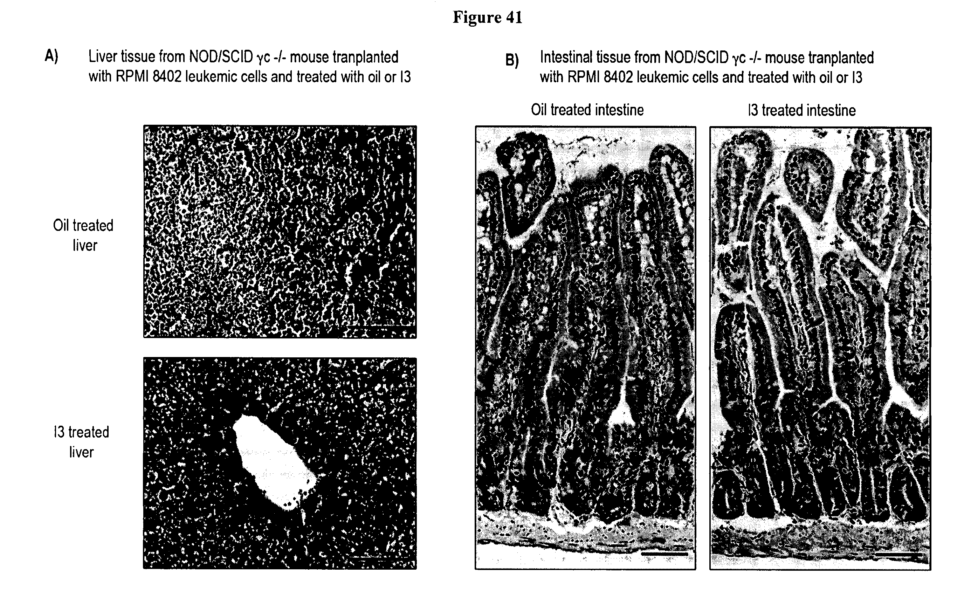

Figure 41 shows NOD/SCIDγc^{-/-} mice transplanted with RPMI 8402 leukemic cells. A) IIemotoxylin and Eosin stains of liver sections from NOD/SCIDγc^{-/-} mice transplanted with RPMI 8402 leukemic cells and treated with oil or I3. I = RPMI 8402 leukemic cell infiltration into liver. B) Alcian blue staining on paraffin embedded intestinal sections from NOD/SCIDγc^{-/-} mice treated with oil or 13 for 27 days, Alcian stains mucous producing goblet cells (blue colored).

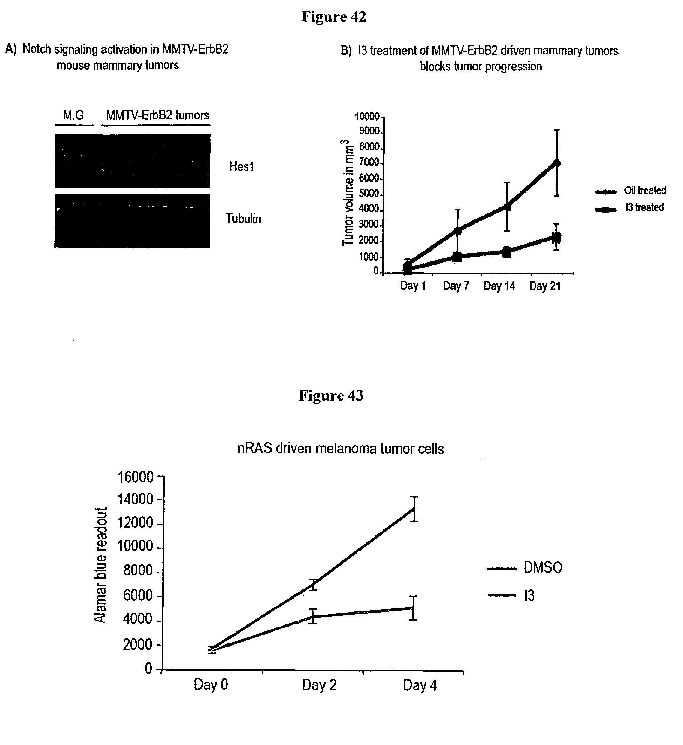

Figure 42 shows 13 treatment blocks MMTV-ErbB2 mouse mammary tumors. A) IIes1 protein expression levels in MMTV-ErbB2 mammary tumors and normal age matched mammary glands (M.G) were compared using Anti-Iles1 antibody. Western blot analyses showed a very high expression of Hcsl protein in MMTV-ErbB2 mammary tumors. Tubulin served as a loading control. B) A single cell suspension of MMTV-ErbB2 mammary tumor was prepared and 1 x 10⁶ cells were injected into the cleared fat pad of recipient FVB mice. Tumor formation was monitored on a regular basis. Once the tumor developed to a volume of 100-300 mm^{3,} mice were treated with oil (n-2) or 25 mg/kg of 13 (n-2). Tumor volume was measured every 6-7 days. Mice treated with I3 exhibited a slow tumor progression compared to oil treated mice.
Figure 43 shows effect of 13 on melanoma tumor cell proliferation. nRAS driven melanoma tumor cells were treated with DMSO or 10 µM of I3 in a 96 well plate format. Effect of I3 on tumor cell proliferation was determined using Alamar blue assay on day0, day2 and day 4. I3 treatment of melanoma cancer cells leads to proliferation arrest when compared to DMSO treated control cells.

### DETAILED DESCRIPTION OF THE INVENTION

Although methods and materials similar or equivalent to those described herein can be used in the practice or testing of the present invention, suitable methods and materials are described below. All publications, patent applications, patents, and other references mentioned herein are incorporated by reference in their entirety. The publications and applications discussed herein are provided solely for their disclosure prior to the filing date of the present application. Nothing herein is to be construed as an admission that the present invention is not entitled to antedate such publication by virtue of prior invention. In addition, the materials, methods, and examples are illustrative only and are not intended to he limiting.

In the case of conflict, the present specification, including definitions, will control. Unless defined otherwise, all technical and scientific terms used herein have the same meaning as is commonly understood by one of skill in art to which the subject matter herein belongs. As used herein, the following definitions are supplied in order to facilitate the understanding of the present invention.

As used herein, the term "comprise" is generally used in the sense of include, that is to say permitting the presence of one or more features or components. The terms "comprise" and "comprising" also encompass the more restricted ones "consist" and "consisting".

As used in the specification and claims, the singular form "a", "an" and "the" include plural references unless the context clearly dictates otherwise.

As used herein the terms "subject" or "patient" are well-recognized in the art, and, arc used interchangeably herein to refer to a mammal, including dog, cat, rat, mouse, monkey, cow, hoise, goat, sheep, pig, camel, and, most preferably, a human. In some embodiments, the subject is a subject in need of treatment or a subject with a disease or disorder, such as cancer. However, in other embodiments, the subject can be a normal subject or a subject who has already undergone a treatment against cancer. The term does not denote a particular age or sex. Thus, adult, children and newborn subjects, whether male or female, arc intended to be covered.

The tenns "cancer", "cell proliferative diseases" and "cell proliferative disorders" as used herein refer to or describe the physiological condition in mammals that is typically characterized by unregulated cell growth. According to the present invention, cancer refers preferably to solid tumors, such as brain, breast, prostate, colorectum, kidney, lung, sarcoma, or melanoma and liquid tumors, affecting the blood, such as leukemia. More preferably according to the present invention, cancers are Notch dependent cancers selected from the group comprising T cell-Acute lymphoblastic leukemia (T-ALL), chronic myeloid leukemia (CML), chronic lymphocytic leukemia (CLL), breast cancer, pancreatic cancer, prostate cancer, melanoma, brain tumors, tumor angiogenesis, colorectal cancer. Alternatively, the Notch dependent cancer is resistant to γ-secretase inhibitor treatment.

The Notch signaling pathway is evolutionarily conserved and the basic molecular players in this pathway are ligands (Delta and Jagged), Notch receptors, and the transcription factors (Cancer Res 2007;67(5):1879-82). Notch is a transmembrane heterodimeric receptor and there are four distinct members (Notch1 to Notch4) in humans and rodents. In a physiologie condition, binding of the Notch ligand to its receptor initiates Notch signaling by releasing the intracellular domain of the Notch receptor (Notch-ICD) through a cascade of proteolytic cleavages by both α-secretase (also called tumor necrosis factur-α-converting enzyme) and γ-secretase. The released intracellular Notch-ICD then translocates into the nucleus where it modulates gene expression primarily by binding to a ubiquitous transcription factor, CBF1, suppressor of hairless, Lag-1 (CSL). This binding recruits transcription activators to the CSL complex and converts it from a transcriptional represser into an activator, which turns on several downstream effectors. The physiologic Functions of Notch signaling are multifaceted, including maintenance of stem cells, specification of cell fate, and regulation of differentiation in development as well as in oncogenesis.

In cancers, molecular genetic alterations, such as chromosomal translocation, point mutations, and chromosomal amplification at the Notch receptor loci, are the known mechanisms for constitutive activation of Notch pathway. Despite the different mechanisms, they all result in increased levels of intracellular Notch-IC. The oncogenic potential of Notch was first discovered in human T-cell acute lymphoblastic leukemia (T-ALL). While Notch I signaling is essential for normal development of T-cell progenitors, constitutive activation of Notch1 signaling due to molecular genetic alterations is associated with T-ALL. For example, interstitial deletions of the extracellular portion of human Notch1 due to (7;9) chromosomal translocation are associated with ∼1% of T-ALL cases and activating point mutations of Notch1 are present in ∼50% of T-ALL cases. Formation of T-cell leukemia/lymphoma were observed in a Notch-ICD transgenic mouse model, which indicates a causal role of Notch activation in T-ALL development. In non-small cell lung cancer, chromosomal translocation (15;19) has been identified in a subset of tumors, and the translocation is thought to elevate Notch3 transcription in tumors. In ovarian cancer, Notch3 gene amplification was found to occur in ∼ 19% of tumors, and overexpression of Notch3 was found in more than half of the ovarian serous carcinomas. Similarly, Notch signaling activation has been shown in the development of breast cancer. In animal models, constitutively active Notch4 expression causes mammary tumors in mice and Notch1 -activating mutations contribute to the development of T-ALL. A recent study further shows that overexpression of activated Notch1 and Notch3 in transgenic mice blocks mammary gland development and induces mouse breast tumors. Notch signaling activation has also been implicated in lung and bone metastasis of breast cancer cells. Overexpression of Notch3 is sufficient to induce choroid plexus tumor formation in a mouse model, suggesting a role of Notch3 in the development of certain types of brain tumors.

With the aim of conducting a High-Through put Screening (HTS) to identify novel modulators (inhibitors) of Notch signalling, Applicants have established a coculture assay to induce a ligand-receptor mediated activation of the pathway. The coculture assay was established using Notch ligand DI,4 and Notch1 receptor specifically, because DL4-N1 ligand-receptor mediated pathway activation plays an important role in pathophysiological conditions such as tumor angiogenesis and the role of Notch1 receptor in inducing T cell leukemia. Since this assay depends on the expression and interaction between DL4 ligand and Notch1 receptor, it provides an opportunity to interrogate ligand-receptor interactions-induced Notch signaling in a controlled manner. The miniaturization of this assay into a 96 well plate and 384 well plate format helped Applicants to adapt this assay to conduct HTS. The use of this coculture assay to screen small molecule libraries can lead to the identification of proteins or chemical compounds that arc able to modulate Notch signaling at different steps along the pathway. For example, a HTS using small molecule libraries can yield modulators of the pathway able to act in the signal sending or signal receiving cells. Small molecule or protein mediated alterations in the recycling or trafficking of tbe ligands and receptors to the plasma membrane can potentially block the Notch pathway and could be studied using this assay. In addition, this assay can also help identify proteins or chemical entities able to block ligand-receptor interactions, ADAM10/17 mediated S2 cleavage or γ-secretase catalyzed S3 cleavage of the Notch receptor, nuclear translocation of the active form of Notch or entities able to block transcriptional activation complex.

Applicants have also been able to screen three different chemical compounds libraries (Microsource NIMDS, Prestwick and Maybridge flit finder) that have led to the identification of several chemicals; which are able to block Notch signaling at different levels along the pathway.

The use of the Notch-independent renilla system as an internal control allowed Applicants to eliminate cytotoxic chemical compounds, thereby limiting the rate of false positive hits. In addition, this cell-based assay also helped to circumvent issues related to the cell permeability of the chemical compounds for further hit validation.

The development of DL4:N1 coculture assay system laid the foundation for a IITS campaign. This assay provided a robust and sensitive readout system to identify novel modulators (inhibitors) of the Notch pathway.

Applicants identified three chemical compounds for their ability to block the Notch pathway activation. These compounds are referred to as follows
- Lasalocid (Lasal in figures only)
- Cyclopiazonic acid (CPA)
- 6-4[-(tertbutyl)-phenoxy] pyridine-3-amine (13)

The invention also encompasses chemical modifications of the compounds of the invention to prolong their circulating lifetimes.

Also encompassed in the present invention are chemical derivatives of the compounds of the invention.

Examples of derivatives of the compound 13 of the invention might be selected among the group comprising: 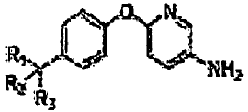 wherein R1, R2, R3 = CH₃, CH₃(CH₂)ₙCH₂ and n= 1-15; 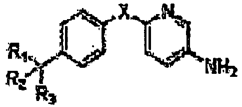 wherein R1, R2, R3 = CH_{3,} CH₃(CH₂)ₙCH₂, n=1-15,
X= CH₂, O, S, NR, R = phenyl, benzyl, CH₃(CH₂)nCH₂ n=1-15; 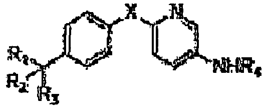 wherein R1, R2, R3 = CH_{3.} CH₃(CH₂)ₙCH₂ n=1-15
X= CH₂O, S, NR, R = phenyl, benzyl, CH₃CH₂)nCH₂ n=1-15
R4=CH₃(CH₂)ₙCH₂ n= 1-15 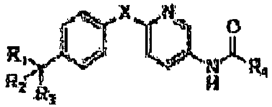 wherein R1, R2, R3 =CH₃, CH₃(CH₂)ₙCH₂ , n = 1-15
X= CH₂, O, S, NR, R = phenyl, benzyl, CH₃(CH₂)ₙCH₂ n=1-15
R4- CH₃(CH₂)ₙCH₂ n≐ 1-15 *;* 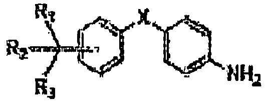 wherein substituent is at C-2, C-3 and C-4 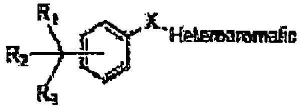 wherein R1, R2, R3 = CH₃, CH₃(CH₂)ₙCH₂ n= 1-15
X= CH₂, O, S, NR, R = phenyl, benzyl, CH₃(CH₂)nCH₂ n=1-15 or an Heteroaromatic: aminopyrrole, aminofurane, aminothiofurane, pyrimidine.

The invention also relates to pharmaceutically acceptable salts or solvates of the compounds, chemical modified compounds and derivatives of said compounds of the invention. According to the present invention, pharmaceutically acceptable salts are produced from acidic inorganic or organic compounds, or alkaline inorganic or organic compounds. As used herein, the phrase "pharmaceutically acceptable salt" refers to a salt that retains the biological effectiveness of the free acids and bases of a specified compound and that is not biologically or otherwise undesirable.

Unless specified otherwise, it is further understood that all isomers, including enantiomers, stereoisomers, rotamers, tautomers and raccmates of the compound(s) of the invention are contemplated as being part of this invention. The invention includes stereoisomers in optically pure form and in admixture, including racemic mixtures. Isomers can be prepared using conventional techniques, either by reacting optically pure or optically enriched starting materials or by separating isomers of compounds of the present invention.

"Racemates" refers to a mixture of enantiomers.

"Stereoisomer" or "stereoisomers" refer to compounds that differ in the chirality of one or more stereocentres. Stereoisomers include enantiomers and diastcrcomers. The compounds of this invention may exist in stcrcoisomeric form if they possess one or more asymmetric centres or a double bond with asymmetric substitution and, therefore, can be produced as individual stereoisomers or as mixtures. Unless otherwise indicated, the description is intended to include individual stereoisomers as well as mixtures. The methods for the determination of stereochemistry and the separation of stereoisomers are well-known in the art (see discussion in Chapter 4 of Advanced Organic Chemistry, 4th cd., J. March, John Wilcy and Sons, New York, 1992).

"Tautomer" refers to alternate forms of a compound that differ in the position of a proton, such as enol-keto and imine-enamine tautomers, or the tautomeric forms of heteroaryl groups containing a ring atom attached to both a ring -NH- moiety and a ring =N-moiety such as pyrazoles, imidazoles, benzimidazoles, triazoles, and tetrazoles.

A skilled person will know that, if a compound of the invention contains charged group, a suitable counterion will be derived from an organic or inorganic acid. Such counterions include halide (such as chloride, bromide, fluoride, iodide), sulfate, phosphate, acetate, succinate, citrate, lactate, maleate, fumarate, palmitate, cholate, glutamate, glutarate, tartrate, stearate, salicylate, methanesulfonate, benzenesulfonate, sorbate, picrate, benzoate, cinnamate, and the like. If the polar moiety is a negatively charged group, a suitable counterion will be selected from sodium, ammonium, barium, calcium, copper, iron, lithium, potassium and zinc, and the like.

Lasalocid belongs to a group of carboxylic ionophorcs. The complete chemical name is 6-(7-(5-ethyl-5-(5-ethyltetrahydro-5-hydroxy-6-methyl-2H-pyran-2-yl)tetrahydro-3-methyl-2-furanyl)-4-hydroxy-3,5-dimethyl-6-oxononyl)-2-hydroxy-3-methyl-, (2R-(2alpha(2S*(3R*,4S*,5S*,7R*),3S*,5S*),alpha,6beta))-benzoic acid and the chemical structure is

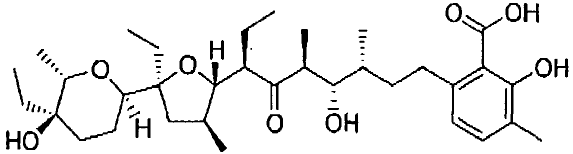

It has been widely used as an antibiotic for protection against coccidioses in domestic animals. The use of Lasalocid in cattle feed has also been shown to improve feed efficiency and weight gain. The fact that Lasalocid has been well studied in various animals (ranging from mice to cattle and horses) for its toxic effects makes it an attractive compound to be manipulated as a Notch signaling inhibitor. Data available from various governmental health agencies suggest that Lasalocid is well tolerated in the majority or the animals without any severe side effects (EMEA/MRL/912/04, Committee for veterinary medicinal product, Lasalocid Sodium, Summar report).

Applicants' *in vitro* studies have shown that Lasalocid can block Notch signaling in a concentration dependent manner and downregulates Notch target genes in Notch1- dependent leukemic cell lines, thus causing a proliferation arrest (Figures 4 and 5).

Affymetrix GeneChip analyses also confirmed the Notch inhibitory effect of Lasalocid in the human T-ALL cell line KOPTK1 (Figures 7 and 8). Despite their differences in the number of genes regulated by Lasalocid and DAPT, both compounds in part appear to impinge upon similar pathways. However, DAPT appears to he more selective when compared to Lasalocid treated samples. GSEA analyses of Lasalocid treated samples showed that majority of the genes differentially regulated by Lasalocid belong to various metabolic pathways. The differential regulation of several metabolic pathways could be due to its general property as an ionophore or it could be due to a reduced level of metabolism in growth arrested cells. GeneGo analyses using a much stricter criterion also confirmed Notch pathway regulation by Lasalocid when compared to DMSO. In addition these analyses also helped identify several signaling pathways differentially and commonly regulated by Lasalocid and DAPT. This information will be of extreme value when predicting the side effects of Lasalocid treatment in an *in vivo* setting.

According to the present invention, cyclopiazonic acid (CPA) is another compound identified as inhibitor of Notch signalling pathway. The complete chemical name is (6a*R*,11a*S*,11b*R*)-10-acetyl-11-hydroxy-7,7-dimethyl-2,6,6a,7,11a,11b-hexahydro-9*H*-pyrrolo[1',2':2,3] isoindolo[4,5,6-*cd*]indol-9-one and the chemical structure is

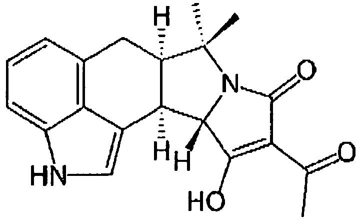

Data obtained from *in vitro* studies have shown that CPA treatment blocks Notch driven luciferase activity in a concentration dependent manner with an IC50 value in the low µM range. CPA has demonstrated a remarkable ability to block the Notch pathway activation in human leukemic cell lines as indicated by the downregulation of NICD and Notch target genes Hes1, cMyc and Dtx1. In addition Applicants have also shown that the downmodulation of Hes1 expression is due to an absence of NICD recruitment to the CSL/RBP-jk binding sites in the Hes1 promoter. Proliferation assay using several Human T-ALL cell lines indicate that most of these cell lines are sensitive to CPA treatment, as it causes a more pronounced proliferation arrest when compared to DAPT or DMSO. Mechanistic studies using fluorescence microscopy and Western blot analyses have shown that CPA blocks the Notch pathway activation by inhibiting its S1 cleavage and/or membrane transport. CPA mediated block in Notch signaling can be rescued by a transient introduction of NICD. This data is further corroborated by Affymetrix geneChip array. Affymetrix geneChip analyses on RNA extracted from CPA treated KOPTK1 cells have shown an enrichment of genes involved in vesicle and cndosomal sorting. Furthermore, the Affymetrix geneChiP array also identified additional signaling cascades (such as MTF and protein C signaling) potentially regulated by CPA treatment. Thus growth inhibitor effects of CPA on human leukemic cell lines could be due to additive effects of blocking Notch signaling as well as additional pathways. Interestingly, the Notch inhibitory effect of CPA appears to be specific against Notch1 only, as Notch2 mediated signaling remained intact when exposed to CPA.

Applicant's *in vivo* studies have shown that CPA treatment of mice docs not cause intestinal toxicity. The induction of goblet cell metaplasia in the mouse intestine requires ablation of both Notch1 and Notch2 mediated pathway activation. The lack of goblet cell metaplasia in the CPA injected mice could be due to the fact that CPA blocks only Notch1 mediated signaling but not via Notch2.

The chemical compound 6-4|-(tertbutyl)-phenoxy] pyridine-3-amine (I3) was also identified as a potential Notch inhibitor. Interestingly, 13 was found to block NICD mediated pathway activation. Because of its ability to attenuate NICD mediated Notch activation, 13 is able to block proliferation of DAPT resistant, NICD overexpressing leukemic cell lines. The Notch inhibitory potential of I3 was further confirmed by the downregulation of Notch target genes in human T-ALL cell lines and Affymetrix geneChip array. The fact that 13 can induce differentiation of C2Cl2 cells into MIIC expressing multinucleated myotubes further validated the anti-Notch role of 13. The Notch pathway inhibition caused by 13 can be rescued by the overexpression of MAML1 above certain levels. For example, I3 was able to block the signaling with 800 ng of NICD and 1 µg of MAML1 was transiently introduced into the cells, however when the amount of MAML1 was increased to 3 µg, I3 was no longer able to block the pathway activation. These data suggest that, 13 may interfere with the Notch transcriptional activation complex thereby inhibiting the signaling activation. Microscopic studies by introduction of MAML at levels where 13 could still block the pathway activation showed that 13 treatment does not impede co-localization of NICD, MAML1 and CSL/RBP-jk in the sub-nuclear compartments. Without being bound to theory, one of the possible mechanisms of action of 13 could be to disrupt the recruitment of transcriptional coactivators to the core CSL/RBP-jk-NICD-MAML1 complex. Therefore, the status of additional coactivators involved in the formation of functional transcriptional activation complex still needs to be determined. Under physiological conditions, following the formation of CSL/RBP-jk-NICD-MAML1 complex, CBP/p300 histone acetyltransferase (HAT) is recruited to the complex leading to its autoacetylation and acetylation of histone 3 and 4.

Affymetrix geneChip and subsequent GeneGo analyses on the most significantly altered genes (about 700 genes with p value <0.05 and fold change in the expression level > 2) showed that in addition to blocking Notch pathway, I3 can also alter the expression of genes involved in MIF (Macrophage migration inhibitory factor) and protein kinase C. In addition, I3 treatment of KOPTK1 cells also led to a change in the expression of genes implicated in several human diseases such as multiple sclerosis, demyelinating and autoimmune disease. However, GeneGo analyses and *in vitro* reporter assays did not reveal 13 activity against other developmental pathways such as Wnt and Hedgehog signaling.

The chemical compound I3 was further investigated in an *in vivo* context to determine its Notch inhibitory as well as toxic side effects in the nice. Notch signaling is essential for the maintenance of normal homoeostasis in the intestine. Genetic ablation or pharmacological inhibition of Notch1 and Notch2 signaling in the intestine leads to goblet cell metaplasia in the intestine. Since, I3 has been observed to block Notch1 and Notch2 mediated signaling, mice treated with I3 were expected to develop goblet cell metaplasia. Surprisingly, treatment of mice with 25 mg/kg of I3 for 7 days (more than a month in case of xenotransplants) did not perturb intestinal homeostasis and without any indication of goblet cell accumulation. This unexpected outcome could be due to two reasons. One possible explanation could be that the concentration of I3 (25mg/kg) used is not sufficient to block Notch pathway activation in the intestine. However, a second more plausible explanation could be the differences in the composition of transcriptional activation complexes downstream of Notch1 and Notch2 signaling. Due to these possible differences, Notch1 and Notch2 mediated signaling may have different sensitivities against 13 treatment.

Notch signaling plays an important role in the regulation of hematopoietic system. For instance, DL4-Notch1 signaling is essential for T cell development in the thymus. Notch2 and MAML1 mediated pathway activation is critical for MZB cell development in the spleen. To address whether I3 can impair T cell and MZB cell development in the thymus and spleen respectively, these tissues were analyzed by flow cytometry. The treatment of mice with I3 led an increase in apoptosis in the thymus. I3 treatment of mice had a modest effect on the CD4+CD8+ DP cells in the thymus, however this was not accompanied by an accumulation of B cells. The reduction observed in the percentage of CD4+CD8+ DP cells could be due to an increase in apoptosis. Therefore, I3 treated thymocytes do not mimic the loss of Notch1 phenotype in the thymus and a slight decrease of DP population could be due to general cytotoxic effect of I3. Since, I3 also regulates expression of genes involved in several other signaling pathways, a Notch1-independent effect of I3 on the thymus cannot he ruled out. However, if the mechanism of action of I3 involves MAML1 protein, the observed phenotype, in part, is in agreement with the loss of MAML1 studies. The T cell development in MAML1^{-/-} chimeras occurs normally and this has been proposed due to compensation by MAML2 and MAML3.

Similar studies conducted to investigate the effect of I3 on the MZB cell development in the spleen were convoluted by an unexpected development of splenomegaly. However, the introduction of a recovery phase between injections and the analyses time points allowed the mice to recover from splenomegaly. Analyses following the reversal of splenomegaly phenotype, showed a decrease in the numbers of MZB cells in the spleen. This decrease in the numbers and percentage of MZB cells in the spleen very well could be due to I3 mediated block in Notch2 signaling, as all the other cell populations in the spleen were at normal levels. Therefore, apart from the development of transient splenomegaly, I3 treatment recapitulate MZB cell phenotype observed due to the loss of Notch2 and MAML1 transcription activator.

Anti-cancer activity of I3 was investigated in transplant models for human diseases, namely T-cell leukemia and breast cancer. In these studies, I3 has demonstrated a remarkable ability to slow down the progression and metastasis of very aggressive form of leukemic cell lines. In addition, in a preliminary study using breast cancer as a model of solid tumors, I3 treatment has led to a block in tumor progression in the mice.

The chemical compound I3 has shown ability to block NICD mediated signaling. Therefore this compound could be potentially used in cancers where Notch driven tumors are resistant to γ-secretase inhibitor treatment.

The present invention also provides a pharmaceutical composition comprising the inhibitor of Notch signaling pathway selected from the group consisting of 6-4[-(tertbutyl)-phenoxy] pyridine-3-amine (I3), Cyclopiazonic acid and Lasalocid, and a pharmaceutically acceptable carrier. Preferably said inhibitor of Notch signaling pathway is 6-4-[tertbutyl)-phenoxy] pyridine-3-amine (I3). As to the appropriate carriers, reference may be made to the standard literature describing these, e.g. to chapter 25.2 of Vol. 5 of "Comprehensive Medicinal Chemistry", Pergamon Press 1990, and to "Lexikon der Hilfsstoffe für Pharmazie, Kosmetik und angrenzende Gebiete", by H.P. Fiedler, Editio Cantor, 2002. The term "pharmaceutically acceptable carrier" means a carrier or excipient that is useful in preparing a pharmaceutical composition that is generally safe, and possesses acceptable toxicities. Acceptable carriers include those that are acceptable for veterinary use as well as human pharmaceutical use. A "pharmaceutically acceptable carrier" as used in the specification and claims includes both one and more than one such carrier.

Optionally, the pharmaceutical composition of the present invention further comprises one or more additional active agents.

The compounds of the invention that are used in the treatment and/or prevention of cancers can be incorporated into a variety of formulations and medicaments for therapeutic administration. More particularly, a compound as provided herein can be formulated into pharmaceutical compositions by combination with appropriate, pharmaceutically acceptable carriers, and can be formulated into preparations in solid, semi-solid, liquid or gaseous forms, such as tablets, capsules, pills, powders, granules, dragees, gels, slurries, ointments, solutions, suppositories, injections, inhalants and aerosols. As such, administration of the compounds can be achieved in various ways, including oral, buccal, rectal, parenteral, intraperitoneal, intradermal, transdermal, intracranial and/or intratracheal administration. Moreover, the compound can be administered in a local rather than systemic manner, in a depot or sustained release formulation. The compounds can be formulated with common excipients, diluents or carriers, and compressed into tablets, or formulated as elixirs or solution for convenient oral administration, or administered by the intramuscular or intravenous routes. The compounds can be administered transdermally, and can he formulated as sustained release dosage forms and the like. The compounds can be administered alone, in combination with each other, or they can be used in combination with other known compounds. Suitable formulations for use in the present invention are found in Remington's Pharmaceutical Sciences (Mack Publishing Company (1985) Philadelphia, PA, 17th ed.), which is incorporated herein by reference. Moreover, for a brief review of methods for drug delivery, see, Langer, Science (1990) 249:1527-1533, which is incorporated herein by reference.

The amount of a compound as provided herein that can be combined with a carrier material to produce a single dosage form will vary depending upon the disease treated, the mammalian species, and the particular mode of administration. However, as a general guide, suitable unit doses for the compounds of the present invention can, for example, preferably contain between 0.1 mg to about 1000 mg, between 1 mg to about 500 mg, and between 1 mg to about 300 mg of the active compound. In another example, the unit dose is between 1 mg to about 100 mg. Such unit doses can be administered more than once a day, for example, 2, 3, 4, 5 or 6 times a day, but preferably 1 or 2 times per day, so that the total dosage for a 70 kg adult is in the range of 0.001 to about 15 mg per kg weight of subject per administration. A preferred dosage is 0.01 to about 1.5 mg per kg weight of subject per administration, and such therapy can extend for a number of weeks or months, and in some cases, years. It will be understood, however, that the specific dose level for any particular patient will depend on a variety of factors including the activity of the specific compound employed; the age, body weight, general health, sex and diet of the individual being treated; the time and route of administration; the rate of excretion; other drugs that have previously been administered; and the severity of the particular disease undergoing therapy, as is well understood by those of skill in the area. A typical dosage can be one 1 mg to about 100 mg tablet or 1 mg to about 300 mg taken once a day, or, multiple times per day, or one time-release capsule or tablet taken once a day and containing a proportionally higher content of active ingredient The time-release effect can be obtained by capsule materials that dissolve at different pH values, by capsules that release slowly by osmotic pressure, or by any other known means of controlled release. It can he necessary to use dosages outside these ranges in some cases as will be apparent to those skilled in the art.

The present invention further provides an inhibitor of Notch signaling pathway for use in treating and/or preventing cancers, wherein said inhibitor of Notch signaling pathway is selected from the group consisting of 6-4[-(tertbutyl)-phenoxy] pyridine-3-amine (I3), Cyclopiazonic acid and Lasalocid. Preferably said inhibitor is 6-4[-(tertbutyl)-phenoxy] pyridine-3-amine (I3).

Preferably cancers are Notch dependent cancers selected from the group comprising T cell-Acutc lymphoblastic leukemia (T-ALL), chronic myeloid leukemia (CML), chronic lymphocytic leukemia (CLL), breast cancer, pancreatic cancer, prostate cancer, melanoma, brain tumors, tumor angiogenesis, colorectal cancer.

The compounds of the present invention, 6-4[tertbutyl)-phenoxy] pyridine-3-amine (I3), Cyclopiazonic acid and Lasalocid, preferably 6-4[-(tertbutyl)-phenoxy] pyridine-3-amine (I3) can be also used in treatment of cancers where Notch dependent cancers are resistant to γ-secretase inhibitor treatment.

The present invention also provides a method for treating and/or preventing cancers, said method comprising administering the inhibitor of Notch signaling pathway selected from the group consisting of 6-4[-(tertbutyl)-phenoxy] pyridine-3-amine (I3), Cyclopiazonic acid and Lasalocid or the pharmaceutical composition of the invention a subject in need thereof. Preferably said inhibitor is 6-4[-(tertbutyl)-phenoxy] pyridine-3-amine (I3).

In another embodiment, the present invention provides a method of treatment of a disease associated with an up-regulated Notch signalling pathway activity, said method comprising administrating the inhibitor of Notch signaling pathway selected from the group consisting of 6-4[-(tertbutyl)-phenoxy] pyridine-3-amine (I3), Cyclopiazonic acid and Lasalocid or the pharmaceutical composition of the invention to a subject in need thereof. Preferably said inhibitor is 6-4[-(tertbutyl)-phenoxy] pyridine-3-amine (I3).

The daily dose or compounds of the present invention will necessarily be varied depending upon the host treated, the particular route of administration, and the severity and kind of the illness being treated. Accordingly the optimum dosage may be determined by the practitioner who is treating any particular patient. Further, it is noted that the clinician or treating physician will know how and when to start, interrupt, adjust, or terminate therapy in conjunction with individual patient response.

For any compound used in the method of the present invention, a therapeutically effective dose can be estimated initially from cell culture assays, animal models, or microdosing of human subjects.

"Treatment" refers to both therapeutic treatment and prophylactic or preventative measures. Those in need of treatment include those already with the disorder, such as cancer, as well as those in which the disorder, such as cancer, is to be prevented. Hence, the mammal, preferably human, to be treated herein may have been diagnosed as having the disorder, such as cancer, or may be predisposed or susceptible to the disorder, such as cancer.

"Mammal" for purposes of treatment refers to any animal classified as a mammal, including humans, domestic and farm animals or pet animals, such as dogs, horses, cats, cows, monkeys etc. Preferably, the mammal is human.

The term "therapeutically effective amount" refers to an amount of a drug effective to treat a disease or disorder in a mammal. In the case of cancer, the therapeutically effective amount of the drug may reduce the number of tumor cells, reduce the tumor size; inhibit (i.e., slow to some extent and preferably stop) cancer cells infiltration into peripheral organs; inhibit (i.e., slow to some extent and preferably stop) tumor metastasis; inhibit, to some extent, tumor growth; and/or relieve to some extent one or more of the symptoms associated with the cancer. To the extent the compounds of the present invention may prevent growth and/or kill existing cancer cells, it may be cytostatic and/or cytotoxic. The phrase "therapeutically effective amount" is used herein to mean an amount sufficient to prevent, or preferably reduce by at least about 30 percent, preferably by at least 50 percent, preferably by at least 70 percent, preferably by at least 80 percent, preferably by at least 90%, a clinically significant change in the growth or progression or mitotic activity of a target cellular mass, group of cancer cells, or other feature of pathology.

Optionally the compounds of the present invention may be used against cell proliferate diseases in combination (for example either at the same time, or almost at the same time, or one after the other) with conventional treatment such as standard radiotherapy and/or standard chemotherapy. The standard radiotherapy and chemotherapy can be also the concomitant chemo-radiotherapy.

Therefore, optionally, the standard radiotherapy and/or chemotherapy can be performed before, simultaneously or after the administration of a therapeutically effective amount of the compound of the present invention, or pharmaceutical compositions containing thereof.

The term "concomitant chemo-radiotherapy" is used when these two treatments (chemotherapy and radiotherapy) are given either at the same time, or almost at the same time, for instance one after the other, or on the same day, etc.

The term "standard radiotherapy" refers to the use of ionizing radiation as part of cancer treatment to control malignant cells. Preferably the ionizing radiation is γ-irradiation. It is also common to combine radiotherapy with surgery, chemotherapy, hormone therapy, or combinations thereof. Most common cancer types can be usually treated with radiotherapy. The precise treatment intent (curative, adjuvant, neoadjuvant or palliative) will depend on the tumor type, location, and stage, as well as the general health of the subject in need thereof.

The term "standard chemotherapy" generally refers to a treatment of a cancer using specific chemotherapeutic/chemical agents. A chemotherapeutic agent refers to a pharmaceutical agent generally used for treating cancer. The chemotherapeutic agents for treating cancer include, for example, Altretamine, Bleomycin, Busulphan, Capecitabine, Carboplatin, Carmustine, Chlorambucil, Cisplatin, Cladribine, Crisantaspase, Cyclophosphamid, Cytarabine, Dacarbazine, Daunorubicin, Doxorubicin, Epirubicin, Etoposide, Fludarabine, Fluorouracil, Gemcitabine, Idarubicin, Ifosfamide, Irinotecan, Lomustine, Melphalan, Mercaptopurine, Methotrexate, Mitomycin, Mitoxantrone, Oxaliplatin, Pentostatin, Procarbazine, Streptozocin, Taco, Temozolomide,, Tioguanine/Thioguanine, Thiotepa, Topotecan, Treosulfan, Vinblastine, Vineristine, Vindesine or Vinorelbine.

When a chemotherapeutic agent is used in combination with the compound according to the present invention, then this may be used in the form of a medicament containing a combination of these two agents, for simultaneous administration, or they may be used in the form of separate dosage forms, each containing one of the agents, and in the latter case the individual dosage forms may be used e.g. sequentially, i.e. one dosage form with the compound of the invention , followed by a dosage form containing the chemotherapeutic agent (or vice versa). This embodiment of two separate dosage forms may be conceived and provided in the form of a kit.

Also optionally the compounds of the present invention may be used against cell proliferate diseases, such as cancers, in combination with conventional removal of a tumor bulk, by for example segmental resection (biopsy or gross resection).

The term "removal of n tumor bulk" refers to any removal, ablation or resection of a tumor bulk from a subject. The removal can be chemical, radiation or surgical. Preferably said removal is surgical, such as ablation or resection. Resection can he "segmental resection" (or segmentectomy), a surgical procedure to remove part of an organ or gland from a subject, It may also be used to remove a tumor and normal tissue around it. Debulking agent may be also used to remove tumor bulk. The term "debulking agent" includes any molecule (e.g. chemical, biological) or any external/environmental agent (e.g. γ-irradiation) or traditional surgery that would allow killing cancer cells from the tumor bulk (e.g.FL1⁰ and FL1⁻ cells as mentioned above).

Another object of the present invention is a kit comprising one or more doses of the inhibitor of Notch signaling pathway selected from the group consisting of 6-4[-(tertbutyl)-phenoxy] pyridine-3-amine (13), Cyclopiazonic acid and Lasalocid, or the pharmaceutical composition of the present invention for use in a method for treatment and/or prevention of cancers. The kit can further comprise one or more doses of a chemotherapeutic agent.

Generally, the kit comprises a container and a label or package insert on or associated with the container. Suitable containers include, for example, bottles, vials, syringes, etc. The containers may he formed from a variety of materials such as glass or plastic. The container holds the compound's composition that are effective for treating the condition and may have a sterile access port (for example the container may be an intravenous solution bag or a vial having a stopper pierceable by a hypodermic injection needle). The label or package insert indicates that the composition is used for treating the condition of choice, such as cancer.

Those skilled in the art will appreciate that the invention described herein is susceptible to variations and modifications other than those specifically described. It is to he understood that the invention includes all such variations and modifications without departing from the spirit or essential characteristics thereof. The invention also includes all of the steps, features, compositions and compounds referred to or indicated in this specification, individually or collectively, and any and all combinations or any two or more of said steps or features. The present disclosure is therefore to be considered as in all aspects illustrated and not restrictive, the scope of the invention being indicated by the appended Claims, and all changes which come within the meaning and range of equivalency arc intended to be embraced therein.

Various reference are cited throughout this Specification, each of which is incorporated herein by reference in its entirety.

The foregoing description will be more fully understood with reference to the following Examples. Such Examples, are, however, exemplary of methods of practicing the present invention and are not intended to limit the scope of the invention.

### EXAMPLES

### Constructs and gene reporter assays:

Mouse DL4-IRES dsRED cDNA was cloned into a pENTRI vector (Invitrogen) and finally shuttled into a destination lentivirus vector using Gateway cloning strategy (Invitrogen). A Phosphoglycerate kinase (PGK) promoter drove the expression of DL4 protein. DL4-lentiviral particles were produced in 293T cells by cotransfection of DL4-lentivirus vector, (Gag/pol expression plasmid and plasmid encoding for viral envelope proteins. To overexpress Notch1 protein, mouse full length Notch1 cDNA was a cloned in a pCDNA3.1-IRES-puromycin vector. Notch1 cDNA was cloned upstream of IRES-puromycin between HindIII and XbaI restriction sites. A CMV promoter controlled the expression or Notch1 protein.

To measure the Notch signaling activation, CSL/RBP-jk consensus DNA binding sequences [C(T)GTGGGAA] (12x) were cloned in a head to tail conformation in the pGL4 luciferase vector (Promega), thus named 12xCSL/RBP-jjk luciferase vector. In order to determine the Notch pathway activation in chemical compound screening assay, 12x CSL/RBP-jk consensus DNA binding sequences were cloned into pGL4.26.luciferase vector (Promega). As an internal control for transfection efficiency, SV40 Renilla vector was used (Promega).

NICD-GFP overexpression studies were performed using pEGFP-CI-NICD expression plasmid. The FLAG-CMV2 plasmid expressing MAML1-FLAG was a kind gift from Dr. Lizi Wu, Harvard Medical School, Boston.

### Generation of DL4 and N1 stable HeLa cells:

In order to generate DL4 and N 1 stable cell lines, HeLa cell were bought from ATCC (catalog # CCL-2). To generate DL4 stable lines, cells were transduced with DL4-lentiviral particles. DL4 stable clones were selected using puromycin. High DL4 expressing clones were sorted using antibodies against DL4 with Fluorescence activated cell-sorting (FACS). To generate N1 stable line, cell were transfected with a pCDNA3.1+ (Invitrogen) plasmid containing mouse full length Notch1 under the control of CMV promoter. To select for Notch1 expressing clones, an IRES puromycin cassette was cloned downstream of Notch1 cDNA. HeLa cells expressing high levels of Notch1 were enriched by FACS using anti-Notch1 antibodies. DL4- andNl-HeLa cells were cultured in DMEM (GIBCO, Invitiogen), 10% FCS and 10ug/ml of puromycin (Sigma).

### High-Throughput Screening and data analyses:

For chemical library screen, the coculture assay was modified as follows. N1-HeLa cells were cotransfected in 10 cm tissue culture dishes with 16 µg of pGL4.26.12xCSL.luciferase vector/plate, 4 µg of Notch1 expression plasmid/plate, and 200 ng of SV40 Rcnilla vector/plate.DL4- and N1-HeLa cells were detached from the plate by using 0.5mM EDTA (IXPBS). The both cell populations were counted and mixed in 1:1 ratio (5,000:5,000 cells/well in a 384 well plate) and dispensed into 384 well plates (white, clear bottom, Coming) using multidrop Combi plate dispenser. The assay plates were pre-dispensed (using automated Biomek 3000 liquid handler) with chemical compound libraries (Microsource NIMDS, Maybridge Hitfinder and Prestwick) to give a final concentration of 10 µM. The final assay volume was 22 µls. Twenty-four hours later, growth media was aspirated and cells were lysed with 1x Passive lysis buffer for 10 minutes at room temperature. Luciferase activity was measured using Luciferase Assay Reagent II and Renilla values were determined by using Stop and Glow reagent (Dual luciferase assay system, Cat # E198U, Promega). Luciferase and renilla readouts were taken using Tecan® F500 (Tecan) multiplate reader. All the liquid handling steps (aspiration of the medium, dispensing of Passive lysis buffer, Luciferase Assay Reagent II and Stop and Glow reagents) were performed using ELF406 liquid handler.

Data analyses were performed using in-house built analyses software at Biomolecular Screening Facility (BSF) at Ecole Polytechnique Federale de Lausanne (EPFL).

### RNA extraction

Total RNA was extracted from cells using TRIzol® extraction kit (Invitrogen). Briefly, 1x 10⁶ cells were washed with ice-cold 1xPBS and lysed in I ml of TRIzol® solution for 5 minutes at room temperature to dissociate nucleoprotein complexes. Lysed cells were then treated with 200 µl of chloroform and shaked vigorously for 15-30 seconds and incubated at room temperature for 2-3 minutes. The samples were centrifuged at 14000 rpm using Eppendorf table top centrifuge for 10 minutes at 4°C. Following centrifugation, upper aqueous phase was transferred to new eppendorf tubes. To precipitate total RNA 500 µl of isomyl alcohol was added to the separated aqueous phase and incubated at room temperature for 10 minutes. A RNA pellet was obtained by centrifuging the samples at 4°C for 10 minutes RNA pellet obtained was washed with 1 ml ice cold 75% ethanol and spun down at 14000 rpm at 4°C. RNA pellet was dried off of excess of ethanol and resuspended in 40 µl DPEC water.

### cDNA synthesis:

Total RNA extracted from the cell was used to synthesize cDNA by reverse transcription reaction. Reverse transcription was performed using SuperScript™ RT (Invitrogen). RNA concentration was measured using NanoDrop®ND-1000 spectrophotometer (Witec AG) and 500 ng of total RNA was mixed with a 10mM mix of dNTPs and 100 ng of random primers. The reaction mix was incubated at 65°C for 5 minutes and quickly incubated on ice for 1 minute. Following incubation on ice, 5x first strand buffer and 0.1M DTT were added and mix was incubated for 2 minutes at 25°C. To start the reverse transcription reaction, 200U of SuperScrip™ II RT was added to the reaction mix and incubated at 42°C for 50 minutes. The reaction was stopped by incubating the reaction mix at 75°C for 15 minutes.

### Western blot analyses:

Cells were lysed in RIPA buffer (50mM Tris.Cl, pH 7.5, 150 mM NaCl. 1% nonidet P-40, 0.5% sodium deoxycholate and 0.1% SDS) for 30 minutes at 4°C. Lysed cells were centrifuged to remove the debris at 14000 rpm at 4°C. Supernatant was transferred to a new eppendorf tube. The protein concentration was determined by Bradford assay using spectrophotometer (Ultrospec 3000 pro). 40 µg of protein were denatured in 1x SDS gel loading buffer (100mM Tris.Cl, pH 6.8,200 mM DTT, 4 % SDS, 0.2 % bromophenol, 20% glycerol) by heating at 99°C for 5 minutes. Denatured protein samples were stored on ice until loading on to the acrylamide gel. The samples were run on 8% or 10% acrylamide gel in Tris-glycine electrophoresis buffer (25mM Tris, 250 mM glycine, 0.1% SDS). Following separation on the acrylamide gel, protein samples were transferred on to PVDF membrane (PEQ lab, catalog number 39-3010) using transfer buffer (39mM glycine, 48 mM Tris base, 0.037 % SDS and 20 % methanol).

For immunoblotting, membranes were blocked with 5 % milk and incubated overnight with primary antibodies at 4°C. Membrane were washed with 1x TBST (1x TBS + 0.5 % tween 20) for 5 minutes (3 times) and incubated with HRP-conjugated secondary antibodies for one hour at room temperature. Signal was detected with Super Signal West chemiluminescent substrate (Thermo Scientific, catalog number 34077).

### Chromatin Immunoprecipitation assay:

Two million cells were fixed with 1% formaldehyde for 10 minutes to crosslink the proteins to the DNA. Cells were washed and detached using 1ml of ice-cold 1xPBS supplemented with protease inhibitors and spun down at 700x at 4°C for 5 minutes. Pellet obtained following centrifugation was suspended in 200µl of 1x SDS lysis buffer (1% SDS, 10mM EDTA, 50mM Tris pH8.1) containing protease inhibitors and sonicated to shear chromatin DNA down to the size of 500-1000 bps. Eight hundred µls of dilution buffer (0.01% SDS, 1-1% triton X-100, 1.2 mM EDTA, 16.7 mM Tris.HCl pH8.1 and 167 mM NaCl, protease inhibitors) was added to the sheared chromatin DNA. The chromatin solution was precleared using 60 µl of Protein G Agarose and incubating at 4°C for one hour with rotation. Solution was centrifuged at 3000x g for one minute and supernatant was transferred to so new tube.

To immunoprecipitate NICD bound chromatin DNA, samples were incubated with 1 µg anti-NICD antibody (Val1744, Cell signal) at 4°C with rotation for 12 hours. An equal amount of Rabbit IgG was used as negative control for immunoprecipitation. Antibody/protein/DNA complexes were precipitated by incubating the samples with Protein G Agarose for 1 hour at 4°C. Subsequently an antibody/protein/DNA pellet was obtained by centrifuging the samples at 3000 x g for one minute. The antibody/protein/DNA complexes were washed once with an ice-cold low salt immune complex wash buffer (0.1% SDS, 1% Triton X-100, 2mM EDTA, 20mM Tric.HCl pH8.1 and 150mM NaCl) and then with high salt immune complex wash buffer (0.1% SDS, 1% Triton X-100, 2mM EDTA, 20mM Tris.HCl pH 8.1 and 500 mM NaCl). The samples were then washed once with LiCl immune complex wash buffer (0.25mM LiCl, 1% IGEPAL-CA630. 1% deoxycholic acid, 1 mM EDTA, 10mM Tris.HCl pH 8.1) and washed twice with TE buffer (10mM Tric.HCl, 1mM EDTA pH 8.0). The Antibody/protein/DNA complexes were clutcd out with 100 µl of elution buffer (1% SDS, 100mM NaHCO₃) by incubating at room temperature for 15 minutes. The Antibody/protein/DNA complexes were centrifuged and supernatant was transferred to a new eppendorf tube. In order to reverse the protein-DNA crosslinking, samples were incubated with 5M NaCl at 65°C for 4 hours and samples were further treated with 4 µl of 0.5M EDTA, 8 µl of Tris.HCl and 1 µl of proteinase K and incubated for another 2 hours at 45 °C.

Following reverse crosslinking step, DNA was extracted by adding 500 µl of pltenol/chloroform/isoamyl alcohol to each tube. Samples were vortexed vigorously and spun down at 14000 rpm for 15 minutes at 4°C. The aqueous phase was transferred to a fresh eppendorf tube. To the aqueous phase, 1.25 ml of 100 % ethanol, 50 µl of 3M sodium acetate pH 5.2 and 4 µl of glycogen was added and stored at -80°C: for one hour. Samples were then centrifuged at 14000 rpm for 15 minutes at 4°C. Supernatant was discarded and DNA pellet was wished with 100 % ethanol and then with 75 % ethanol. DNA pellet was air dried and resuspended in 20 µl of TE buffer. The human Hcsl promoter and intron 3 regions were amplified using 5 µl of eluted DNA mixed with appropriate primer pairs.

### Immunofluorescence staining:

To perform Immunofluorescence staining, HeLa cells or C2C12 cells were grown on cover slips. Cells were washed with 1x ice-cold PBS, fixed with 4% PFA for 5 minutes at room temperature and permeabilized using 0.3% Triton X-100. Subsequently permeabilized cells were blocked for 20 minutes with I % BSA for 20 minutes at room temperature. Cell were incubated with appropriate primary antibodies for one at room temperature. Alexa Fluor-488 conjugated secondary antibodies were used to detect primary antibodies. Cells were counterstained with DAPI and mounted in fluorescent mounting media. Fluorescent images were viewed and captured using Zeiss Axioplan microscope at Bioimaging and optics core facility at EPFL.

**Table 1: List of the antibodies and working dilutions.**

| **Antibodies** | **Application** | **Dilution** | **Source** |
|---|---|---|---|
| Anti-Val 1744 NICD | WB and ChIP | 1:1000 | Cell Signal, 2421 S |
| Anti-Notch1 (C-20) | WB | 1:1000 | Santa Cruz: sc-6014 |
| Anti-RBP-jk | IF | 1:500 | Santa Cruz, sc-28713 |
| Anti-FLAg-M2 | IF | 1:500 | Sigma, F1804 |
| Anti-Hes1 (H-140) | WB | 1:500 | Santa Cruz,sc-25392 |
| Anti-cMyc (9E10) | WB | 1:500 | Abeam, abl1917 |
| Anti-Delta like 4 | Flow cytometry | 1:100 | Produced in-house |
| Anti-Notch1 | Flow cytometry | 1:50 | Produced in-house |
| Anti-Tubulin | WB | 1:3000 | Sigma, |
| Anti-Myosin heavy chain | IF | 1:200 | Sigma, MY-32 |
| HRP-conjugated anti-goat IgG | WB | 1:3000 | Invitrogen, 611620 |
| HRP-conjugated anti-mouse IgG | WB | 1:3000 | GEhealthcare,NA931V |
| HRP-conjugated anti-rabbit IgG | WB | 1:3000 | GEhealthcare,NA934V |
| Alexa Fluor-488 secondary Ab | IF | 1:1000 | Invitrogen |
| Anti-B220-Pacific blue | Flow cytometry | 1:400 | Produced in-house |
| Anti-CD21-FITC | Flow cytometry | 1:200 | eBioscience |
| Anti-CD23-PE | Flow cytometry | 1:400 | BD Pharmingen |
| Anti-TCR β-APC eF780 | Flow cytometry | 1:400 | eBioscience |
| Anti-CD4-FITC | Flow cytometry | 1:800 | Produced in-house |
| Anti-CD8-Alexa 648 | Flow cytometry | 1:600 | Produced in-house |
| Anti-CD71-PE | Flow cytometry | 1:800 | eBioscience |
| Anti-Ter119-APC eF780 | Flow cytometry | 1:200 | eBioscience |
| Anti-AnnexinV-Cy5 | Flow cytometry | 1:50 | BD Pharmingen |

### C2C12 Myoblast differentiation assay:

C2C12 cells were grown on collagen-coaled cover slip in the presence of growth media (10% serum). To induce myoblast differentiation, cells were grown to 100% confluency for 3 days in the presence of differentiation media (2% horse serum) or in the presence of growth media + Notch inhibitors. After 3 days, cells were washed with 1x ice-cold PBS and fixed with 4 % PFA. Immunofluorescence staining was performed using anti-MHC antibody as explained in the section 2.2.6 (Immunofluorescence staining).

### Affymetric geneChip array and data analyses:

Total RNAs from 5x10⁵ cells were isolated and purified with RNeasy® kit (Qiagen). RNA quantities were assessed by NanoDrop®ND-1000 spectrophotometer and the RNA quality was assessed using RNA 6000 NanoChips with the Agilent 2100 Bioanalyzer (Agilent, Palo Alto, USA). For each sample, 100ng of total RNA were amplified using the WT sense strand Target Labelling kit (Affymetrix, Cat.no. 900223); 5.5µg of the resulting sense cDNA was fragmented by UDG (uracil DNA glycosylase) and APE 1 (apurinic/apyrimidic endonuclease 1) and biotin-labelled with TdT (terminal deoxynucleotidyl transferase) using the GeneChip® WT Terminal labelling kit (Affymetrix Cat.no. 900671, Santa Clara, USA). Affymetrix Human Gene 1.0 ST arrays (Affymetrix, Santa Clara , CA, USA) were hybridized with 2.7µg of biotinylated target, at 45°C for 17 hours washed and stained according to the protocol described in Affymetrix GeneChip® Expression Analysis Manual Fluidies protocol FS450_0007).

The arrays were scanned using the GeneChip^{®} Scanner 3000 7G (Affymetrix) and raw data was extracted from the scanned images and analyzed with the Affymetrix Power Tools software package (Affymetrix).

Hybridisation quality was assessed using the Expression Console software (Affymetrix). Normalized expression signals were calculated from Affymetrix CEL files using RMA normalization method. Differential hybridized features were identified using Bioconductor package "limma" that implements linear models for microarray data (Smyth, 2004). The *P* values were adjusted for multiple testing with Benjamini and Hochberg's method to control the false discovery rate (FDR). Probe sets showing at least 2-fold change and a FDR < 0.05 were considered significant.

Hcat map analyses were performed using Cluster 3.0 software (Stanford University) and visualized with Java Tree view software. Gene set enrichment analyses were done using differentially regulated genes with a P value, < 0.05 (Subramanian et al., 2005).

### Flow cytometry analyses:

Fluorescence activated cell sorting (FACS) analyses were performed on CyAn™ ADP instrument platform for flow cytometry at Flow cytometry core facility, EPFL. DL4 and Notch1 expression in DL4- and N 1-HeLa cells was determined using anti-DL4 and anti-N1 antibodies respectively. T cell development in the thymus was investigated using antibodies against CD4, CD8 and TCRβ. MZB cell development was monitored using antibodies against R220, CD21 and CD23. In brief, a single cell suspension was prepared from thymus and spleen. 1x10⁶ cells suspended in 50 µl of staining media (HBSS supplemented with 2% NCS and 25mM HEPES) and stained with appropriate antibody combinations by incubating on ice for 30 minutes.

To quantitate the percentage of apoptotic cells, AnnexinV and 7AAD staining was performed. Thymic cells were suspended in 300 µl of 1x AnnexinV binding buffer (BD Biosciences, San Diego, USA) and incubated with 10 µl of AnnexinV-Cy5 antibody and 10 µl of 7AAD (BD Biosciences, San Diego, USA). Samples were incubated for 15 minutes at room temperature. FACS was performed with in one hour of antibody staining.

Flow cytometry analyses were done on live cells by gating on forward scalter (FSC) and side scatter (SSC). Data were analyzed by FlowJo software (Tree Star, Ashland, OR).

### Alamar blue proliferation assay:

Alamarblue® proliferation assays were performed to determine the growth kinetics of Notch inhibitor treated cells. Alamar blue® consists of a cell permeable substrate resazurin. In metabolically active and proliferating cells, resazurin is converted to resorufin due to an intrinsic reducing power of live cells and produces a red fluorescence. Therefore production of resorufin serves as an indicator of the viability of the cell population.

Proliferation assays were performed by seeding 5000 cells/well in a 96 well plate. Cells were treated with DMSO or Notch inhibitors for different time intervals. Each treatment, for every time intervul was carried out in 8 replicates. To determine the growth kinetics, 10 µl of Alamar blue® (Invitrogen) was added to each well and incubated for 4 hours. Alamarblue readout was taken using Tccan F500 (Tecan) multiplate reader.

### Haematoxylin & Eosin staining:

Organs were harvested, fixed in 4% paraformaldehyde (PFA) overnight at 4°C and embedded in paraffin. Tissue sections were dewaxed and hydrated using decreasing concentration of ethanol (100%-70%) and finally in distilled water. Sections were stained with Hemotoxylin for 5 minutes, rinsed in acid alcohol for about 20 seconds and then rinsed in running water for 10 minutes. Sections were then stained with Eosin for 5 minutes, washed in water and dehydrated using increasing concentration or ethanol (70%-100%) and cleared in xylene solution. Sections were the mounted using mounting solution. Haematoxylin and Eosin stained sections were viewed and images were captured using Leica DMI4000 microscope.

### Alcian blue staining:

Intestinal tissue was flushed with ice-cold 1xPBS, fixed in 4% PFA. Tissues were embedded in paraflin and sectioned to a thickness of 4 microns. Intestinal sections were deparaffinized at 60°C and hydrated with decreasing concentration of alcohol (100% -70%) and finally washed in distilled water. Alcian blue staining was performed for 30 minutes at room temperature washed in running water and finally counterstained in nuclear fast red solution for 5 minutes. Tissue sections were then washed in running water dehydrated in 100% alcohol and cleared in xylene solution. Mounted sections were then viewed and images were captured using Leica DMI4000 microscope.

### In vitro γ-secretase activity assay:

The components of γ-secretase complex were overexpressed and Purified as described previously (Cacquevel et al., 2008). Briefly, The components of the γ-secretase complex were purified from CHO cell line γ-30 (expressing human PS1, Pen-2, Aph1 and Nicastrin). γ-secretase activity was measured by incubating the γ-secretase complex with rccombinant Amyloid precursor protein (APP) fragment containing a Flag tag on its C-terminus (APP C100-FLAG) or Notch based N100-FLAG peptide. To determine the GSI activity of novel Notch inhibitors, the above mentioned reaction mixture was incubated with the candidate chemical compounds and DAPT in an increasing concentration. Western blot were performed to detect APPC100-FLAG and N1C100-FLAG peptides and their cleaved products AICD and NICD respectively.

### Experimental mice:

Mice were kept and bred at Animal facility, EPFL, Lausanne. C57B16 mice were used to assess the intestinal toxicity of the chemical compounds. MMTV-ErB2/Neu-IRES Cre (FVB background) mice were obtained from Dr. William J Muller, McGill University, Montreal and genotyped using MMTV-ErbB2/Neu specific primers (Ursini-Siegel et al., 2008). NOD/SCIDγc ^{-/-}mice were bought from The Jackson Laboratory (USA) and were kept and bred at Animal facility, EPFL, Lausanne.

### Intestinal toxicity and effect on T-cell and B cell development:

C57B16 mice were intra peritoneal (i.p) injected with oil or 25 mg/kg of 13 or 10 mg/kg of CPA, once a day tor 5- 7 days. Mice were weighed using a weighing scale on day 0, day 3 and day 5. On day 8, intestinal tissue, spleen and thymus were harvested for analyses.

### Tumor transplantation assay:

The human leukemic cell lines RPMI 8402 and HPB ALL were transduced with a lentivirus containing luciferase gene constitutively expressed downstream of a CMV promoter, The human leukemic cell lines RPMI 8204 (0.5-1 x10⁶ cells) and HPB ALL (1 x10⁶) were suspended in 100 µl of ice-cold 1xPBS and kept on ice until the transplant. NOD/SCIDγc ^{-/-} mice were transplanted with the human leukemic cell lines by intravenous (i.v) injection. Mice were monitored for tumor development using Caliper IVIS (Xenogen) live imaging system. Briefly, the luciferase substrate luciferin (Biosynth, L-8820) was dissolved in 1xPBS and was injected (intra peritoneal) into the mice at a concentration of 150mg/kg of body weight. Mice were imaged 5 minutes after the luciferin injection using Caliper IVIS live imaging system.
At day 13-15, mice were treated with oil or 25 mg/kg of I3 on a daily basis. Images were captured at the end of the experiments.

Primary MMTV-ErbB2/Neu mammary tumors were harvested from the mice and a single cell suspension was prepared. 1x10^{b} primary tumor cells were suspended in 50 µl of 1x PBS and kept on ice. Three weeks old recipient FVB mice were cleared of their endogenous epithelium and tumor cells were injected into the empty fat pad. Tumor development in the recipient mice was monitored and tumor volumes were measured using digital caliper. Tumor volumes were calculated using following formula: 2 x length x (width)². Once the tumor reached a volume of about 100 mm³, recipient mice were treated with oil or 25 mg/kg of 13 on alternate days.

### Assay development

In order to identify novel modulators of the Notch pathway, Applicants have established a coculture assay in which DL4 ligand expressing HeLa cells were cultured with N1 HeLa cells, thereby activating the Notch pathway. The use of a DL4 and N1 HeLa cell coculture system mimics physiological conditions of cell-cell communication between ligand and receptor expressing cells. The *in vitro* generation of a controlled receptor-ligand assay system allowed Applicants to modify and monitor the Notch signal intensity by γ-seretase inhibitors

### DL4: N1 HeLa cell coculture activates Notch signaling

To set up a coculture assay, Applicants have established DL4 and N1 expressing stable HcLa cell lines. In brief, HeLa cells were transduced with a lentivirus containing DL4 cDNA downstream of the PGK promoter. The D4 expressing cell population was enriched by fluorescence activated cell sorting. Similarly, the N1 stable Hela cell line was established using a plasmid containing the mouse N1 cDNA followed by an IRES Puromycin selection cassette. This system allowed Applicants to select for only Notch1 expressing clones when selected using puromycin. The expression levels of DL4 and N1 proteins in the respective cell lines were detected using anti-DL4 and anti-N1 antibodies. Quantification of the protein levels by flow cytometry showed high level expression of DL4 and N1 compared to parental HeLa cells (Figure 1A and B).

To assess the Notch pathway activation potential of the stable cell lines, DL4 and N1 stable HeLa cells (DL4-HeLa and N 1-HeLa, respectively) were cocultured in 1:1 ratio in a 6-well plate and grown to confluency. The cocultured cells were treated with DMSO or DAPT (10 µM) for 24 hours. For comparison, parental HeLa cells were also cocultured with DL4-HeLa cells and were grown in the-presence or absence of DAPT for 24 hours. Western blot analyses for the active form of Notch 1 (NICD) using VAL1744 antibodies was performed and revealed only modest levels of NICD when parental HeLa cells were cocultured with DL4 HeLa cells (Figure 1C; lane 2 and 3), accounting for a low level of endogenous Notch1 in HeLa cells. On the other hand, in the absence of ligand (DL4-HeLa cells) or in the presence of GSI (DAPT) NICD levels were not detected, indicating a loss of Notch signaling (Figure 7C; lane 1 and 4 respectively). However, cocultures of DL4- and N1-HeLa cells revealed significantly higher levels of NICD, which can be blocked by DAPT treatment (Figure 7C; lane 5 and 6). Transient introduction of full length Notch1 cDNA into N1-Hela cells further enhanced the robustness of the coculture assay as indicated by the increased levels of NICD protein (Figure 1C; lane 7). Inhibiting the N1 cleavage with DAPT can abrogate the increase in Notch signaling activity (Figure 1C; lane 8). These results confirmed that high levels of the Notch pathway activation could be achieved in the DL4:N1 coculture assay that responds to GSI inhibition.

The establishment of DL4:N1 coculture assay in a 6 well plate format allowed Applicants to assess receptor-ligand interaction mediated Notch signaling activation. GSI (DAPT) treatment of the coculture system can block receptor-ligand interaction driven Notch signaling.

### Establishment of High-through put screening (HTS) compatible assay

Initially, DL4:N1 coculture assay was established in u 6-well plate. In order to set up a high-through put screen (HTS), the assay system was further optimized to robustly work in a 384 well plate format. The schematic diagram in Figure 2 summarizes key steps for the chemical compound.

The assay was scaled-down to a 384 well plate format for screening of chemical compound libraries (see schematics in Figure 2). In order to accomplish this, N1-HeLa cells were transfected with a reporter plasmids and N1 expression vector. Twelve hours later, chemical compounds were dispensed into a 384 well plate along with DMSO and DAPT as negative and positive controls. DL4- and N1-HeLa cells were mixed in a 1:1 ratio (5000: 5000 cells/well) and added to 384 well plates using multidropCombi plate dispenser. Luciferase readout was measured using dual luciferase assay system. To optimize and determine the reproducibility of the assay, half of the plate was treated with DMSO (192 wells) and the second half was treated with 10 µM DAPT (192 wells). As shown in figure 3, DAPT treatment led to a 10-fold downregulation of Notch signaling activation. As depicted in the figure 3 lower panel, any positive hits for the inhibitors of the pathway will fall into a window between positive and negative controls. The Z' value for both assays was higher than 0.5. A Z', value of >0.5 confirms the reliability and reproducibility of the assays for a HTS campaign.

### 1. Lasalocid

### 1.1.1 Lasalocid blocks Notch signaling and downregulates Notch target genes:

To validate the Notch inhibitory potential of Lasalocid, DL4:N1 coculture system was treated with an increasing concentration of Lasalocid. The cells were incubated in the presence of DMSO or Lasalocid for 24 hours and luciferase activity was measured using the dual luciferase assay system. As shown in Figure 4A, with an increasing concentration (2.5 µM-10 µM), Lasalocid induced a downregulation of the Notch driven lucifcrasc activity (Figure 4A).

To further test its ability to block Notch signaling, the human T-ALL cell line RPMI 8402 which has high Notch signaling activity, was treated with Lasalocid for 24 hours. Western blot analysis using the Val1744 antibody detecting the cleaved form of Notch1 (NICD) revealed that the treatment of RPMI 8402 cells with Laslaocid caused a decrease in the levels of NICD when compared to DMSO treated cells (Figure 4B). This decrease in NICD levels also translates into a downregulation of Notch target genes. QRT-PCR analysis using RNA from Lasalocid or DMSO treated RPMI 8402 cells showed a statistically significant downregulation of the Notch target genes Hes1, cMyc and Dtx1 (Figure 4C). The loss of NICD levels in the Lasalocid treated cells suggest that the mechanism of action of Lasalocid may involve the blockage of γ-secretase activity, thus leading to an absence of NICD. However this possibility was ruled out by an *in vitro* γ-secretase complex activity assay. Briefly, *γ*-secretase complex activity was reconstituted by purifiying and mixing of different components of the complex in appropriate ratios. The *in vitro* reconstituted complex is able to cleave Notch1 based NC100-FLAG peptide and Amyloid precursor pcptides (APPC100-FLAG) there by giving rise to NICD and AICD (Amyloid intracellular domain) respectively which can be blocked by DAPT. The treatment of γ-secretase complex incubated with Notch1 and Amyloid precursor peptides, with Lasalocid did not lead to a block in γ-secretase activity. Taking into account a decrease of NICD levels and intact γ-secretase complex activity upon Lasalocid treatment suggest that Lasalocid either acts upstream of S3 cleavage event or it may be inducing degradation or cleaved form of Notch1 receptor (NICD) to inhibit Notch signaling activation.

### 1.1.2 Lasalocid mediated inhibition of Notch-dependent growth of T-ALL cell lines:

Human T-ALL cell lines with activating mutations in the Notch1 receptor are dependent on continuous Notch signaling to sustain growth in cultures. If Lasalocid is able to block Notch signaling in these cell lines, it should also be able to impair their growth in cultures. To test this, human T-ALL cell lines, RPMI 8402, CEM, KOPTK1 and DND41 were seeded in a 96 well plate and treated with DMSO, DAPT, (10 µM) and Lasalocid (10 µM) for 5 days. To monitor the cell growth, Alamar blue readout was taken at day 0, 3 and 5. As shown in Figure 5, treatment of human T-ALL cell lines with DAPT and Lasalocid causes a statistically significant reduction in their growth potential (Figure 5).

### 1.1.3 Lasalocid induces differentiation of C2Cl2 myoblasts:

Notch signaling has been shown to be important in regulating the process of myogenesis. Activation of the Notch pathway in undifferentiated C2Cl2 myoblasts block their differentiation by suppressing MyoD, which is required for muscle cell differentiation. Thus overexpression of NICD blocks C2Cl2 myoblast differentiation and inversely, a blockage in the Notch pathway can induce C2Cl2 cell differentiation into MyoD and Mysoin Heavy Chain (MHC) expressing cells. Therefore, if Lasalocid is an inhibitor of Notch1 signaling, it should induce differentiation of C2Cl2 myoblasts into multinucleated MHC expressing myotubes. To that end, C2Cl2 myoblasts were grown to 100 % conflucncy. To maintain the cells in undifferentiated state, myoblasts were grown in the presence of 10 % scrum. Treatment of C2Cl2 cells with Lasalocid caused an upregulation of MHC and formation of multinucleated myotubes (Figure 6). These data suggest that possible inhibition of Notch signaling by Lasalocid induces myoblast differentiation.

### 1.1.4 Global gene expression profile of Lasalocid treated leukemic cells:

Applicants' *in vitro* data have shown that Lasalocid can block Notch signaling in a DL4:N1 coculture assay as well as in Notch1 -dependent human leukemic cell lines. Furthermore, the attenuation of the Notch pathway by Lasalocid in C2Cl2 myoblasts induced their differentiation. Next it was investigated the global gene expression pattern in a cell line treated with Lasalocid. The human leukemic cell line KOPTK1 was treated with DMSO, Lasalocid and DAPT for 24 hours (triplicates for each condition). Total RNA was extracted and hybridized to Affymetrix Human Gene 1.0 ST arrays (Affymetrix, Santa Clara, CA, USA) to determine changes in the gene expression profile between DMSO, DAPT and Lasalocid treated cells. The expression profiles were compared with DAPT that serves as a benchmark for a Notch inhibitor.

Affymetrix data was analysed using Cluster 3.0 program to cluster genes and arrays according to their relationship to each other. Venn diagrams were used to classify differentially or commonly regulated genes between Lasalocid and DAPT treatments. To create clusters and Venn diagrams, all the genes with p value < 0.05 were used, irrespective of the fold change values. Thus these analyses contain genes whose fold change upon treatment with the compounds may not be more than 2 fold (range from 1.0 - >2). As shown in Figure 7, Lasalocid treatment of KOPTK1 cells leads to a change in gene expression profile. Treatment of KOPTK1 cells with Lasalocid and DAPT leads to an upregulation of 865 and 136 genes respectively. Among these, 57 genes were upregulated by both Lasalocid and DAPT (Figure 7C). Similarly, Lasalocid treatment causes a downregulation of larger set of genes when compared to DAPT treatment (1590 genes for Lasalocid compared to 269 for DAPT). Among these 208 genes were regulated by both Lasalocid and DAPT (Figure 7D). The list of genes regulated by both Lasalocid and DAPT includes the Notch pathway genes such as Notch1, Dtx1, Hes1 and Myc (Figure 7A and B).

Furthermore the use of Gene Set Enrichment Analyses (GSEA) also helped Applicants identify alterations in different signaling cascades due to Lasalocid treatment. GSEA of the Affymetrix data showed an enrichment (FDR < 5 % and nominal p value < 0.05) of various components of Notch signaling in the DMSO treated KOPTK1 cells in comparison to Lasalocid treatment (Figures 8A and 8B). This data suggests Lasalocid treatment causes a downregulation of the Notch pathway that reflects as enrichment in DMSO treated samples. In addition to a downregulation of Notch signaling, Lasalocid treatment also caused a downregulation of several metabolic pathway. As an example, glycolysis and gluconeogenesis pathways were under represented in Lasalocid treated cells when compared to DMSO treated samples (Figure 8C). The list of downregulated metabolic pathways in Lasalocid treated cells includes Glycolysis metabolism, Citrate cycle, Pentose phosphate pathway, Fructose and Mannose metabolism, Galactose metabolism, Fatty acid metabolism and Purine metabolism. However it needs to be seen whether alteration in these metabolic pathways is a direct consequence of Lasalocid treatment or is an indirect consequence due to a block in proliferation of the cells.

In order to compare signaling pathways and networks differentially regulated by Lasalocid and DAPT, Affymetrix data from Lasalocid and DAPT treated cells was also analyzed using GeneGo Metacore program. These analyses were carried out using a stricter criteria i.e. only genes with p value <0.05 and a minimum 2 fold change in the gene expression were included for the analyses. This criterion helped to limit analyses to most significantly altered genes in terms of statistical significance and fold change. Analyses were done to compare pathways, networks or disease conditions which are enriched for genes uniquely regulated (positively and negatively) by Lasalocid or DAPT and commonly (positively and negatively) regulated by both compounds. In figure 9, data for Lasalocid and DAPT regulated genes enriched in network and disease condition are presented. Lasalocid treatment of KOPTK1 causes a significant alteration in genes involved in several signaling cascades in addition to the Notch pathway. These pathways include NK cell cytotoxicity, sodium transport, MIF (Macrophage migration inhibitory factor) signaling, Serotonin production and secretion, GnRH (Gonadotropin-releasing hormone) signaling and protein C signaling (orange bars in figure 9A). As shown in figure 9A, both DAPT and Lasalocid cause an alteration in the expression profiles of same genes involved in the Notch and ubiquitin degradation pathways (black bars). In addition, Lasalocid treatment also leads to an alteration in the expression profile of genes involved in several nervous system and immune system diseases (Figure 9B).

Furthermore, Lasalocid and DAPT treatments cause an enrichment of genes that are involved in leukocyte activation, serotonin pathway and mast cell activation (Figure 10B, black bars). However, KOPTK1 cells treated with Lasalocid but not DAPT, exhibit an enrichment of genes involved in A2A signaling, EGFR signaling, p53 mediated apoptosis, CDK5 signaling, CD137 signaling and G protein signaling (Figure 10A, orange bars).

Global gene expression profiling following Lasalocid and DAPT treatment showed that there are several pathway, networks and processes that arc regulated by both compounds, while there also pathways those are uniquely regulated by Lasalocid. These analyses also explains the large number of genes modulated by Lasalocid and may help in determining the side effects with Lasalocid treatment.

### 2. Cyclopiazonic acid (CPA)

### 2.1.1 CPA blocks Notch1 signaling activation in a concentration dependent manner:

To determine the Notch inhibitory activity of CPA, DL4:N1 cocultured HcLa cells were treated with an increasing concentration of CPA, ranging from 2.5 µM to 10 µM. As shown in figure 11A. CPA treatment of cells in the coculture assay attenuated Notch signaling in a concentration dependent manner. Western blot study of whole cell lysate from CPA treated cocultured DL4:N1 HeLa cells suggested that the loss of Notch activity is due to the absence of active form of Notch1 i.c NICD (Figure 11C). Furthermore, the CPA mediated inhibition of Notch pathway can be rescued by Notch1-intracellular domain (NICD) or Notch2-intracellular domain (N2-ICD) overexpression (Figure 11D). The loss of NICD upon CPA treatment suggests that CPA may act as a γ-secretase inhibitor (GSI). To determine whether CPA blocks Notch signaling by abrogating the S3 cleavage activity of γ-secretase complex, an *in vitro* γ-secretase complex reconstitution assay was performed. The *in vitro* reconstituted γ-secretase complex was able to cleave Notch1 based NC100-FLAG peptide and Amyloid precursor pcptidcs (APPC100-FLAG), which can be blocked by DAPT. However, CPA treatment of *in vitro* reconstituted γ-secretase complex did not block its S3 cleavage activity, thus ruling out a GSI role for CPA. These observations suggest that CPA mediated block in Notch signaling is due to its action prior to S3 cleavage event takes place. Interestingly, CPA mediated inhibition of Notch cascade appears to be specific for Notch1 signaling. In the coculture system, CPA blocks DL4-N1 mediated activation, but not DL4-N2 signaling activation (Figure 11B).

### 2.1.2 CPA inhibits Notch1 signaling by blocking S1 cleavage and membrane transport of the receptor:

Applicants' *in vitro* coculture assay studies and γ-secretase complex reconstitution assay has suggested that the potential mechanism of action of CPA involves its activity prior to S3 cleavage of Notch1 receptor hy γ-secretase complex at the plasma membrane. The potential mechanism of action of CPA, prior to S3 cleavage may involve a blockage of S1 cleavage hy furin-like convertase, a block in membrane transport of the receptor or a block in S2 cleavage by ADAM10/17 at the plasma membrane. Therefore, in an attempt to elucidate the mechanism of action of CPA in mammalian cells. HeLa cells were transfected with an expression vector containing a cDNA encoding for Notch1-GFP fusion protein. Notch1-GFP transfected cells were treated with 10 µM CPA or DMSO for 24 hours. The subcellular localization of the Notch1 receptor was monitored by fluorescence microscopy. As shown in figure 12A, DMSO treated HeLa cells express Notch1-GFP fusion protein in a punctate form around nuclei and possibly at the plasma membrane. On the other hand, CPA treatment of these cells leads to a diffused signal within the cytosol and a loss of punctate expression pattern of the protein (Figure 12A). To further determine the effect of CPA on endogenous Notch1 receptor, the human leukemic cell line RPMI 8402 was treated with DMSO or CPA for 24 or 48 hours. Western blot analysis was performed probing for Notch1 with an antibody that binds to C-terminus of Notch1 to detect full length Notch1 and transmembrane bound intracellular domain of Notch1 proteins. The western blot data in figure 12B shows that CPA treatment leads to a slight increase of full length Notch1 accompanied by a loss of transmembrane bound intracellular domain of Notch1. The use of Val1744 antibodies (that recognizes cleaved, thus active form of Notch1) confirmed the loss of NICD in these cells. Taken together these data provide some preliminary evidence that CPA mediated inhibition of Notch1 standing could be due to ablation of S1 cleavage by furin-like convertase and aberrant receptor transport to the cell membrane. A second possible mechanism of blocking Notch signaling could be an induction of NICD degradation following S3 cleavage. This hypothesis can be tested by the use of proteasomal degradation inhibitor such as MG-132 or lysosomal degradation inhibitors in combination with CPA. If CPA blocks Notch signaling by inducing degradation of NICD, the use of proteasomal or lysosomal degradation inhibitors should rescue CPA mediated inhibition of the pathway.

### 2.1.3 CPA mediated inhibition of Notch signaling downregulates Notch target genes:

Next Applicants sought to determine whether the CPA mediated inhibition of the Notch pathway translates into a downregulation of the Notch target genes. To do so, the human T-ALL cell line RPMI 8402 was treated with DMSO. or CPA for 24 hours. QRT- PCR analyses showed a significant downregulation of Notch target genes Hes1, cMyc and Dtx1 (Figure 13A). This downregulation of target genes correlates with the loss of active form of Notch (NICD) (Figure 13B). Furthermore, Chromatin Immunoprecipitation (ChIP) assay using the Val1744 NICD antibody showed an absence of NICD recruitment to CSL/RBP-jk binding sites in the Hes1 promoter (Figure 13C and D). Therefore in the RPMI 8402 T-ALL cell line, CPA blocks Notch signaling by inhibiting S1 cleavage/membrane transport of the Notch1 receptor, which leads to a loss of NICD and ultimately downregulation of Notch target genes.

### 2.1.4 CPA induces growth arrest in Notch1 dependent T-ALL cell lines:

Once CPA was determined to inhibit the Notch1 pathway activation, Applicants sought to investigate whether CPA treatment could also block proliferation of additional Notch1-dependent T-ALL cell lines. To this purpose, Applicants made use of a panel of human T-ALL cell lines with known mutations in the Notch 1 receptor and whose growth properties have been shown to be dependent on active Notch signaling.

To determine the anti-proliferative potential of CPA on Notch1-dependent cell lines, 5000 cells (RPMT 8402 or CEM or DND41 or KOPTK1) were seeded per well in a 96 well plate. The cells were grown in the presence of 10µM of CPA. As negative and positive controls, cells were also treated with an equal concentration of DMSO and DAPT respectively. The proliferation of these cell lines was measured using Alamar blue dye from 8 different replicates for each time point. As shown in figure 14, CPA treatment of T-ALL cell lines caused a proliferation arrest when compared to DMSO treated cells.

### 2.1.5 C2C12 myoblasts treated with CPA undergo differentiation to form multinucleated myotubes:

As mentioned in a previous section, activated Notch signaling maintains C2C12 myoblasts in an undifferentiated state. A blockage in the Notch pathway induces differentiation of the myoblasts into MI1C expressing multinucleated myotubes. This system was used as a functional assay to determine the ability of novel Notch inhibitors to induce myoblast differentiation by blocking Notch signaling. As expected, when C2C12 cells were grown to confluency in the presence of CPA and DAPT, the cells started to differentiate and form MHC positive multinucleated myotubes compared to cells grown in the growth medium containing 10% serum (Figure 15). This functional assay confirms the Notch1 inhibitory effect of CPA.

### 2.1.6 Global gene expression comparison between CPA and DAPT:

The mechanism of action of CPA in blocking Notch signaling is different from that of DAPT. While DAPT blocks S2 cleavage of Notch receptors by inhibiting the γ-secretase complex, CPA appears to specifically inhibit Notch1 mediated signaling by blocking its S1 cleavage and membrane transportation. Due to these differences, Applicants hypothesize, that these two compounds may also be regulating diverse signaling cascades in addition to Notch signaling. The identification of differentially and commonly modulated signaling pathways by both CPA and DAPT can help Applicants determine potential side effects of using CPA in *in vivo* studies. In order to accomplish this, an Affymetrix GeneChip Array was performed on RNA extracted from CPA, DAPT and DMSO treated KOPTK1 cells. Clustering analyses and Venn diagrams were prepared using all the genes with p value <0.05 (irrespective of fold change). Figure 16A shows heat map analyses of 56 genes commonly downregulated by CPA and DAPT in comparison to DMSO treated samples. As expected, CPA and DAPT treatment led to a downregulation of known Notch target genes and other components of the Notch pathway (Figure 16B). To determine the numbers and identity of genes differentially regulated by CPA and DAPT, Venn diagrams were prepared using all the genes with p value < 0.05 (irrespective of fold change). These analyses showed that 2122 and 76 genes were uniquely upregulated by CPA and DAPT respectively, while 60 genes were upregulated by both CPA and DAPT (fig 16C). The list of downregulated genes include, 207 genes commonly regulated by both CPA and DAPT, while CPA and DAPT differentially regulated 2460 and 62 genes respectively (Figure 16D).

To further identify the pathways and networks corresponding to genes regulated by CPA and DAPT, GeneGo analyses were performed using a stricter criterion. For this set of analyses, only genes with p value of <0.05 and demonstrating a fold change of> 2 were included in the list. As shown in figure 17A, treatment of cells with both CPA and DAPT caused an enrichment of genes implicated in the Notch, BCR and ubiquitin proteasomal degradation pathways (black bars). In addition, CPA treatment also caused an enrichment of unique genes involved in Notch, MIF, Melatonin, Erythropoictin and protein C signaling, while DAPT led to an enrichment of unique genes involved in BCR and complement pathways. GeneGo analyses for the genes involved in human diseases showed that both CPA and DAPT treatment led to an alteration of expression profiles of common genes involved in human leukemias (e.g. lymphocytic, B cell and lymphoid leukemias) (Figure 17B). Similarly, both chemical compounds also appear to impinge upon similar signal transductions and cellular processes. KOPTK1 cells treated with CPA and DAPT exhibit an alteration in the genes involved in PIP, IP3, LI-7 and NFAT signaling (Figure 18A, black bars) as well as genes implicated in vesicle and endosomal trafficking, leukocyte activation and B cell proliferation (Figure 18B, black bars). Therefore, Affymetrix array analyses, suggest that CPA may be able to regulate multiple pathways in addition to Notch signaling. However, it remains to be seen whether this is due to a direct effect of CPA on the multiple pathways or an indirect effect amplified by an arrest in the proliferation of the cells.

### 2.1.7 In vivo effects of CPA in the mice:

Once the role of CPA in blocking Notch signaling was established *in vitro* systems, Applicants decided to determine its effect in an *in vivo* context. As discussed previously, a pan blockage of Notch signaling causes intestinal toxicity by driving goblet cell metaplasia and perturbing the proliferation of crypt progenitors. Applicants *in vitro* data indicates that CPA treatment inhibits specifically Notch1 mediated pathway activation but not Notch2. Applicants thus speculated that the treatment of mice with CPA would not disturb the normal homeostasis of the intestine. Mice were injected with 10 mg/kg of CPA or oil as vehicle, continuously for 7 days. On day 8, animals were sacrificed and intestinal tissue was analyzed by Alcian blue staining to detect mucous producing goblet cells. As shown in figure 19A, treatment of mice at 10 mg/kg of CPA for 7 days did not compromise the structural integrity of the intestine. The numbers of goblet cells in each villus were comparable to control group (Figure 19A). In addition the changes in the body weight were also monitored following oil and CPA treatment. Mice were treated for 5 days and body weights were measured on day 0, 3 and 5. The percentage changes in the body weights are depicted in figure 19B. Treatment of mice with CPA did not lead to any significant difference in the body weight when compared to animals treated with the oil (Figure 19B). The lack of goblet cell metaplasia in the mice could be due to the fact that CPA blocks only Notch1 mediated signaling but not via Notch2.

### 3. 6-4[-(tertbutyl)-phenoxy] pyridine-3-amine (13)

### 3.1.1 I3 inhibits NICD mediated activation of Notch signaling:

To validate the Notch inhibitory activity and determine the IC50 value of the 13 compound, the DL4:N1 coculture assay system was used. The cells in the coculture assay were treated for 24 hours with an increasing concentration of 13 (2 -10µM). The activation of the Notch pathway was measured using a Notch driven luciferase reporter assay. As shown in figure 20A, 13 blocks Notch signaling in a concentration dependent manner with an TC50 value in the lower µM range.

To determine whether over expression of NICD can rescue I3 mediated inhibition of Notch signaling, HeLa cells were co-transfected with a NICD expression plasmid and 12xCSL luciferase construct. The transfected cells were treated with an increasing concentration of I3 and DAPT. Surprisingly, treatment of NICD expressing cells with I3 could block pathway activation in a dose-dependent manner, while DAPT had no effect on the signaling activation (Figure 20B). This data suggests that I3 mediated inhibition of the Notch pathway is due to its activity downstream of the S3 cleavage event.

Next Applicants investigated whether 13 could block pathway activation via other Notch receptors or is it specific to Notch1 signaling. To address this, a coculture assay was used where Notch signaling was activated via DL4:N1 or DL4:N2 ligand receptor pairs. The treatment of cells in these two coculture assays with I3 caused an inhibition of Notch signaling via both DL4:N1 and DL4:N2 ligand-receptor pairs (Figure 20C). Similarly, 13 could also block pathway activation by Notch1-intracellular domain (NICD) and Notch2-intraccllular domain (N2-ICD), suggesting that 13 is not specific for NICD mediated activation (Figure 20D).

### 3.1.2 I3 does not block nuclear localization of NICD:

*In vitro* data from NICD transfected HeLa cells suggested that I3 blocks Notch signaling by acting downstream of S3 cleavage event. This raises several possibilities about the mechanism of action of I3. For example, 13 treatment could impair nuclear localization of the NICD. A second possible mechanism of inhibition could be targeting of one or more individual components of the transcriptional activation complex in the nucleus. To test whether T3 has an impact on nuclear transport of NICD, HeLa cell were transfected with a NICD-GFP fusion construct and treated with DMSO and 13. This allowed Applicants to follow transport of fusion protein within the cell. In parallel, the 13 mediated pathway inhibition was determined by Notch driven luciferase measurement (data not shown). Microscopic studies showed that in DMSO treated cells NICD-GFP fusion protein translocate to the nucleus, which was not perturbed upon 13 treatment (Figure 21. This data rule out nuclear exclusion of NICD as a mechanism of action of I3.

### 3.1.3 Over expression of MAML1 above a certain threshold can rescue I3 induced Notch signaling inhibition:

As I3 mediated blockage of Notch signaling does not involve nuclear exclusion of NICD, Applicants addressed whether 13 blocks interaction and thereby sub-nuclear localization of NICD, MAML1 and CSL-RBP-jk (all parts of the core transcriptional activation complex). To resolve this, HcLa cells were co-transfected with 800 ng of NICD-GFP plasmid, 1 µg of MAMLI-FLAG expression vector and grown on cover slips. The transfected HeLa cells were treated with DMSO or T3 (10µM) for 24 hours. The ability of 13 to block Notch activation at this concentration of NICD and MAML1 was verified by Notch driven luciferase measurement (data not shown). Following treatment, the cells were fixed with 4% PFA, blocked with 1 % BSA and stained with antibodies against FLAG tagged MAML1 and CSL-RBP-jk. NICD-GFP fusion protein was visualized by tracing the GFP protein. As shown in figure 22A, when expressed alone, NICD-GFP protein was localized in the nucleus in a diffused manner and T3 treatment did not alter its nuclear localization. However overexpression of NICD-GFP and MAML1 led to co-localization of both proteins into sub-nuclear compartments (possibly nuclear bodies). The I3 treatment of the cells did not perturb the translocation and co-localization of NICD-GFP and MAML1 into sub-nuclear compartment (Figure 22B).

Similarly, the location of CSL/RBP-jk was investigated using antibodies specific against this protein. As shown in figure 22C, MAML1-FLAG and CSL/RBP-jk co-localized in sub-nuclear compartments and 13 treatment did not perturb their distribution in the nucleus (Figure 22C). This data suggest that 13 treatment does not perturb co-localization of components of the Notch transcriptional activation complex in the nucleus. However, whether it blocks interaction between various components of the complex still need to be investigated.

To further investigate the mechanism of action of 13, Applicants speculated that I3 might be targeting one of the components of the transcriptional activation complex. The over expression of this target protein may titrate out I3 compound and thus rescue I3 induced pathway inhibition. To address this question, HeLa cells were co-transfected with 800 ng of NICD-GFP plasmid and with an increasing amount (0, 1 and 3 µg) of MAML1-FLAG expression vector. The Notch pathway activation was measured by introducing 12xCSL luciferase plasmid. As shown in figure 23, in HeLa cells transfected with NICD alone or NICD + I µg of MAML1, I3 treatment can block Notch signaling in a concentration dependent manner. However, when the amount of MAML1 plasmid was increased to 3µg, I3 treatment was no longer able to inhibit the activation of Notch signaling (Figure 23). Therefore, over expression of MAML1 above a certain threshold could rescue T3 mediated inhibition of the Notch pathway. This data suggest that MAML1 itself might be the target of I3 compound. An increase in the concentration of MAML1 may be able to titrate out the inhibitor and thus rendering it incapable of blocking the signaling cascade.

### 3.1.4 13 treatment decreases Notch signaling in human cancer cell lines:

Aberrant activation of Notch signaling plays an important role in tumor initiation and/or maintenance of human cancers. To determine whether T3 treatment can block Notch signaling in human cancer cells, various cancer cell lines (T-ALL cell lines RPMI 8402, HPBALL, KOPTK1 and pancreatic cancer cell line PANCI) were treated with 13 for 24 hours. The effect on Notch signaling was determined by measuring the expression levels ofNotch target genes. I3 treatment of human cancer cell lines (RPMI 8402, HPBALL, KOPTK1 and PANC1) for 24 hours and subsequent analyses of Notch target genes by qRT-PCR or Western blot analyses showed that T3 induced a statistically significant downregulation of Notch target genes such as Hes1, cMyc and Dtx1 at the mRNA as well as at the protein levels (Figure 24). The downregulation of Notch target genes correlates with reduced levels of NICD (Figure 24B, C and D).

As treatment of the human T-ALL cell lines and PANC1 pancreatic cancer cell line with I3 induced a downregulation of Notch signaling, Applicants questioned whether this inhibition of the pathway translates into growth arrest in cancer cells. To this end, RPMI 8402, KOPTK1 and PANC1 cell lines were grown in the presence or absence of I3 for several days and their proliferative index was measured using the Alamar blue assay. In addition, B-lymphocyte RAJI cell lines with no known Notch mutations were used as a control. As shown in figure 25,13 and DAPT treatment induced a significant proliferation block in T-ALL cell lines RPMI 8402, KOPTK1 and pancreatic cancer line PANC1. However neither DAPT nor 13 had any effect on the proliferation of Notch-independent RAJI cells (Figure 25).

### 3.1.5 13 but not DAPT blocks Notch signaling in NICD overexpressing human T-ALL cell line.

As shown in section 3.1.1, I3 can block NICD mediated activation of the Notch pathway. To determine whether I3 can also induce a proliferation block in NICD overexpressing cells, the human T-ALL cell line DND41 (DND41-Parental) was transduced with a NICD expressing lentivirus to generate DND41-NICD cell line. These two cell lines were treated with DMSO, I3 and DAPT. The treatment of DND41-parental cell line with DAPT and I3 led to a downregulation of Hes1 when compared to DMSO treated cells. However, when DND41-NICD cells were treated with DMSO, I3 and DAPT, only 13, but not DAPT treatment caused a downregulation of Hes1 (Figure 26A). In addition, these two cell lines were also monitored over several days for anti-proliferative effects of 13 and DAPT. It was observed that while both 13 and DAPT treatment caused a significant proliferative block in the DND41-Parental cell line (Figure 26B), only 13 was able to induce a growth arrest in DND41-NICD cells (Figure 26C). This data further strengthen the notion that the 13 compound can block NICD mediated pathway activation and proliferation in human cancer cells.

### 3.1.6 I3 mediated Notch signaling inhibition induces C2C12 myoblast differentiation:

To further confirm the Notch inhibitory potential of I3 in different systems, C2C12 myoblast differentiation was used as a functional assay. The Notch pathway activation in C2C12 myoblasts rctains them in an undifferentiated state, while abrogation of Notch signaling induces their differentiation. C2C12 myoblasts were treated with DMSO, I3 and DAPT and grown to 100% confluency for 3 days. After three days, cells were fixed and stained with antibodies against Myosin Heavy Chain (MHC) protein. Cell nuclei were counterstained with DAPL C2C12 myoblasts grown in the presence of 10% serum (growth medium) maintain their undifferentiated state, while the cells treated with DAPT and I3 started to differentiate into multinucleated MHC positive myotubes (Figure 27).

### 3.1.7 I3 does not impede upon Wnt and Hedgehog signaling cascades:

One of the concerns about the activity of the chemical compound I3 is its specificity towards the Notch signaling pathway. In order to test whether I3 could also block other developmental pathways, Applicants have tested its ability to block the Wnt and Hedgehog signaling pathways. In summary, to measure Wnt signaling, HeLa cells were transfected with a plasmid containing a promoter consisting of TCF/LEF binding sites and thereby driving the expression of a luciferase gene (TOP-luciferase). To activate the Wnt pathway, a plasmid encoding for β-catenin was co-transfected into HeLa cells. The co-transfected cells were incubated in the presence or absence of the I3 chemical compound. As shown in figure 28A, transient introduction of β-catenin leads to an upregulation of Wnt signaling as measured by β-catenin-TCF/LEF driven luciferase activity. Importantly, 13 treatment of cells with activated Wnt signaling does not block the Wnt pathway activation (Figure 28A).

Using a similar strategy, Hedgehog signaling was activated in HeLa cells by introducing the Gli1 transcription factor and pathway activation was monitored using a promoter sequence containing Gli1 binding sites driving the luciferase expression. The treatment of these cells with I3 did not inhibit the Hedgehog signaling cascade (Figure 28B). Taken together these data suggest that the I3 chemical compound may not impair other developmental pathways and might be specific for Notch signaling inhibition. However il still needs to be determined whether the resistance of Wnt and Hedgehog signaling towards I3 is cell type specific or whether it is a general phenomenon.

### 3.1.8 Effect of 13 treatment on global gene expression pattern:

The newly identified Notch inhibitor 13 blocks the Notch pathway possibly by disrupting or modifying the downstream transcriptional activation complex downstream of Notch signaling. Applicants decided to study the impact of I3 on global gene expression profiles of the 13 treated cells. This analysis can help Applicants identify the signaling cascades and cellular processes altered due to the effect of I3. In addition, this may also indicate the potential mechanism of action. For example, if 13 were able to block the general transcription machinery, treatment of cells with 13 would lead to an enrichment of several canonical signaling cascades. To determine changes in the gene expression profiles due to I3 treatment, KOPTKI T-ALL cells were treated with 13, DAPT and DMSO for 24 hours. Total RNA extracted from these cells was subjected to Affymetrix geneChIP array. Clustering analyses of all the genes with a p value < 0.05 (irrespective of fold change in gene expression levels) led to the identification several genes commonly regulated by 13 and DAPT and uniquely regulated by I3 alone. In figure 29A, a heat map analyses of the top 50 genes commonly regulated by both I3 and DAPT is presented. The heat map analyses of Notch pathway components also revealed that both DAPT and I3 led to a downregulation of Notch signaling (Figure 29B). However, Venn diagram analyses of I3 and DAPT regulated genes showed that T3 regulates almost 15 times more genes than DAPT (Figure 29C and 29D). Even though several genes enriched in the I3 treated cells do not show a fold change of more than 2 in their expression level, but it still indicates that T3 impinges upon several signaling cascades in addition to the Notch pathway.

In order to identify signaling pathways represented by the I3 regulated genes GeneGo analyses were performed. As shown in figure 30A, I3 or DAPT treatment of KOPTK1 cells caused an enrichment of genes involved in ubiquitin proteasomal degradation and the Notch pathway (Figure 30A, black bars). Similarly, I3 or DAPT treatment appears to have an impact on common cellular processes such as negative regulation of leukocyte activation, Serotonin production/secretion and mast cell activation (Figure 31B, black bars). However, I3 treatment also leads to an alteration in the expression of genes involved in MIF, Protein C, TICAM1, ACM, CD28, CD40 and EGFR signaling (Figure 30A and Figure 31A, orange bars) as well as genes implicated in multiple sclerosis, demyelinating autoimmune disease and nervous system diseases (Figure 30B, orange bars).

The unique enrichment of genes involved in several pathways and disease conditions explains the large numbers of differentially expressed genes upon I3 treatment. However it remains to be seen, whether the differential regulation of genes observed is a direct consequence of I3 treatment or an indirect effect due to deregulation of one or more cellular processes.

### 3.1.9 In vivo effects of 13 in C57BL6 mice:

Notch signaling regulates homeostasis of several organs during development. For example, Notchl mediated pathway activation is essential for T cell development in the thymus (Radtke ct al., 1999). However; the Notch1 driven T cell development docs not appear to be dependent on MAML1, as the loss of MAML1 did not perturb T cell development in the mice. This could be due to a compensatory mechanism by MAML2 and MAML3 family members for the loss of MAML1. In the spleen, Notch2 driven signaling exclusively via MAML1 is required for MZB cell development. Genetic ablation loss of Notch2 and MAML1 cause a block in the development of MZB cells. In addition, Notch signaling via both Notch1 and Notch2 is essential for the maintenance of the crypt compartment. A compound genetic ablation of Notch1 and Notch2 in the intestine leads to goblet cell metaplasia. Applicants therefore, investigated whether I3 could impair above-mentioned Notch-dependent developmental processes.

In *in vitro* culture assays, chemical compound 13 was able to block Notch1 and Notch2 mediated pathway activation. Therefore, Applicants hypothesized that treatment of mice with 13 may lead to a goblet cell metaplasia of the intestine. To test this hypothesis, mice were intra peritoneally (i.p) injected with 25 mg/kg of I3 for 7 consecutive days. On day 8, animals were sacrificed and intestinal tissues were fixed and embedded in paraffin. Histological analyses were carried out using Alcian blue to stain for goblet cells. Surprisingly, despite its ability to block both Notch1 and Notch2 mediated pathway activation in *in vitro* cultures, the intestinal tissue of I3 treated mice was completely normal with intact architecture and no indication of goblet cell metaplasia (Figure 32A). Similarly, the effect of 13 on body weight changes was also monitored. Mice were injected for 5 consecutive days with 25 mg/kg of I3 and the changes in body mass were recorded. As shown in figure 32B, treatment of mice with 13 did not cause a loss in the body weight.

### 3.1.10 Compound I3 does not block T cell development in the thymus:

One of the consequences of the loss of Notch1 signaling in the thymus is a block in T cell development and a concomitant accumulation of B cells. However, Notch1 driven T cell development is independent of MAML1 transcription coactivator due to a redundancy in the function of MAML family members. The loss of MAML1 alone does not perturb T cell development. The chemical compound 13 blocks Notch signaling activation downstream of both Notch1 and Notch2 receptors. In addition, 13 appears to exert its Notch inhibitory activity by targeting MAML1 transcription activator. Therefore Applicants expected, I3 treated mice to mimic the MAML1 deficient phenotype and it should not effect T cell development in the thymus. To test this hypothesis, wild type mice were treated with 13 (25mg/kg i.p.) for 7 consecutive days and thymus development was analyzed the following day. Thymic cells were analyzed by flow cytometry using antibodies against CD4 and CD8. Furthermore, since loss of Notch1 signaling causes an accumulation of B cells in the thymus, Applicants also quantitated the number of B cells (B220⁺ cells) following I3 treatment. As shown in figure 33A, treatment of mice with 13 caused a modest reduction in CD4' CD8⁺ double positive cells. The reduction in DP compartment was accompanied with a slight increase in CD4 and CD8 single positive and double negative (DN) compartments. However, this reduction of DP T cells was not accompanied with a significant accumulation of B cells in the thymus (Figure 33A lower right panel). The phenotype observed in 13 treated thymocytes did not recapitulate loss of Notch1 phenotype observed in Notch1 deficient thymocytes (Radtke et al., 1999). The reduction in CD4⁺CD8⁺ DP compartment could be due to a general cytotoxic effect of 13. To determine the cytotoxic effects of 13, the percentage of apoptotic cells was determined using AnnexinV and 7AAD staining. As shown in figure 33B, 13 treatment causes an increase in apoptosis of thymocytes in the thymus.

### 3.1.11 I3 induces transient splenomegaly with concomitant erythrocytosis:

The Notch pathway activation via Notch2 receptor signaling regulates the generation of MZB cells in the spleen. In addition, Notch2 receptor drives MZB cell development via MAML1 transcription coactivator. In *in vitro* studies, the chemical compound 13 appears to block both Notch1 and Notch2 signaling activations and this inhibition appears to be mediated via MAML1. Therefore, Applicants speculated that the *in vivo* treatment of 13 will inhibit Notch2 signaling and thus should block the generation of MZB in the spleen. To Applicants' surprise, mice treated with I3 were found to develop splenomegaly when compared to the control group (Figure 34B) with a significant increase in the total cellularity of the spleen (Figure 34C). This phenotype was accompanied by an accumulation of CD71⁺ Ter119' erythrocytes in the spleen (Figure 34D and E). However the accumulation of erythrocytes and resulting splenomegaly was only transient. Mice were treated as described before with I3 for 7 days but were then left to recover for another 7 days. Treated animals were subsequently analyzed on day 15 to assess the gross morphology of the spleen and cellular composition (Figure 35). As shown in figure 35B, the size of the spleen and total number of spleen cells gradually started to return to normal levels observed in the control mice (Figure 35C). Furthermore, following the recovery time, the numbers of CD71⁺ Ter119⁺ erythrocytes also started to return to normal levels as observed in the oil treated mice (Figure 35D and E). This data show that one of the side effects of 13 treatment in mice is the development of splenomegaly and erythrocytosis that can be reversed by incorporating a recovery phase in the treatment regiment.

### 3.1.12 I3 treatment induces a block in MZB cell development:

Applicants hypothesized that I3 mediated inhibition of Notch2 signaling should lead to a block in MZB cell development in the spleen. MZB cell development was assessed by flow cytometry staining of splenocytes with antibodies directed against B220, CD21 and CD23. As shown in figure 36B treatment of mice with I3 leads to a reduction in the percentage of B220⁺ B cells in the spleen. The observed reduction in the percentage of B220⁺ B cells can be simply an artifact due to the accumulation of erythrocytes in the spleen at this stage. Therefore, to determine whether the observed reduction in the percentage of B cells is due to direct effect of T3 treatment, absolute numbers of B cells were calculated following I3 treatment. The calculation of absolute numbers of B cells in the spleen revealed a reduction in the B cells following I3 treatment (Figure 37B). Flow cytometry analyses for MZB cells within the B cell compartment showed a significant reduction (from about 10% to 0.368%) of MZB cells in the spleen (Figure 36B). However this reduction in MZB cells could be due to the presence of reduced numbers of B220' B cells. The comparison of absolute numbers of B cells (2 fold reduction in 13 treated mice compared to control mice) and MZB cells (20 fold reduction in I3 treated mice compared to controls) suggested that the reduction in the B220⁺ B cells alone could not account for the loss of MZB cells in the spleen (Figure 37B).

To further rule out that a decrease in the numbers of B220⁺ B cells is responsible for the loss of MZB cells, mice were treated for 7 consecutive days and let to recover for another 7 days and analyzed at day 15 (Figure 36C and 37C). Flow cytometry studies following recovery period showed that B220⁺ B cells return to a normal levels both in terms of percentage and absolute cell numbers in the spleen (Figure 36D left panel and figure 36D left panel). However the percentage and absolute numbers of MZB cells in the spleen remained low even after recovery period (Figure 36D right panel and figure 37D right panel). This data suggest that the loss of MZB cells observed in the spleen is not due to a reduction in the B cells, but probably is due to a blockage in Notch2 mediated signaling by 13 chemical compound. Therefore, I3 mediated block in MZB cell development mimies loss of Notch2 and MAMLI phenotype. However, it is still need to be seen, whether 13 exert its Notch inhibitory effect only via MAML1 or it could block Notch signaling via other MAML family members as well.

### 3.1.13 13 treatment slows tumor growth of human T cell leukemia in a xenotransplantation model:

Activation of Notch signaling due to activating mutations in different components of the pathway are known to cause more than 50% of the human T cell acute lymphoblastic leukemias. Therefore, Applicants decided to investigate the anti-cancer activity of the chemical compound I3 in Notch driven human T cell leukemia *in vivo.* To achieve this goal, xenotransplant models of human leukemia were established using NOD/SCID γc^{-/-} mice. Human T-ALL cell lines HPB ALL and RPMI 8402 were used for this purpose. The HPB ALL cell line harbours a L1575P mutation in the heterodimerization domain and an insertion in the PEST domain of the Notch1 receptor, thereby constitutivcly activating the Notch1 signaling. Similarly, RPMI 8402 cells exhibit ligand independent Notch signaling activation due to an insertion at 1584 a.u residue in the heterodimerization domain and also an inactivating mutation (R465H) in the E3 ligase FBW7. Both these cells line were found to respond to 13 treatment in *in vitro* culture assays in terms of proliferation and/or downregulation of the Notch target genes. To determine whether these cell lines establish leukemia in a xenotransplant setting, one million cells from each line were intra venously (i.v) injected into NOD/SCIDγc^{-/-} mice. The animals developed leukemia with 100% penetrance and die within 4 weeks after transplantation. Histological analyses revealed that RPMI 8402 cells primarily infiltrate to the liver while IIPB ALL cells infiltrate to the kidneys after homing intro the bone marrow (Figure 38 A and B).

Once RPMI 8402 and HPB ALL cell lines were shown to develop leukemia in a xenotransplantation assay, they were transduced with a lentivirus constitutively expressing luciferase gene. This allowed Applicants to visualize and monitor the leukemia progression in the mice using the Caliper IVIS (Xenogen) live imaging detection system. In order to determine the anti-cancer efficacy of I3 in established tumors, a maintenance experiment was performed. One million HPB ALL cells were injected (i.v) into NOD/SCID γε^{-/-} mice. Mice were monitored for leukemia development by detecting luciferase expressing leukemic cells. Once the disease was established around day 15, the mice were split into two groups. One group was treated with oil as a control and the second group was treated with 25 mg/kg of I3 on a daily basis. As shown in figure 39A, the mice treated with oil develop leukemia with 100% penetrance while leukemia in the 13 treated mice did not progress at the same rate as in the oil treated group (Figure 39A). Furthermore, majority of the oil treated mice die around day 28, while 13 treated mice started to die of the disease past 30 days with 50% survival rate by day 38 (Figure 39B).

Similarly in a preliminary experiment, NOD/SCID γc^{-/-} mice were transplanted with 5 x 10⁵ RPM1 8402 cells and treated with oil or I3 following the establishment of the disease. As shown in figure 40, animals treated with oil, developed leukemia while I3 treated mice were free of the disease. Furthermore, histological analyses revealed that in oil treated mice, leukemie cells progressed to infiltrate the liver, but I3 treated mice did not develop any metastatic lesions in the liver (Figure 41A). Since these animals were treated with the chemical compound 13 for 27 days, the intestinal tissue was analyzed to detect any toxicity in the gut. Alcian blue staining of intestinal tissue did not reveal any abnormality in the goblet cell numbers and intestinal architecture (Figure 41B).

Taken together Applicants' data from xenotransplantation model for human leukemia suggest that 13 has the ability to slow down disease progression of an already established leukemia. Because of its ability to impact tumor progression, I3 may be a suitable candidate for further development as an anti-cancer agent.

### 3.1.14 MMTV-ErbB2 mouse mammary tumors exhibit Notch signaling activation and effect of 13 on mammary tumor progression.

In human breast cancer, high levels of Notch1 and Jagged1 proteins correlated with a poor survival of breast cancer patients. In addition, activation of Notch signaling in human breast cancer also facilitates bone and lung metastasis. Therefore in order to determine anti-cancer potential of chemical compound I3 in breast cancer, a mouse model of breast cancer was investigated. The mouse mammary tumor virus (MMTV) driven overexpression of ErbB2 is known to cause mouse mammary tumors. MMTV-ErbB2 transgenic mice develop mammary tumors with a latency of about 5-6 months along with the development of lung metastasis. One of the characteristics of MMTV-ErbB2 mouse mammary tumors is the presence of predominantly luminal epithelial cell types. Notch signaling is known to drive luminal cell differentiation from mouse mammary stem cells. Therefore Applicants hypothesized that the activation of the Notch pathway in MMTV-ErbB2 mammary tumors may contribute towards tumorigenesis in part by favouring luminal epithelial cell differentiation. To this end, Applicants investigated the levels of Notch signaling activation in MMTV-ErbB2-IRES-Cre mammary tumors by measuring the levels of Hes1 by Western blotting. As shown in figure 42A, MMTV-ErbB2 driven mammary tumors express very high levels of Hes1 protein compared to age matched normal mammary glands. To investigate the effect of I3 on breast cancer development, MMTV-ErbB2 mammary tumors were harvested from FVB mice carrying the MMTV-ErbB2 transgene. A single cell suspension was prepared and 5x10⁵ tumor cells were injected into an empty fat pad of a recipient FVB mouse. Once palpable tumors had developed, recipient mice were treated with either oil or 25 mg/kg of I3 on alternate days until the end of the experiment. The tumor volume was measured and recorded on regular intervals. Preliminary results showed that the treatment of tumor-bearing recipient mice with 13 caused significant tumor growth retardation when compared to mice treated with oil alone (Figure 42B). This data showed that 13 has the ability to slow down the growth of established breast cancer.

## Claims

1. Inhibitor of Notch signaling pathway for use in treating and/or preventing a cancer, wherein said inhibitor of Notch signaling pathway is selected from the group consisting of 6-4[-(tertbutyl)-phenoxy] pyridine-3-amine (13), Cyclopiazinic acid and Lasalocid.

2. The inhibitor of Notch signaling pathway of claim 1, wherein said inhibitor is 6-4[-(tertbutyl)-phenoxy] pyridine-3-amine (I3).

3. The inhibitor of Notch signaling pathway of claim 1 or 2, wherein the cancer is a Notch dependent cancer.

4. The inhibitor of Notch signaling pathway of any one of the preceding claims, wherein the Notch dependent cancer is selected from the group comprising T cell-Acute lymphoblastic leukemia (T-ALL), chronic myeloid leukemia (CML), chronic lymphocytic leukemia (CLL), breast cancer, pancreatic cancer, prostate cancer, melanoma, brain tumors, tumor angiogenesis, and colorectal cancer.

5. The inhibitor of Notch signaling pathway of any one of the preceding claims, wherein the Notch dependent cancer is resistant to γ-secretase inhibitor treatment.

6. A pharmaceutical composition comprising an inhibitor of Notch signaling pathway selected from the group consisting of 6-4[-(tertbutyl)-phenoxy] pyridine-3-amine (13), Cyclopiazinic acid and Lasalocid, and a pharmaceutically acceptable carrier.

7. The pharmaceutical composition of claim 6, wherein said inhibitor of Notch signaling pathway is 6-4[-(tertbutyl)-phenoxy] pyridine-3-amine (I3).

8. A kit comprising one or more doses of an inhibitor of Notch signaling pathway selected from the group consisting of 6-4[-(tertbutyl)-phenoxy] pyridine-3-amine (13), Cyclopiazinic acid and Lasalocid.

9. The kit of claim 8, further comprising one or more doses of a chemotherapeutic agent.
